# EUROPEAN PATENT SPECIFICATION

(11) **EP 1 587 840 B1**
(45) Date of publication and mention of the grant of the patent: **20.08.2008**
(21) Application number: 04701793.4
(22) Date of filing: 13.01.2004
(51) Int. Cl.: C07K 17/02, C07K 14/47, G01N 33/543

(54) **METHOD TO FORM A PROTEIN MICROARRAY SYSTEM**
VERFAHREN ZUR BILDUNG EINES PROTEIN-MIKROARRAYSYSTEMS
SYSTEME DE JEU ORDONNE DE MICROECHANTILLONS DE PROTEINES

(30) Priority: 13.01.2003 US 439625 P
(43) Date of publication of application: 26.10.2005
(73) Proprietor: THE REGENTS OF THE UNIVERSITY OF MICHIGAN, Ann Arbor, Mi 48109-1280 (US)
(72) Inventor: LUBMAN, David, M., Ann Arbor, MI 48104 (US); CHINNAIYAN, Arul, M., Plymouth, MI 48170 (US); SREEKUMAR, Arun, Ann Arbor, MI 48105 (US); LAXMAN, Bharathi, Ann Arbor, MI 48105 (US); YAN, Fang, Ann Arbor, MI 48105 (US)
(74) Representative: Glawe, Delfs, Moll
(86) International application number: PCT/US2004/000774
(87) International publication number: WO 2004/063719

(56) References cited:
- DATABASE CHEMABS [Online] CHEMICAL ABSTRACTS SERVICE, COLUMBUS, OHIO, US; YAN, FANG ET AL: "Protein microarrays using liquid phase fractionation of cell lysates" XP002287079 retrieved from STN Database accession no. 139:226767 & PROTEOMICS , 3(7), 1228-1235 CODEN: PROTC7; ISSN: 1615-9853, 2003,
- DATABASE CHEMABS [Online] CHEMICAL ABSTRACTS SERVICE, COLUMBUS, OHIO, US; MADOZ-GURPIDE, JUAN ET AL: "Protein based microarrays: a tool for probing the proteome of cancer cells and tissues" XP002287080 retrieved from STN Database accession no. 136:17625 & PROTEOMICS , 1(10), 1279-1287 PUBLISHED IN ELECTROPHORESIS, 22(18) CODEN: PROTC7; ISSN: 1615-9853, 2001,
- JOURNAL OF CHROMATOGRAPHY B, vol. 782, no. 1-2, December 2002 (2002-12), pages 183-196, XP004394255 NL ELSEVIER SCIENCE PUBLISHERS B.V. AMSTERDAM. 1431977

## Description

### FIELD OF THE INVENTION

The present invention relates to automated methods, systems, and apparatuses for protein separation and analysis. In particular, the present invention provides an automated system for the separation, identification, and characterization of protein samples, including the generation and analysis of protein microarrays.

### BACKGROUND OF THE INVENTION

As the nucleic acid sequences of a number of genomes, including the human genome, become available, there is an increasing need to interpret this wealth of information. While the availability of nucleic acid sequence allows for the prediction and identification of genes, it does not explain the expression patterns of the proteins produced from these genes. The genome does not describe the dynamic processes on the protein level. For example, the identity of genes and the level of gene expression does not represent the amount of active protein in a cell nor does the gene sequence describe post-translational modifications that are essential for the function and activity of proteins. Thus, in parallel with the genome projects there has begun an attempt to understand the proteome (*i.e.,* the quantitative protein expression pattern of a genome under defined conditions) of various cells, tissues, and species. Proteome research seeks to identify targets for drug discovery and development and provide information for diagnostics (e.g., tumor markers).

An important area of research is the study of the protein content of cells (*i.e.,* the identity of and amount of expressed proteins in a cell). Lubman et al. discloses 2-D liquid separations methods to map protein content of human cancer cells (Lubman et al., "Two-dimensional liquid separations-mass mapping of proteins from human cancer cell lysates", Journal of chromatography B, 782 (2002) 183-196 This field requires methods that can separate out large numbers of proteins and can do so quantitatively so that changes in expression or structure of proteins can be detected. The method generally used to achieve such cellular protein separations is 2-D PAGE. This method is capable of resolving hundreds of proteins based upon pI in one dimension and protein size in the second dimension. The proteins separated by this method are visualized using a staining method that can generally be quantified. The result is a 2-dimensional image where the protein map is based on pI and approximate molecular weight. By the use of computer based image analysis techniques, one can search for proteins that are differentially expressed in various cell lines. These methods are used to monitor changes in protein expression that are linked to conditions such as cell transformation and cancer progression, cell aging, the response of cells to environmental insult, and the response of cells to pharmaceutical agents. Once changes in protein expression have been identified, then one can further analyze target proteins to determine their identity and whether they have been altered from their expected structure by sequence changes orpost-translational modifications.

Although 2-D PAGE is still widely used for protein analysis, the method has several limitations including the fact that it is labor intensive, time consuming, difficult to automate and often not readily reproducible. In addition, quantitation, especially in differential expression experiments, is often difficult and limited in dynamic range. Also, while the 2-D gel does produce an image of the proteins in the cell, the mass determination is often only accurate to 5-10%, and the method is difficult to interface to mass spectrometric techniques for further analysis.

Another limitation of 2-D PAGE is the amount of protein loaded per gel which is generally below 250 µg. The amount of protein in any given spot may therefore be too low for further analysis. For Coomassie brilliant blue (CBB) stained gels the limit of detection is 100 ng per spot while for silver stained gels the limit of detection is 1-10 ng. Furthermore, proteins that have been isolated in 2-D gels are embedded inside the gel structure and are not free in solution, thus making it difficult to extract the protein for further analysis. Because of these limitations, the art is in need of protein mapping methods that are efficient, automated, and have broader resolution capabilities than presently available technologies.

Several protein microarray methodologies have been developed for investigation of proteins as disclosed for example in Madoe-Gúrpide et al. "Protein based microarrays: A tool for probing the proteome of cancer cells and tissues," Proteomics, 1 (2001) 1279-1287.

### SUMMARY OF THE INVENTION

The present invention is defined in the claims and relates to automated methods, systems, and apparatuses for protein separation and analysis. In particular, the present invention provides a method for generating protein microarrays as claimed in claim 1.

The present invention is useful for a system comprising an apparatus configured for automated sequential capillary electrophoresis - mass spectroscopy - mass spectroscopy of at least one protein sample. The at least one protein sample comprises a plurality of polypeptides, and wherein each of the at least one sample corresponds to a separated protein fraction. The system may further comprises a software program configured for performing the automated sequential capillary electrophoresis - first mass spectroscopy - second mass spectroscopy of the at least one protein sample. The software may be configured for determining mass-to-charge ratio of ions contained in the at least one protein sample in real time. The software may be further configured to apply a correct frequency to an ion trap of the second mass spectroscopy apparatus based on the mass to charge ratio. The system may further comprises a separated protein fraction separated in two dimensions. The system may further comprise a separated protein fraction separated in a first dimension by isoelectric point and a second dimension by hydrophobicity. The system may further comprise liquid separated protein fraction. The apparatus may be configured for automated sample preparation. The apparatus can further comprise an automatic fraction injector configured for the injection of the at least one sample into the apparatus. The software may be configured for automated sample analysis. The software can be configured for the generation of a multi-dimensional map corresponding to the at least one sample. The multi-dimension map may comprise information about two or more properties of the at least one sample, the properties selected from the group including, but not limited to, protein mw, protein hydrophobicity, protein isoelectric point, protein structure and protein sequence. The first and second mass spectroscopy can be ion trap TOF mass spectroscopy. The ion trap TOF mass spectroscopy may comprise the step of fragmenting the at least one sample prior to performing the ion trap TOF mass spectroscopy. The at least one protein sample may be enzymatically digested. The system may further comprise a plurality of the apparatuses, wherein the plurality of apparatuses are configured for the simultaneous analysis of a plurality of the at least one sample.

The present invention is further useful for an apparatus configured for automated sequential capillary electrophoresis - mass spectroscopy - mass spectroscopy of at least one sample, wherein each of the at least one sample comprises a separated protein fraction. The apparatus may further comprise a software program configured for performing the automated sequential capillary electrophoresis - first mass spectroscopy - second mass spectroscopy of the at least one sample. The software may be configured for determining mass-to-charge ratio of ions contained in the at least one protein sample in real time. The software may be further configured to apply a correct frequency to an ion trap of the second mass spectroscopy apparatus based on the mass to charge ratio. The separated protein fraction can be separated in two dimensions (*e.g.,* including, but not limited to, isoelectric point and hydrophobicity). The separated protein fraction can be a liquid. The apparatus can be configured for automated sample preparation. The apparatus may further comprises an automatic fraction injector configured for the injection of the at least one sample into the apparatus. The software can be configured for automated sample analysis. The software may be configured for the generation of a multi-dimensional map corresponding to the at least one sample. The multi-dimension map may comprise information about two or more properties of the at least on sample, the properties selected from the group including, but not limited to, protein mw, protein hydrophobicity, protein isoelectric point, protein structure, and protein sequence. The first and second mass spectroscopy can be ion trap TOF mass spectroscopy. The ion trap TOF mass spectroscopy may comprise the step of fragmenting the at least one sample prior to performing the ion trap TOF mass spectroscopy.

The present invention provides a method as claimed in claim 1 useful for the automated analysis of separated protein samples, comprising providing at least one sample comprising a plurality of polypeptides, wherein each of the at least one sample corresponds to an separated protein fraction; and an analysis apparatus configured for automated sequential capillary electrophoresis - mass spectroscopy - mass spectroscopy of the at least one sample; and treating the at least one sample with the analysis apparatus to generate analyzed sample. The apparatus may further comprise software configured for performing the automated sequential capillary electrophoresis - first mass spectroscopy - second mass spectroscopy of the at least one sample. The software may be configured for determining mass-to-charge ratio of ions contained in the at least one protein sample in real time. The software may be further configured to apply a correct frequency to an ion trap of the second mass spectroscopy apparatus based on the mass to charge ratio. The separated protein fraction can be separated in two dimensions (*e.g.,* including, but not limited to, isoelectric point and hydrophobicity). The separated protein fraction can be a liquid. The apparatus can be configured for automated sample preparation and the treating further comprises the step of automated sample preparation. The automated sample preparation can comprise automated partial dry down of the at least one sample. The automated sample preparation may further comprise automated enzymatic digestion of the at least one sample. The apparatus may further comprise an automatic fraction injector configured for the injection of the at least one sample into the apparatus. The software can be configured for automated sample analysis. The software can be configured for the generation of a multi-dimensional map corresponding to the at least one sample. The multi-dimension map may comprise information about two or more properties of the at least on sample, the properties selected from the group including, but not limited to, protein mw, protein hydrophobicity, protein isoelectric point, protein structure, and protein sequence. The first and second mass spectroscopy may be ion trap TOF mass spectroscopy. The ion trap TOF mass spectroscopy may comprise the step of fragmenting the at least one sample prior to performing the ion trap TOF mass spectroscopy. The method may further provide a plurality of the apparatuses. The treating may comprise simultaneous analysis of a plurality of the at least one sample with the plurality of apparatuses.

The present invention provides a method as claimed in claim 1 useful for the automated analysis of protein samples, comprising providing at least one sample comprising a plurality of polypeptides; at least one separation apparatus configured for the separation of proteins based on a physical property; and an analysis apparatus configured for automated sequential capillary electrophoresis - mass spectroscopy - mass spectroscopy of the at least one sample; and treating the at least one sample with the separation apparatus to generate a plurality of separated polypeptide fractions; and treating at least one of the plurality of separated polypeptide fractions with the analysis apparatus to generate an analyzed polypeptide sample. The analysis apparatus may further comprise software configured for performing the automated sequential capillary electrophoresis - first mass spectroscopy - second mass spectroscopy of the at least one sample. The software may be configured for determining mass-to-charge ratio of ions contained in the at least one protein sample in real time. The software may be further configured to apply a correct frequency to an ion trap of the second mass spectroscopy apparatus based on the mass to charge ratio. The at least one separation apparatus may comprises two separation apparatuses configured to separate the sample in two dimensions (*e.g.,* including, but not limited to, isoelectric point and hydrophobicity). The separated polypeptide fraction can be a liquid. The analysis apparatus may be configured for automated sample preparation and the treating further comprises the step of automated sample preparation. The automated sample preparation can comprise automated partial dry down of the separated sample. The automated sample preparation may further comprise automated enzymatic digestion of the separated sample. The analysis apparatus may further comprise an automatic fraction injector configured for the injection of the separated protein sample into the analysis apparatus. The software may be configured for automated sample analysis. The software may be configured for the generation of a multi-dimensional map corresponding to the analyzed sample. The multi-dimension map may comprise information about two or more properties of the at least on sample, the properties selected from the group including, but not limited to, protein mw, protein hydrophobicity, protein isoelectric point, protein structure, and protein sequence. The first and second mass spectroscopy can be ion trap TOF mass spectroscopy. The ion trap TOF mass spectroscopy may comprise the step of fragmenting the at least one sample prior to performing the ion trap TOF mass spectroscopy. The method may further provide a plurality of the analysis apparatuses. The treating may comprise simultaneous analysis of a plurality of the at least one sample with said plurality of analysis apparatuses.

The present invention provides a method for generating protein microarrays. The method for generating microarrays involves providing at least one sample comprising polypeptides. In addition, a first protein separation apparatus is provided. The first protein separation apparatus is a chromatofocusing apparatus. In addition, a second protein separation apparatus is provided (HPLC). In addition, a solid surface is provided.

The sample comprising polypeptides is treated with the first protein separation apparatus to generate a first separated polypeptide preparation. Next, the first separated polypeptide preparation is treated with the second protein separation apparatus to generate a second separated polypeptide preparation. At least a portion of the second separated polypeptide preparation is spotted onto a solid surface, thereby generating a protein microarray. In some preferred embodiments, the solid surface comprises a nitrocellulose slide.

The protein microarray is exposed to sera or other biological fluids. The sera sample is taken from a subject having a diseases (e.g., to be compared to a control sample from non-diseased subject). The sera sample exposed to the protein microarray is analyzed to detect antibody binding (e.g., to identify antibodies that differentially bind from the experimental sample to a different degree than those from a control sample).

### DESCRIPTION OF THE FIGURES

Figure 1 shows an example 2-D protein display using Isoelectric Focusing Non-Porous Reverse Phase HPLC (IEF-NP RP HPLC) separation of human erythroleukemia cell lysate proteins in one embodiment of the present invention.
Figure 2 shows a zoom area of a portion of the display in Figure 1 (pI = 4.2 to 7.2 and tR = 6.0 to 9.0) (right panel showing banding patterns) and a corresponding example of linked HPLC data (left panel showing peaks).
Figure 3 shows a quantification of rotofor fractions in one embodiment of the present invention.
Figure 4 shows NP RP HPLC separation from a Rotofor fraction of HEL cell lysate in one embodiment of the present invention.
Figure 5A and 5B show short (5A) and long (5B) NP RP HPLC separation gradient times for a rotofor fraction of HEL cell lysate in one embodiment of the present invention.
Figure 6 shows an example of Coomassie blue stained 2-D PAGE separation of HEL cell lysate proteins.
Figure 7 shows a direct side-by-side comparison of IEF-NP RP HPLC (four lanes on the left) with 1-D SDS PAGE (four lane on the right) for several Rotofor fractions in certain embodiments of the present invention.
Figures 8A and 8B show MALDI-TOF MS tryptic peptide mass maps for - enolase isolated by IEF-NP RP HPLC (8A) and by 2-D PAGE (8B).
Figure 9 shows a 2D protein image of Isoelectric Focusing - Non-porous RP HPLC - ESI oa TOF/MS (IEF-NPS RP HPLC-ESI oa TOF/MS) separation of human erythroleukemia cell lysate proteins.
Figure 10 shows a zoom of the 2D protein image from Figure 9 of 35 kDa to 52 kDa mass range.
Figure 11A and 11B show actin multiply charged umbrella with MaxEnt deconvoluted molecular weight mass spectrum. The umbrella for beta and gamma actin is shown in Figure 11A, each form of actin being labeled with the charge state. Figure 11B shows the resulting molecular weight mass spectrum for actin where the two forms of actin are separated.
Figure 12 shows combined protein molecular weight mass spectrum from a Rotofor fraction shown in traditional peak format.
Figure 13 shows a zoom of 2D protein image from Figure 9 of 5 kDa to 40 kDa mass range.
Figure 14 shows a chromatofocusing profile of MCF-10A whole cell lysate.
Figures 15A, B, and C show NP-RP-HPCL-ESI-oaTOF TIC (total ion count) profile of three sample fractions identified in Figure 14.
Figure 16 shows an integrated and deconvoluted TIC profile of the three sample fractions from Figure 15, as generated with MaxEntl software.
Figure 17 shows the anion exchange profile of Siberian Permafrost whole cell lysate of sample 23-9-25.
Figures 18A and 18B show the NP-RP-HPLC-ESI-oaTOF TIC profile of two fractions from Figure 17.
Figure 19 shows an overview of the automated protein analysis system utilized in some embodiments of the present invention.
Figure 20 shows a flow chart of the automated protein separation and analysis methods of the present invention.
Figure 21 shows an overview of the separation methods used in one embodiment of the present invention.
Figure 22 shows the multiple ionic capillary coating procedure utilized in some embodiments of the present invention.
Figure 23 shows a schematic description of CE-MS instrumentation used in some embodiments of the present invention.
Figure 24 shows a representative chromatogram of an rpHPLC run.
Figure 25 shows a representative 3-D profile of a CE-MS run.
Figure 26 shows a CE-MS elution profile of a tryptic digest.
Figure 27 shows a MS/MS spectrum of a tryptic digest of heat shock protein.
Figure 28 shows a comparison between theoretical and experimental oTs and MW of identified proteins.
Figure 29 shows a comparison of coverage between different MS methods.
Figure 30 shows a flow chart of the flow of information.
Figure 31 shows a 2-D map of the liquid separations of the LnCAP cells using chromatofocusing over a pH range of 4-7 followed by separation in the second dimension using NPS RP BPLC.
Figure 32 shows the NPS RP HPLC separation of CF fraction #5 in Example 11 at pH 6.0-6.2.
Figure 33 shows a concentration profile as a function as a function of pI for the chromatofocusing of the LnCap cell line from pH 7.0 to pH 4.0.
Figure 34 shows the detection of humoral response in serum from normal and prostate cancer patients.
Figure 35 shows detection of humoral response in prostate cancer against fraction B6.
Figure 36 shows detection of humoral response in prostate cancer against Creatine Kinase fractionated at pH 6-6.2.
Figure 37 shows detection of humoral response in prostate cancer against 54 kDa nuclear RNA-DNA binding protein fractionated at pH 6-6.2.

### GENERAL DESCRIPTION OF THE INVENTION

The present invention is defined in the claims and relates to protein separation and analysis methods capable of resolving large numbers of cellular proteins, including methods for efficiently facilitating the transfer of protein samples between separation phases. In some embodiments, the present invention provides automated methods for the separation and characterization of proteins using capillary electrophoresis and tandem ion trap mass spectroscopy.

The methods of the present invention further provide protein profile maps for imaging and comparing protein expression patterns. The present invention provides alternatives to traditional 2-D gel separation methods for the screening of protein profiles. Many limitations of traditional 2-D PAGE arise from its use of the gel as the separation media. The present invention provides alternative media for the separation that offer significant advantages over 2-D PAGE techniques. For example, the present invention provides methods that use two dimensional separations, where the second dimensional separation occurs in the liquid phase, rather than 2-D PAGE techniques where the final separation occurs in gel.

The present invention provides methods for protein separation and mapping that are highly efficient, amenable to automation, and provide detailed resolution. For example, in some methods of the present invention, proteins are separated according to their pI, using isoelectric focusing (IEF) (*e.g.*, in the Rotofor); according to their hydrophobicity using non-porous reverse phase HPLC (NPS RP HPLC); and according to mass using ESI oa TOF/MS or other mass spectrometry techniques. The present invention further relates for novel techniques for eluting proteins from a separation apparatus (*e.g.,* the first phase separation apparatus). For example, the proteins eluted from the first dimension are peeled off from the column according to their pH, either one pH unit or fraction thereof, at a time. In some embodiments, these focused liquid fractions are then separated according to their hydrophobicity and size (or other desired properties) in the second dimension. Liquid fractions from, for example, NP-RP-HPLC can be conveniently analyzed directly on-line using mass spectrometry (e.g., ESI-oaTOF) to obtain their molecular weight and relative abundance, which provides a third dimension. As a result, a virtual 2-D protein image is created and is analogous to a 2-D gel image.

Experiments conducted during the development of the present invention have demonstrated that these methods are capable of separating large numbers of proteins. The 2-D image of these proteins, analogous to that of a 2-D gel, can be generated for the purpose of observing distinctive patterns from a particular cell line. This protein pattern provides relative quantitative information, high mass resolution and high accuracy pI and mass values. Given that the intensity, mass and pI values are reproducible, one can study differential expression of proteins where the resulting 2-D images from different cells, tissues, or samples can be quantitatively compared to identify points of interest. Furthermore, automation and speed of analysis are greatly facilitated given that the proteins remain in the liquid phase throughout the separation. The method, abbreviated IEF-NPS RP HPLC-ESI oa TOF/MS is shown to be a viable alternative for the separation of complex protein mixtures and the generation of high-resolution 2-D images of cellular protein expression.

In some embodiments of the present invention, proteins are separated in a first dimension using any of a large number of protein separation techniques including, but not limited to, ion exclusion, ion exchange, normal/reversed phase partition, size exclusion, ligand exchange, liquid/gel phase isoelectric focusing, and adsorption chromatography. In some preferred embodiments of the present invention, the first dimension is a liquid phase separation method. The sample from the first separation is passed through a second dimension separation. In preferred embodiments of the present invention, the second dimension separation is conducted in liquid phase. The products from the second dimension separation are then characterized. For example, in preferred embodiments, the products of the second separation step are detected and displayed in a 2-D format based on the physical properties of the proteins that were distinguished in the first and second separation steps (*e.g.,* under conditions such that the first and the second physical properties are revealed for at least a portion of the proteins). The products may be further analyzed, for example, by mass spectrometry to determine the mass and/or identity of the products or a subset of the products. In these embodiments, a three dimensional characterization can be applied (*i.e.,* based on the physical properties of the first two separation steps and the mass spectrometry data). It is contemplated that other protein processing steps can be conducted at any stage of the process.

In certain embodiments of the present invention, the steps are combined in an automated system. In preferred embodiments, each of the steps is automated. For example, the present invention is useful for a system that includes each of the separation and detection elements in operable combination so that a protein sample is applied to the system and the user receives expression map displays or other desired data output. To achieve automation, in preferred embodiments, the products of each step should be compatible with the subsequent step or steps.

In one illustrative embodiment of the present invention proteins are separated according to their pI, using isoelectric focusing (IBF) in a Rotofor and according to their hydrophobicity and molecular weight using NP RP HPLC. This combined separation method is abbreviated IEF-NP RP HPLC. When coupled with mass spectrometry (MS) this technique becomes three-dimensional and allows for the creation of a protein map that tells the pI and the molecular weight of the proteins in question. This information can be plotted in an image that also depicts protein abundance. The end result is a high-resolution image showing a complex pattern of proteins separated by pI and molecular weight and indicating relative protein abundances. This image can be used to determine how the proteins in a given cell line or tissue may change due to some disease state, pharmaceutical treatment, natural or induced differentiation, or change in environmental conditions. The image allows the observer to determine changes in pI, molecular weight, and abundance of any protein in the image. When interfaced to MS the identity of any target protein may also be obtained via enzymatic digests and peptide mass map analyses. In addition, this technique has the advantage of very high loadability (e.g., 1 gram) such that the lower abundance proteins may be detected.

In traditional 2-D PAGE separation and display techniques, the second phase separation is conducted in a gel *(i.e.,* not a liquid phase) and the proteins are separated and detected by differences in molecular weight. In contrast, in some embodiments of the present invention, the second phase separation is conducted in liquid phase. The products of the second phase separation techniques of the present invention are much more amenable to further characterization and to interpretation of data produced from the second phase. For example, in some embodiments of the present invention, the second phase is conducted using HPLC where the separated protein products are readily detected as peak fractions and interpreted and displayed in two dimensions by a computer based on the physical properties of the first and second separation steps. The products of HPLC separation, being in the liquid phase, are readily used in further detection steps (e.g., mass spectrometry). The methods of the present invention, as compared to traditional 2-D PAGE, allow more sample to be analyzed, are more efficient, facilitate automation, and allow for the analysis of proteins that are not detectable with 2-D PAGE.

For example, in one illustrative embodiment of the present invention, the protein profile of human erythroleukemia (HEL) cells has been analyzed using the methods of the present invention as well as traditional gel based methods for comparison purposes. Two-dimensional images were generated representing each of the separation methods used. Proteins were separated and then collected using both the IEF-NP RP HPLC of the present invention and 2-D PAGE methods. These proteins were then enzymatically digested and the peptide mass maps were determined by MALDI-TOF MS (if a protein cannot be unambiguously identified by this method, further analysis is made by any number of techniques including, but not limited to, LC/MS-MS, PSD-MALDI, NMR, Western blotting, Edman sequence analysis and mass spectrometry can help with further analysis of proteins [*See e.g.,* Yates, J. Mass Spec., 33:1 (1998); Chen et al., Rap. Comm. Mass Spec., 13:1907 (1999); Neubauer and Mann, Anal. Chem. 71:235 (1999); Zugaro et al., Electrophoresis 19:867 (1998); Immler et al., Electrophoresis 19:1015 (1998); Reid et al., Electrophoresis 19:946 (1998); Rosenfeld, et al., Anal. Biochem., 203:173 (1992); Matsui et al., Blectrophoresis 18:409 (1997); Patterson and Aebersold, Electrophoresis 16:1791 (1995)]).

In some embodiments, the proteins were tentatively identified using MS-Fit to search the peptide mass maps against the Swiss and NCBInr protein databases. This work demonstrated that a large number of proteins, with a useful mass range, were separated using the methods of the present invention and that a 2-D image of these proteins was reproducibly generated for the purpose of observing distinctive patterns that are associated with a particular cell line. The methods of the present invention allowed for the detection of proteins not observed with the 2-D PAGE technique. Automation and speed of analysis are also greatly facilitated given that the proteins remain in the liquid phase throughout the separation.

In some embodiments, the present invention is useful for an automated protein separation and characterization system. The system is fully integrated and transfers and coordinates multi-phase, orthogonal separation methods. The information can be transferred by the automated system to software for the generation of multi-dimensional protein maps. Automation provides increased speed, efficiency, and sample recovery while eliminating potential sources of contamination and sample loss.

Thus, the methods of the present invention are shown to be an advantageous technique for the generation of images of protein expression profiles as well as for the collection of individual proteins for further analyses: These capabilities allow one to monitor changes in protein expression that are linked to differentiation pathways as well as particular conditions such as cancer *(See e.g.,* Hanash, Advances in Electrophoresis; Chrambach, A., Editor, pp 1-44 [1998]), cell aging *(See e.g.,* Steller, Science 267:1445 [1995]), the response of cells to environmental insult *(See e.g.,* Welsh et al., Biol. Reprod., 55:141 [1996]), or the response of cells to some pharmaceutical agent. Having identified significant changes in protein expression, one can then further analyze proteins of interest to determine their identity and whether they have been altered from their expected structure by sequence changes or post-translational modifications.

### Definitions

To facilitate an understanding of the present invention, a number of terms and phrases are defined below:

As used herein, the term "separated protein fraction" refers to one fraction of a sample separated in one or more dimensions (e.g., a fraction from a chromatography separation).

As used herein, the term "microarray" refers to a solid surface comprising a plurality of addressed biological macromolecules (e.g., proteins, polypeptides, nucleic acids, antibodies, etc.). In some preferred embodiments, the location of each of the macromolecules in the microarray is known, so as to allow for identification of the samples following analysis.

As used herein, the term "array of target molecules" or "array of target molecule binding targets" refers to a microarray of target molecules that are known to, or are suspected of, associating with a "cellular product."

As used herein, the terms "solid support" or "support" refer to any material that provides a solid or semi-solid structure with which another material can be attached. Such materials include smooth supports (e.g., metal, glass, plastic, silicon, and ceramic surfaces) as well as textured and porous materials. Such materials also include, but are not limited to, gels, rubbers, polymers, and other non-rigid materials. Solid supports need not be flat. Supports include any type of shape including spherical shapes (e.g., beads). Materials attached to solid support may be attached to any portion of the solid support (e.g., may be attached to an interior portion of a porous solid support material). Preferred embodiments of the present invention have biological molecules such as proteins attached to solid supports. A biological material is "attached" to a solid support when it is associated with the solid support through a non-random chemical or physical interaction. In some preferred embodiments, the attachment is through a covalent bond. However; attachments need not be covalent or permanent. In some embodiments, materials are attached to a solid support through a "spacer molecule" or "linking group." Such spacer molecules are molecules that have a first portion that attaches to the biological material and a second portion that attaches to the solid support. Thus, when attached to the solid support, the spacer molecule separates the solid support and the biological materials, but is attached to both.

As used herein, the term "an apparatus configured for automated sequential capillary electrophoresis - mass spectroscopy - mass spectroscopy of said at least one sample" refers to an apparatus configured for automated sequential analysis by capillary electrophoresis, mass spectroscopy, and a second mass spectroscopy step without user intervention. The apparatus may perform automated sample preparation, injection, and analysis. The apparatus may include robotics for automated sample handling. The apparatus may include software and a computer system for directing the operation of the apparatus (*e.g.,* "a software program configured for performing said automated sequential capillary electrophoresis - mass spectroscopy - mass spectroscopy of said at least one sample").

As used herein, the term "automated sample preparation" refers to the preparation of a sample for analysis using the apparatus of the present invention in the absence of operator intervention. Automated sample preparation may be performed by robotics. Software and a computer system may control the automated sample preparation. Automated sample preparation includes all steps necessary to prepare a sample for analysis including, but not limited to, partial dry down and enzymatic digestion.

As used herein, the term "automatic fraction injector configured for the injection of said at least one sample into said apparatus" refers to an apparatus configured for the injection of sample into a capillary electrophoresis - mass spectroscopy - mass spectroscopy apparatus in the absence of operator intervention. The automated fraction injector may utilize robotics controlled by software and a computer system.

As used herein, the term "CE-MS/MS analyzed sample" refers to a sample that has been treated by the capillary electrophoresis - mass spectroscopy - mass spectroscopy apparatus of the present invention. The analyzed sample may comprise information relating to a polypeptides contained in the sample (e.g., including, but not limited to, protein MW, identity, and structure).

As used herein, the term "multiphase protein separation" refers to protein separation comprising at least two separation steps. Multiphase protein separation may refer to two or more separation steps that separate proteins based on different physical properties of the protein (e.g., a first step that separates based on protein charge and a second step that separates based on protein hydrophobicity).

As used herein, the term "protein profile maps" refers to representations of the protein content of a sample. For example, "protein profile map" includes 2-dimensional displays of total protein expressed in a given cell. Protein profile maps may also display subsets of total protein in a cell. Protein profile maps may be used for comparing "protein expression patterns" (*e.g*., the amount and identity of proteins expressed in a sample) between two or more samples. Such comparing find use, for example, in identifying proteins that are present in one sample (*e.g*., a cancer cell) and not in another (e.g., normal tissue), or are over- or under-expressed in one sample compared to the other.

As used herein, the term "separating apparatus capable of separating proteins based on a physical property" refers to compositions or systems capable of separating proteins (*e.g.,* at least one protein) from one another based on differences in a physical property between proteins present in a sample containing two or more protein species. For example, a variety of protein separation columns and composition are contemplated including, but not limited to ion exclusion, ion exchange, normal/reversed phase partition, size exclusion, ligand exchange, liquid/gel phase isoelectric focusing, and adsorption chromatography. These and other apparatuses are capable of separating proteins from one another based on their size, charge, hydrophobicity, and ligand binding affinity, among other properties. A "liquid phase" separating apparatus is a separating apparatus that utilizes protein samples contained in liquid solution, wherein proteins remain solubilized in liquid phase during separation and wherein the product (*e.g.,* fractions) collected from the apparatus are in the liquid phase. This is in contrast to gel electrophoresis apparatuses, wherein the proteins enter into a gel phase during separation. Liquid phase proteins are much more amenable to recovery/extraction of proteins as compared to gel phase. Liquid phase proteins samples may be used in multi-step (*e.g.,* multiple separation and characterization steps) processes without the need to alter the sample prior to treatment in each subsequent step (*e.g*., without the need for recovery/extraction and resolubilization of proteins).

As used herein, the term "displaying proteins" refers to a variety of techniques used to interpret the presence of proteins within a protein sample. Displaying includes, but is not limited to, visualizing proteins on a computer display representation, diagram, autoradiographic film, list, table, chart, etc. "Displaying proteins under conditions that first and second physical properties are revealed" refers to displaying proteins (*e.g*., proteins, or a subset of proteins obtained from a separating apparatus) such that at least two different physical properties of each displayed protein are revealed or detectable. For example, such displays include, but are not limited to, tables including columns describing (*e.g.,* quantitating) the first and second physical property of each protein and two-dimensional displays where each protein is represented by an X,Y locations where the X and Y coordinates are defined by the first and second physical properties, respectively, or vice versa. Such displays also include multi-dimensional displays (*e.g.,* three dimensional displays) that include additional physical properties.

As used herein, "characterizing protein samples under conditions such that first and second physical properties are analyzed" refers to the characterization of two or more proteins, wherein two different physical properties are assigned to each analyzed (*e.g.,* displayed, computed, etc.) protein and wherein a result of the characterization is the categorization (*i.e.,* grouping and/or distinguishing) of the proteins based on these two different physical properties. For example, two proteins are separated based on isoelectric point and hydrophobicity.

As used herein, the term "comparing first and second physical properties of separated protein samples" refers to the comparison of two or more protein samples (or individual proteins) based on two different physical properties of the proteins within each protein sample. Such comparing includes grouping of proteins in the samples based on the two physical properties and comparing certain groups based on just one of the two physical properties *(i.e.*, the grouping incorporates a comparison of the other physical property).

As used herein, the term "delivery apparatus capable of receiving a separated protein from a separating apparatus" refers to any apparatus (*e.g.*, microtube, trough, chamber, etc.) that receives one or more fractions or protein samples from a protein separating apparatus and delivers them to another apparatus (*e.g.*, another protein separation apparatus, a reaction chamber, a mass spectrometry apparatus, etc.).

As used herein, the term "detection system capable of detecting proteins" refers to any detection apparatus, assay, or system that detects proteins derived from a protein separating apparatus (*e.g.*, proteins in one or fractions collected from a separating apparatus). Such detection systems may detect properties of the protein itself (*e.g.*, UV spectroscopy) or may detect labels (e.g., fluorescent labels) or other detectable signals associated with the protein. The detection system converts the detected criteria (*e.g.*, absorbance, fluorescence, luminescence etc.) of the protein into a signal that can be processed or stored electronically or through similar means (*e.g*., detected through the use of a photomultiplier tube or similar system).

As used herein, the term "buffer compatible with an apparatus" and "buffer compatible with mass spectrometry" refer to buffers that are suitable for use in such apparatuses (*e.g.*, protein separation apparatuses) and techniques. A buffer is suitable where the reaction that occurs in the presence of the buffer produces a result consistent with the intended purpose of the apparatus or method. For example, a buffer compatible with a protein separation apparatus solubilizes the protein and allows proteins to be separated and collected from the apparatus. A buffer compatible with mass spectrometry is a buffer that solubilizes the protein or protein fragment and allows for the detection of ions following mass spectrometry. A suitable buffer does not substantially interfere with the apparatus or method so as to prevent its intended purpose and result (*i.e.*, some interference may be allowed).

As used herein, the term "automated sample handling device" refers to any device capable of transporting a sample (*e.g.,* a separated or un-separated protein sample) between components (*e.g.,* separating apparatus) of an automated method or system (*e.g.,* an automated protein characterization system). An automated sample handling device may comprise physical means for transporting sample (*e.g.,* multiple lines of tubing connected to a multi-channel valve). An automated sample handling device may be connected to a centralized control network.

As used herein, the term "switchable multi channel valve" refers to a valve that directs the flow of liquid through an automated sample handling device. The valve preferably has a plurality of channels (*e.g.,* 2 or more, and preferably 4 or more, and more preferably, 6 or more). In addition, flow to individual channels may be "switched" on an off. Valve switching may be controlled by a centralized control system. A switchable multi-channel valve allows multiple apparatus to be connected to one automated sample handler. For example, sample can first be directed through one apparatus of a system (*e.g.,* a first chromatography apparatus). The sample can then be directed through a different channel of the valve to a second apparatus (*e.g.,* a second chromatography apparatus).

As used herein, the terms "centralized control system" or "centralized control network" refer to information and equipment management systems (*e.g.*, a computer processor and computer memory) operably linked to multiple devices or apparatus (*e.g.*, automated sample handling devices and separating apparatus). The centralized control network may be configured to control the operations of the apparatus and/or device linked to the network For example, the centralized control network may control the operation of multiple chromatography apparatuses, the transfer of sample between the apparatuses, and the analysis and presentation of data.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (*e.g.,* data and instructions) to a computer processor. Examples of computer readable media include, but are not limited to, DVDs, CDs, hard disk drives, magnetic tape and servers for streaming media over networks.

As used herein, the terms "processor" and "central processing unit" or "CPU" are used interchangeably and refers to a device that is able to read a program from a computer memory (*e.g.,* ROM or other computer memory) and perform a set of steps according to the program.

As used herein, the term "directly feeding" a protein sample from one apparatus to another apparatus refers to the passage of proteins from the first apparatus to the second. apparatus without any intervening processing steps. For example, a protein that is directly fed from a protein separating apparatus to a mass spectrometry apparatus does not undergo any intervening digestion steps (*i.e.,* the protein received by the mass spectrometry apparatus is undigested protein).

As used herein, the term "sample" is used in its broadest sense. In one sense it can refer to a cell lysate. In another sense, it is meant to include a specimen or culture obtained from any source, including biological and environmental samples. Biological samples may be obtained from animals (including humans) and encompass fluids, solids, tissues, and gases. Biological samples include blood products (*e.g.,* plasma and serum), saliva, urine, and the like and includes substances from plants and microorganisms. Environmental samples include environmental material such as surface matter, soil, water, and industrial samples. These examples are not to be construed as limiting the sample types applicable to the present invention.

### DETAILED DESCRIPTION OF THE INVENTION

The present invention is defined in the claims and relates to automated methods, systems, and apparatuses for protein separation and analysis. In particular, the present invention provides a method for generating protein, microarrays as claimed in claim 1.

In some embodiments, the present invention is useful for systems and apparatuses for liquid separation of the protein content of a cellular sample with subsequent structural analysis using capillary electrophoresis tandem time-of-flight mass spectrometry. The liquid separation uses a 2-D dimensional liquid separation of the protein content of the cell using chromatofocusing to generate a pI based separation followed by nonporous RP HPLC separation to generate an image of the protein content of the sample. In some embodiments, the liquid protein are collected in a fraction collector and then each sample is digested and analyzed by capillary electrophoresis separation with on-line mass spectrometric analysis using a quadrupole ion trap/time-of-flight tandem mass spectrometer for identification and sequence analysis. The methods of the present invention are easily automated to identify and perform structural analysis on hundreds of proteins collected in the liquid phase per day. The below description provides a description of the present invention.

### I. Automated Protein Analysis System

The present invention is useful for automated protein analysis system. Proteins are first separated using any suitable separation method (*e.g*., including, but not limited to, those disclosed herein). Proteins are separated using the 2-D liquid separation methods described herein. For example, the method to be used in the first dimension involves a liquid ph based pI separation (*e.g*., including, but not limited to, chromatofocusing, isoelectric focusing in a Rotofor device or Isoprime-type device, or free flow electrophoresis in the liquid phase; *See e.g.,* below description of separating methods). The pI fractions from the first separation may be automatically collected and injected into the second phase for analysis. The second phase uses nonporous reversed phase chromatography to separate the proteins from each pI fraction in the second dimension.

The present invention is not limited to liquid separation. Any separation method may be utilized (*e.g.,* gel electrophoresis). The present invention is also not limited to 2-dimension separation. Any separation method that separates samples in one or more dimensions may be utilized.

Software to generate a 2-D image of the protein content of the cell may be used. Following separation, samples are analyzed using the CB-MS/MS methods described below.

### A. CE-MS/MS

In some preferred embodiments, the present invention provides a capillary electrophoresis - Mass Spectroscopy - Mass spectroscopy (CE-MS/MS) apparatus to identify and characterize separated proteins in the samples. In some preferred embodiments, isolated proteins are collected in the liquid phase directly from the liquid eluent of the HPLC or other protein separation apparatus *(See e.g.,* above description of separation methods). For example, in some embodiments, each protein fraction is collected by an automatic fraction collector, partially dried down and enzymatically digested.

The protein digests are then sequentially analyzed by capillary electrophoresis (CE)-mass spectrometry (*e.g.*, TOF mass spectroscopy). An automatic fraction injector may be utilized to inject each sample. For example, the capillary is dipped into an enzyme digest (*e.g.*, by a computer controlled robotic arm). The enzyme digest is injected into the capillary by applying a loading voltage (*e.g.,* using a computer controlled robotic arm). The capillary is then removed from the digest vial (*e.g.,* using the robotic aim) and lowered into a buffer vial. The CE running voltage is then turned on and separation of the digest occurs in approximately ten minutes. The peptides are then detected on-line using mass spectrometry (*e.g.,* TOF mass spectroscopy). In some preferred embodiments, an ion-trap/TOF MS is used to detect the peptide masses. One advantage of this method is that the intact ions are detected and the MW of the peptide determined.

As the eluting peak is being detected, the MW is identified (*e.g.*, by an automated computer system), and the parent ion and fragment ion are isolated to perform tandem MS (MS/MS) to obtain structural analysis of the peptide or manual override during the single injection is used to force fragmentation at a specific MW when desired. A second injection with manually loaded parameters may be utilized. The automated selection method may be applied to the entire peptide digest as it elutes from the CE and a peptide map with MS/MS obtained for the protein. The MW, protein digest, and additional data (*e.g.,* pI) is then be used to identify the protein against a database with high certainty (See *e.g.,* below description of database searching). In addition, the MS/MS data maybe used to confirm identification and also for detailed structural analysis or to identify the presence and position of posttranslational modifications.

For example, for the real time MW identification and subsequent structural analysis of eluting peptides, the MS is initially configured to collect intact ions without fragmentation. Each mass spectra is analyzed over the entire mass range for the presence of ions exceeding a threshold. When a computer algorithm determines that a peptide has begun eluting, its MW is determined. Fragmentation for structural identification is then performed for the remaining duration of the peak's elution. The ions in the eluting peak are confined to an ion trap and a waveform consisting of all frequencies except for the frequency resonance with the desired ion is applied to the cell. This notched waveform excludes all interferences from the ion trap. Then a low amplitude frequency that is resonant with the desired ion is applied to partially fragment it. When subsequently ejected from the ion trap, both parent and fragment ion information is obtained free of background In order to know the appropriate frequency to apply to the ion trap, the mass-to-charge ratio of the desired ion must be known. Software can be used to determine this in real time as a peak begins to elute. The notched waveform is immediately calculated and applied to the ion trap so that background-free spectra can be acquired over as much of the peak as possible. This capability avoids the necessity of performing two injections with the first done to obtain the desired ion-of-interest information. This more than doubles the throughput. The amplitude of the frequency resonant with the desired ion-of-interest is feedback adjusted in real time from spectra to spectra to optimize the parent to fragment ion ratio.

The CE-MS/MS apparatus provides several advantages over currently available systems. For example, the ion trap MS/MS system is able to rapidly process samples separated by CE. The ion trap MS systems of the present invention are able to process spectrum at a rate of 2-10 spectra/sec. This is in contrast to currently available scanning MS systems (*e.g.,* those available from Micromass, and Applied Biosystems, Foster City, CA), which are not able to process spectrum fast enough to keep up with CE separations. The present invention is useful for an all liquid, automated separation and analysis system. This system (See below description of automation and software) provides the added benefit of sequential purification and analysis without additional user intervention.

### B. Software

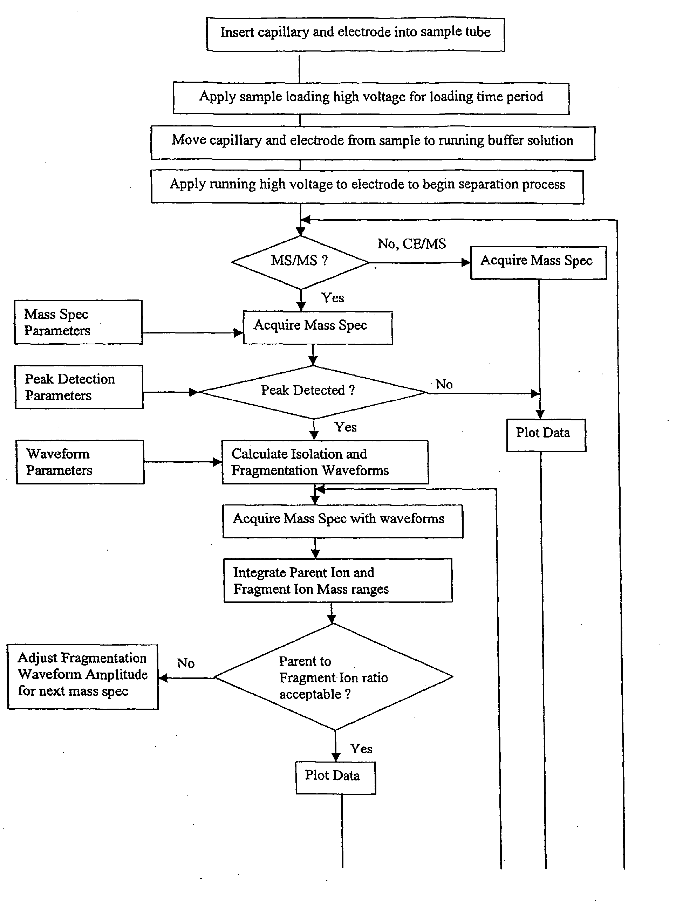

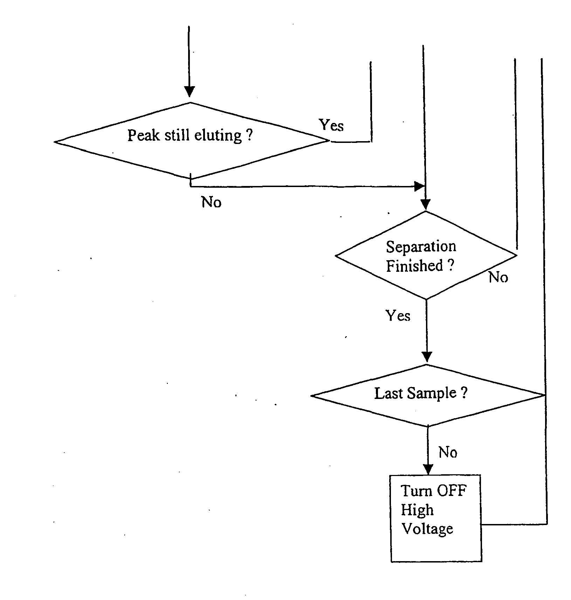

Software for the operation of the automated apparatus and the analysis of data can le used. Figure 30 shows a flow chart describing the flow of information The software is in communication with a computer processor and the CE-MS/MS apparatus

The software may control all aspects of the analysis and characterization. For example, the software controls robotic arms and auto injectors used to prepare samples for CE-MS/MS and inject samples into the CE. The software further controls a high voltage power supply for CE sample loading and running, and CE column/electrode mechanical positioning for loading and running and overall system timing.

The software may analyze the parent ion peak and determines whether or not to fragment the remaining peak for a second round of MS. The notched waveform is immediately calculated and applied to the ion trap so that background-free spectra can be acquired over as much of the peak as possible. This capability avoids the necessity of performing two injections with the first done to obtain the desired ion-of interest information. This more than doubles the throughput. The amplitude of the frequency resonant with the desired ion-of-interest is feedback adjusted in real time from spectra to spectra to optimize the parent to fragment ion ratio.

Data from the time of flight mass spectrometer is digitized and recorded by a computer using the software The software may further provide representation of the data as a two dimensional image for visual identification of important mass spectral peak locations.

Software analyses multi-dimension protein maps generated from separation steps and determines which fractions to select for further applications. The software may analyze the CE-MS/MS data, in combination with data obtained in separation steps, to determine protein identity (*e.g.,* by searching peptide databases) and to display results. Results may be displayed as a multidimensional protein map with information corresponding to protein MW, identity, and other physical properties. The results may be displayed on a computer display screen.

### C. Automation

The present invention can provide fully automated protein separation and characterization. Previously, two mass spectroscopy injections were required to accomplish structural determination. The first was obtained without fragmentation in order to acquire the protein MW information. Manual analysis was performed to construct a table of expected protein elution times, MW and corresponding waveforms. The second injection used that information to decide when to apply the specified pre-determined information for fragmentation. The real time on-the-fly method of the present invention results in time savings of more than a factor of two (only one vs. two injections required plus manual analysis time not required) and uses one half the sample amount. Furthermore, multiple samples can be sequentially analyzed without having to wait for manual analysis of the first injection for each.

Once a protein digest is analyzed by CE-TOF MS/MS, the computer places the data in a format that it is imported directly into a database for identification and analysis. The computer then instructs the automatic fraction injector to inject the next sample. The next CE can then proceed while data analysis of the first digest is performed by the computer. The 50-100 samples typically isolated from each pI fraction in the liquid phase can be easily processed using the automated methods. For example, at approximately 10 minutes per sample, it takes approximately 8-10 hours to analyze the proteins from each pI in detail. Multiple CE-MS/MS systems can be linked in tandem to one purification system, allowing for the simultaneous analysis of multiple separated fractions. In addition, the computer can be programmed to automatically select proteins from the 2-D liquid map for analysis.

### D. The System in Operation

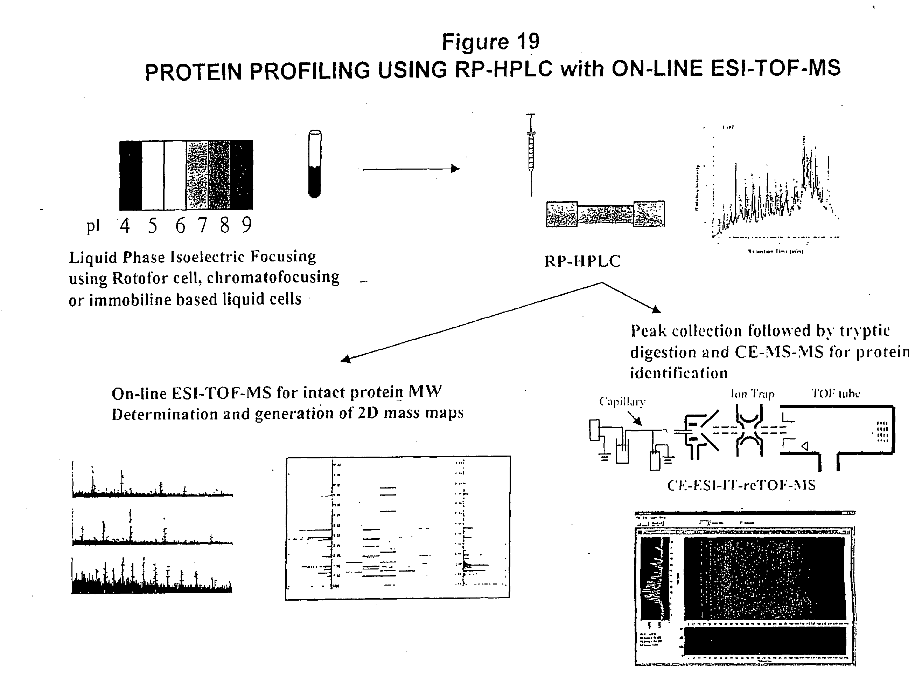

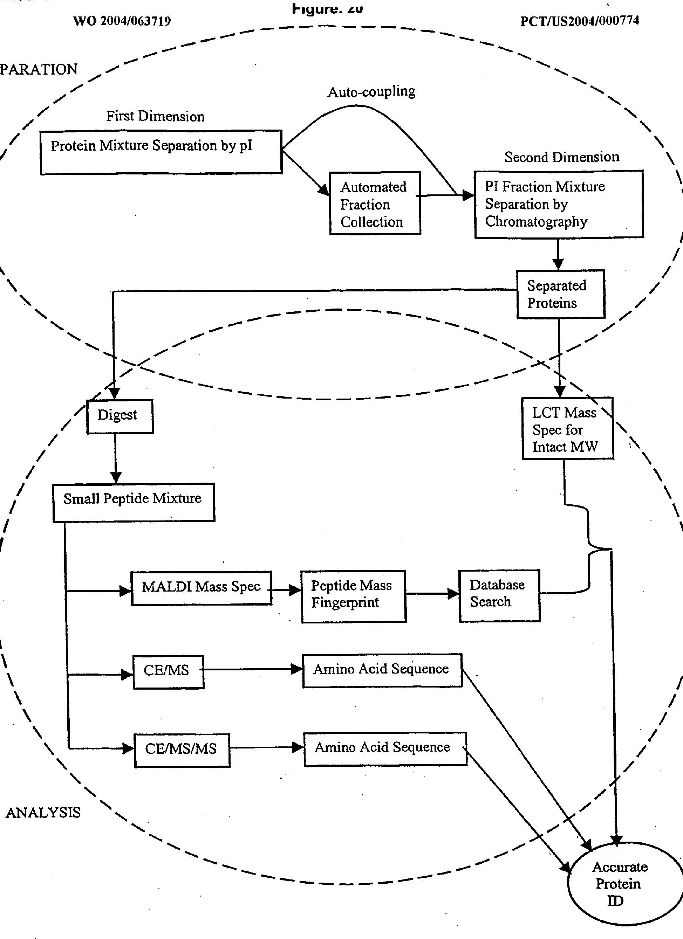

Figure 19 shows an overview of one illustrative embodiment of the present invention. Figure 20 shows a flow chart of the separation and analysis methods of the present invention. Example 9 provides an exemplary method used in some embodiments of the present invention.

In some embodiments, the method of the present invention provides an all liquid method for separation, identification and detailed analysis of proteins from cells. In some embodiments, the method utilizes separation of proteins using a 2-D liquid separation with subsequent on-line detection using electrospray (ESI)-TOF mass spectrometry. This method provides a 2-D image of the protein content of the cell according to pI versus MW of the intact protein. The map is used as the standard to compare different cell lines for changes in protein expression or structure. The mass map can be used to identify proteins that change in MW or quantitative expression for further analysis.

In some embodiments, differential display maps are used to compare different cell lines. Software is used to identify proteins that change using the differential display mode. The computer then searches for these changes and instructs the automatic fraction injector to analyze only these samples in detail.

The methods of the present invention maybe utilized for high-throughput proteome analysis. For example, an analysis system that comprises a plurality of CE-MS/MS apparatuses can be utilized for the simultaneous analysis of MW, structural characteristics (e.g., post translational modifications), physical properties, and identity of proteins in a plurality of separated fractions. Using such methods, an entire proteome can be analyzed in the course of a day, with small sample requirements and little or no operator intervention required. The rapid methods of the present invention allow for the analysis of an organism or cell's proteome on a recurring basis (*e.g.,* daily in response to an external stimulus). Such methods are useful, *e.g.,* in drug screening applications, to characterize development, and to monitor malignancies.

### II. Two-Phase Separation Techniques

The first dimension separates proteins based on a first physical property. For example, proteins are separated by pI using isoelectric focusing in the first dimension *(See e.g.,* Righetti, Laboratory Techniques in Biochemistry and Molecular Biology; Work, T. S.; Burdon, R H., Elsevier: Amsterdam, p 10 [1983]). However, the first dimension may employ any number of separation techniques including, but not limited to, ion exclusion, ion exchange, normal/reversed phase partition, size exclusion, ligand exchange, liquid/gel phase isoelectric focusing, and adsorption chromatography. In some embodiments (*e.g.,* some automated embodiments), it is preferred that the first dimension be conducted in the liquid phase to enable products of the separation step to be fed directly into a second liquid phase separation step.

The second dimension separates proteins based on a second physical property (*i.e.*, a different property than the first physical property) and is preferably conducted in the liquid phase (*e.g.*, liquid-phase size exclusion). Proteins are separated by hydrophobicity using non-porous reversed phase HPLC in the second dimension (*See e.g.,* Liang et al., Rap. Comm. Mass Spec., 10:1219 [1996]; Griffin et al., Rap. Comm. Mass Spec., 9:1546 [1995]; Opiteck et at, Anal. Biochem. 258:344 [1998]; Nilsson et al., Rap. Comm. Mass Spec., 11:610 [1997]; Chen et al., Rap. Comm. Mass Spec., 12:1994 [1998]; Wall et al., Anal. Chem., 71:3894 [1999]; Chong et al., Rap. Comm. Mass Spec., 13:1808 [1999]). This method provides for exceptionally fast and reproducible high-resolution separations of proteins according to their hydrophobicity and molecular weight. The non-porous (NP) silica packing material used in these reverse phase (RP) separations eliminates problems associated with porosity and low recovery of larger proteins, as well as reducing analysis times by as much as one third. Separation efficiency remains high due to the small diameter of the spherical particles, as does the loadability of the NP RP HPLC columns. However, the second dimension may employ any number of separation techniques. For example, 1-D SDS PAGE lane gel is used. Having the second dimension conducted in the liquid phase facilitates efficient analysis of the separated proteins and enables products to be fed directly into additional analysis steps (*e.g*., directly into mass spectrometry analysis).

Proteins obtained from the second separation step may be mapped using software (available from Dr. Stephen J. Parus, University of Michigan, Department of Chemistry, 930 N. University Ave., Ann Arbor, MI 48109-1055) in order to create a protein pattern analogous to that of the 2-D PAGE image-although based on the two physical properties used in the two separation steps rather than by a second gel-based size separation technique. RP HPLC peaks are represented by bands of different intensity in the 2-D image, according to the intensity of the peaks eluting from the HPLC. Peaks can be collected as the eluent of the HPLC separation in the liquid phase.

In some embodiments, the proteins collected from the second dimension were identified using proteolytic enzymes, MALDI-TOF MS and MSFit database searching. In an example using human erythroleukemia cell lysate, using IEF-NP RP HPLC, approximately 700 bands were resolved in a pI range from 3.2 to 9.5 and 38 different proteins with molecular weights ranging from 12 kDa to 75 kDa were identified. In comparison to a 2-D gel separation of the same human erythroleukemia (HEL) cell line lysate, the IEF-NP RP HPLC produced improved resolution of low mass and basic proteins. In addition, the proteins remained in the liquid phase throughout the separation, thus making the entire procedure highly amenable to automation and high throughput.

Certain preferred embodiments are described in detail below. Although the examples are described using human tissues and samples, the methods of the present invention can be used with any desired protein samples including samples from plants and microorganisms.

### A. IEF-NP RP HPLC Method

The following description provides certain preferred embodiments for conducting isoelectric separation (first dimension) and NP RP HPLC separation (second dimension) according to the methods of the present invention.

### 1. IEF Separation

Proteins are extracted from cells using a lysis buffer. To facilitate an efficient process, this lysis buffer should be compatible with the downstream separation and analysis steps (e.g., NP RP HPLC and MALDI-TOF-MS) to allow direct use of the products from each step into subsequent steps. Such a buffer is an important aspect of automating the process. Thus, the preferred buffer should meet two criteria: 1) it solubilizes proteins and 2) it is compatible with each of the steps in the separation/analysis methods. Although the present invention provides suitable buffers for use in the particular method configurations described below, one skilled in the art can determine the suitability of a buffer for any particular configuration by solubilizing protein sample in the buffer. If the buffer solubilizes the protein, the sample is run through the particular configuration of separation and detection methods desired. A positive result is achieved if the final step of the desired configuration produces detectable information (e.g., ions are detected in a mass spectrometry analysis). Alternately, the product of each step in the method can be analyzed to determine the presence of the desired product (e.g., determining whether protein elutes from the separation steps).

After extraction in the lysis buffer, proteins are initially separated in a first dimension. The goal in this step is that the proteins are isolated in a liquid fraction that is compatible with subsequent NP RP HPLC and mass spectrometry steps. In these embodiments, n-octyl β-D-glucopyranoside (OGI, from Sigma) is used in the buffer n-octyl β-D-glucopyranoside is one of the few detergents that is compatible with both NP RP HPLC and subsequent mass spectrometry analyses. It is contemplated that detergents of the formula n-octyl SUGARpyranoside find use in these embodiments. The lysis buffer utilized was 6M urea, 2M thiourea, 1.0 % n-octyl ß-D-glucopyranoside, 10 mM dithioerythritol and 2.5 % (w/v) carrier ampholytes (3.5 to 10 pI)). After extraction, the supernatant protein solution is loaded to a device that can separate the proteins according to their pI by isoelectric focusing (IEF). Here the proteins are solubilized in a running buffer that again should be compatible with NP RP HPLC. A suitable running buffer is 6M urea, 2M thiourea, 0.5 % n-octyl ß-D-glucopyranoside, 10 mM dithioerythritol and 2.5 % (w/v) carrier ampholytes (3.5 to 10 pI).

Three exemplary devices that may be used for this step are:

### a) Rotofor

This device (Biorad) separates proteins in the liquid phase according to their pI *(See e.g.,* Ayala et al., Appl. Biochem. Biotech. 69:11 [1998]). This device allows for high protein loading and rapid separations that require only four to six hours to perform. Proteins are harvested into liquid fractions after a 5-hour IEF separation. These liquid fractions are ready for analysis by NP RP HPLC. This device can be loaded with up to 1 g of protein.

### b) Carrier Ampholyte based slab gel IEF separation with a whole gel eluter

In this case the protein solution is loaded onto a slab gel and the proteins separate in to a series of gel-wide bands containing proteins of the same pI. These proteins are then harvested using a whole gel eluter (WGE, from Biorad). Proteins are then isolated in liquid fractions that are ready for analysis by NP RP HPLC. This type of gel can be loaded with up to 20 mg of protein.

### c) IPG slab gel IEF separation with a whole gel eluter

Here the proteins are loaded onto a immobiline pI gradient slab gel and separated into a series of gel-wide bands containing proteins of the same pI. These proteins are electro-eluted using the WGE into liquid fractions that are ready for analysis by NP RP HPLC. The IPG gel can be loaded with at least 60 mg of protein.

### 2. Protein Separation by NP RP HPLC

Having obtained liquid fractions containing large amounts of pI-focused proteins, the second dimension separation is non-porous RP HPLC. The present invention provides the novel combination of employing non-porous RP packing materials (Eichrom) with another RP HPLC compatible detergent (*e.g.,* n-octyl β-D-galactopyranoside) to facilitate the multi-phase separation of the present invention. This detergent is also compatible with mass spectrometry due to its low molecular weight. The use of these types of RP HPLC columns for protein separations as a second dimension separation after IEF in order to obtain a 2-D protein separation is a novel feature of the present invention. These columns are well suited to this task as the non-porous packing they contain provides optimal protein recovery and rapid efficient separations. It should be noted that though several detergents have been mentioned thus far for increasing protein solubility while being compatible with RP HPLC there are many other different low molecular weight non-ionic detergents that could be used for this purpose. Several important features that allow the RP HPLC to work as a second dimension are as follows: The mobile phase should contain a low level of a non-ionic low molecular weight detergent such as n-octyl β-D-glucopyranoside or n-octyl β-D-galactopyranoside as these detergents are compatible with RP HPLC and also with later mass spectrometry analyses (unlike many other detergents); the column should be held at a high temperature (around 60°C); and the column should be packed with non-porous silica beads to eliminate problems of protein recovery associated with porous packings.

### 3. Protein Detection and Identification via Mass Spectrometry

In some embodiments of the present invention, the products of the second separation step are further characterized using mass spectrometry. For example, the proteins that elute from the NP RP HPLC separation are analyzed by mass spectrometry to determine their molecular weight and identity. For this purpose the proteins eluting from the separation can be analyzed simultaneously to determine molecular weight and identity. A fraction of the effluent is used to determine molecular weight by either MALDI-TOF-MS or ESI oa TOF (LCT, Micromass) *(See e.g.,* U.S. Pat. No. 6,002,127). The remainder of the eluent is used to determine the identity of the proteins via digestion of the proteins and analysis of the peptide mass map fingerprints by either MALDI-TOF-MS or ESI oa TOF. The molecular weight 2-D protein map is matched to the appropriate digest fingerprint by correlating the molecular weight total ion chromatograms (TIC's) with the UV-chromatograms and by calculation of the various delay times involved. The UV-chromatograms are automatically labeled with the digest fingerprint fraction number. The resulting molecular weight and digest mass fingerprint data can then be used to search for the protein identity via web-based programs like MSFit (UCSF).

### 4. Automation

All of the above described steps are automated, for example, into one discrete instrument. In one illustrative embodiment, the first dimension is carried out by a Rotofor, with the harvested liquid fractions being directly applied to the second dimension non-porous RP HPLC apparatus through the appropriate tubing. The products from the second dimension separation are then scanned and the data interpreted and displayed as a 2-D representation using the appropriate computer hardware and software. Alternately, the products from the second dimension fractions are sent through the appropriate microtubing to a mass spectrometry pre-reaction chamber where the samples are treated with the appropriate enzymes to prepare them for mass spectrometry analysis. The samples are then analyzed by mass spectrometry and the resulting data is received and interpreted by a processor. The output data represents any number of desired analyses including, but not limited to, identity of the proteins, mass of the proteins, mass of peptides from protein digests, dimensional displays of the proteins based on any of the detected physical criteria (e.g., size, charge, hydrophobicity, etc.), and the like. In preferred embodiments, the proteins samples are solubilized in a buffer that is compatible with each of the separation and analysis units of the apparatus. Using the automated systems of the present invention provides a protein analysis system that is an order of magnitude less expensive than analogous automation technology for use with 2-D gels (*See e.g.,* Figeys and Aebersold, J. Biomech. Eng. 121:7 [1999]; Yates, J. Mass Spectrom., 33:1 [1998]; and Pinto et al., Electrophoresis 21:181 [2000]).

### 5. Software and Data Presentation

The data generated by the above listed techniques may be presented as 2-D images much like the traditional 2-D gel image. may be the chromatograms, TIC's or integrated and deconvoluted mass spectra converted to ASCII format and then plotted vertically, using a 256 step gray scale, such that peaks are represented as darkened bands against a white background. The scale could also be in a color format. The image generated by this method provides information regarding the pI, hydrophobicity, molecular weight and relative abundance of the proteins separated. Thus the image represents a protein pattern that can be used to locate interesting changes in cellular protein profiles in terms of pI, hydrophobicity, molecular weight and relative abundance. Naturally the image can be adjusted to show a more detailed zoom of a particular region or the more abundant protein signals can be allowed to saturate thereby showing a clearer image of the less abundant proteins. This information can be used to assess the impact of disease state, pharmaceutical treatment, and environmental conditions. As the image is automatically digitized it may be readily stored and used to analyze the protein profile of the cells in question. Protein bands on the image can be hyper-linked to other experimental results, obtained via analysis of that band, such as peptide mass fingerprints and MSFit search results. Thus all information obtained about a given 2-D image, including detailed mass spectra, data analyses, and complementary experiments (*e.g.,* immuno-affinity and peptide sequencing) can be accessed from the original image.

The data generated by the above-listed techniques may also be presented as a simple read-out. For example, when two or more samples are compared (*See,* Section J, below), the data presented may detail the difference or similarities between the samples (e.g., listing only the proteins that differ in identity or abundance between the samples). In this regard, when the differences between samples (e.g., a control sample and an experimental sample) are indicative of a given condition (e.g., cancer cell, toxin exposure, etc.), the read-out may simply indicate the presence or identity of the condition. In one embodiment, the read-out is a simple +/- indication of the presence of particular proteins or expression patterns associated with a specific condition that is to be analyzed.

### 6. IEF-NP RP HPLC in Operation

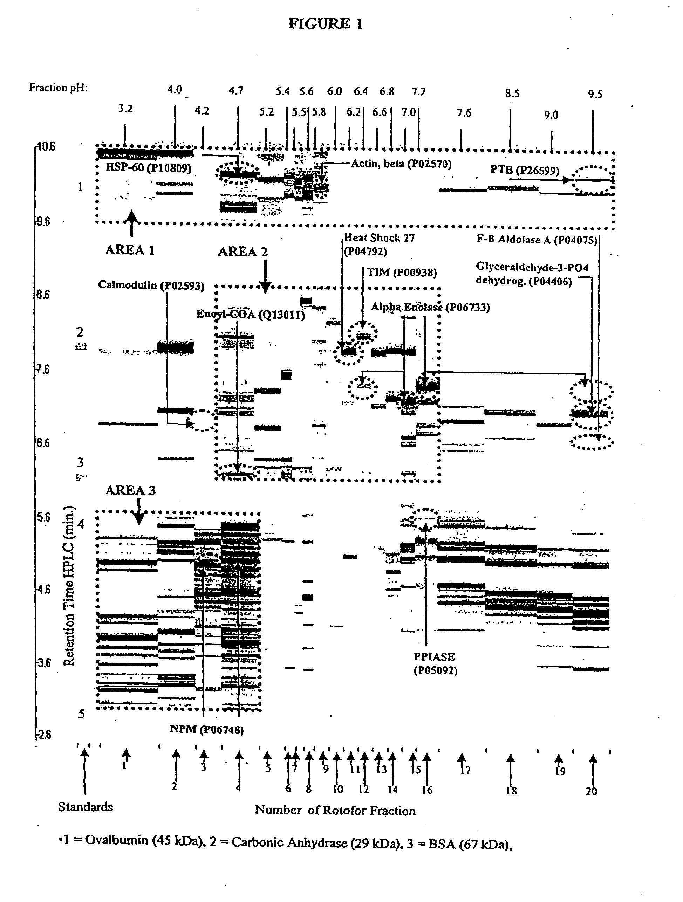

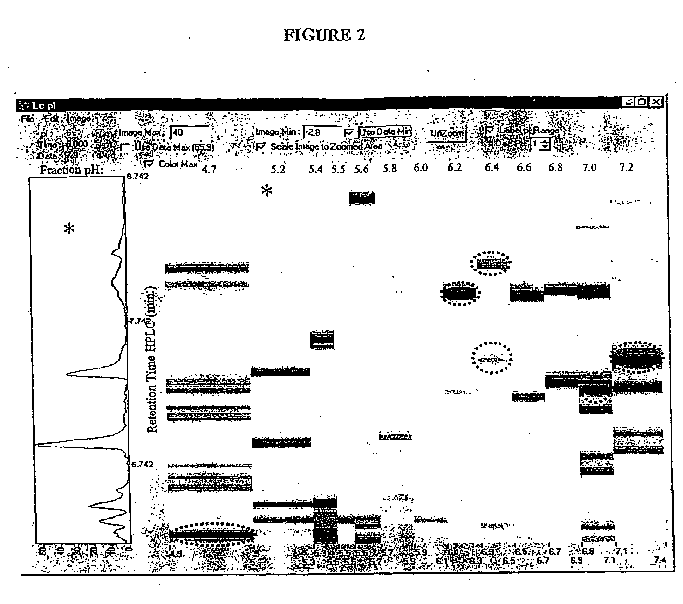

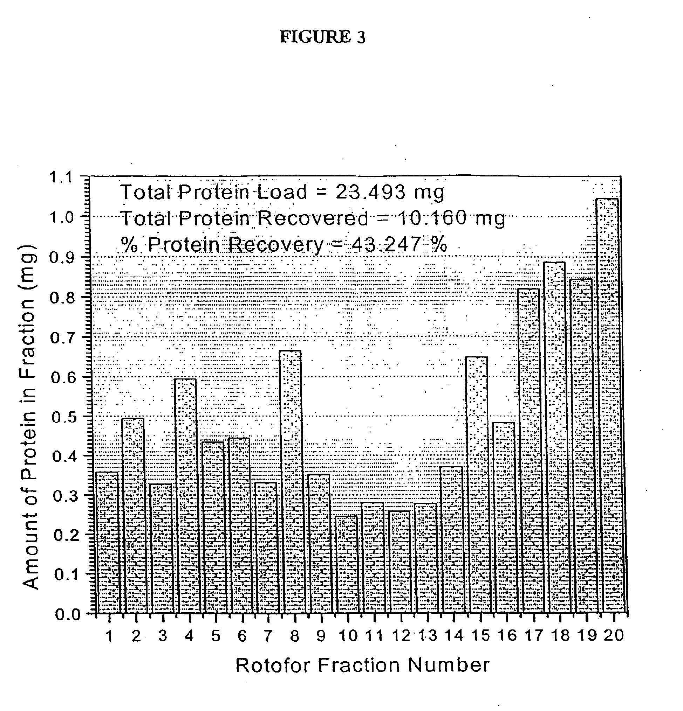

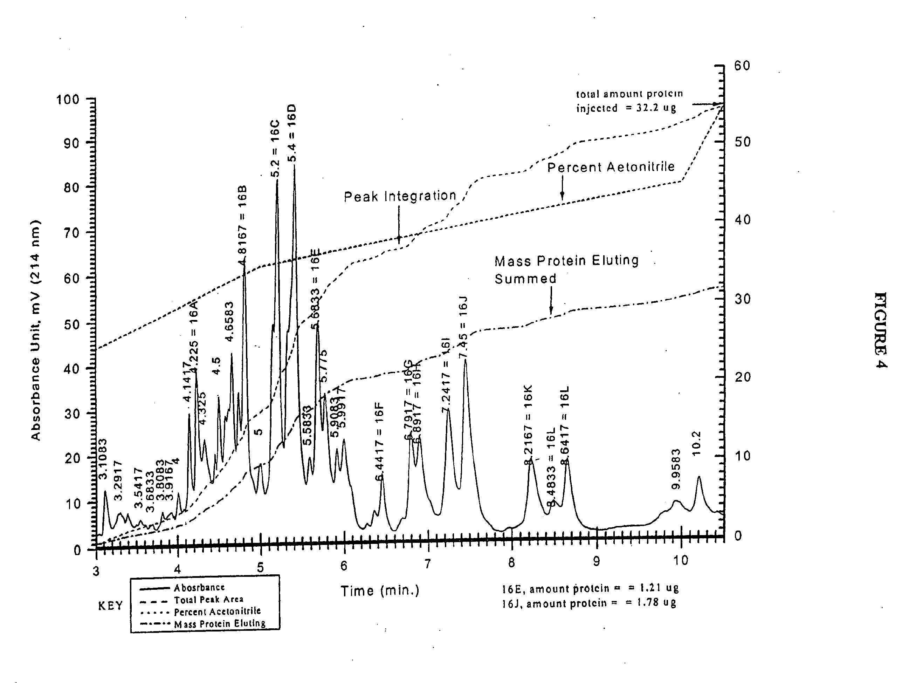

The IEF-NP RP HPLC image shown in Figure 1 is a digital representation of a 2-dimensional separation of a whole cell protein lysate from a human erythroleukemia (HEL) cell line. This image is designed to offer the same advantages of pattern recognition and protein profiling that may be obtained using a 2-D gel. The horizontal and vertical dimensions are in terms of isoelectric point and protein hydrophobicity, respectively. The isoelectric focusing step, performed using the Rotofor, resulted in 20 protein fractions ranging in pH from 3.2 to 9.5. These fractions were then injected onto a non-porous reversed phase column for separation by HPLC and detection by UV absorbance (214 nm). The resulting chromatograms were converted to ASCII format and then plotted vertically, using a 256 step gray scale, such that peaks are represented as darkened bands against a white background. Protein profiles may be viewed in greater detail by using the zoom feature as shown in Figure 2 and/or by selecting a particular Rotofor fraction and observing the NP RP HPLC chromatogram as shown in the left panel of Figure 2. The zoom and chromatogram image features provide a means to observe details in band patterns that may not be observable in the original image *(See,* Figure 1). In addition, because of the limitations of the 256 step gray scale representation the band intensities in areas 1, 2 and 3 of Figure 1 were rescaled by a factor of 3 to better show the low abundance proteins. This was preferred since the presence of several high abundance protein bands may cause low intensity bands in some regions to be undetected. In Figure 1, the total peak area for each individual chromatogram was scaled to reflect the relative amount of protein that was found in the original Rotofor fraction *(See,* Figure 3). The band intensities in different chromatograms can therefore be compared directly thus providing a true image of relative protein abundance in the cell lysate. The width of the Rotofor fraction columns was adjusted to represent their estimated pH range. The molecular weight of proteins observed by IEF-NP RP HPLC ranged from 12 kDa to 75 kDa. Typical NP RP HPLC separations, as shown in Figure 4, resulted in 35 peaks in 10.5 minutes. The total number of peaks that could be observed from all 20 fractions is estimated to be approximately 700.

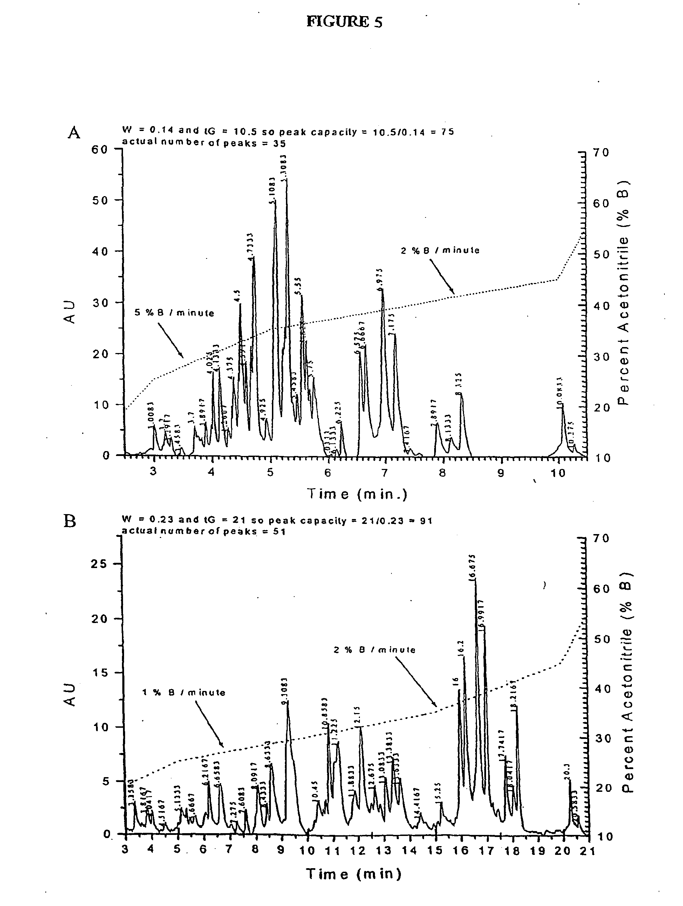

The gradient time (t_{G}) used in the above experiments is very short and a significant increase in peak capacity is expected with longer gradients. This is shown using Rotofor fraction 17 where two separations were performed with gradient times of 10.5 minutes (See, Figure 5A) and 21 minutes (See, Figure 5B). With t_{G} =10.5 minutes, the average peak width was 0.14 minutes and the peak capacity was therefore 75. The actual number of peaks resolved was 35. With t_{G} = 21 minutes the average peak width was 0.23 minutes and the peak capacity was therefore 91. The actual number of peaks resolved was 51. Using the longer separation time with t_{G} = 21 minutes the total number of peaks observed should increase from 700 to 1000. However, it should be noted that when using mass spectrometric detection, that sufficient resolution should be available to ultimately resolve the same number of peaks without using a longer gradient time.

The proteins in a representative sampling of these peaks were identified using the traditional approach of enzymatic digestion, MALDI-TOF MS peptide mass analysis and MSFit database searching. The magnification of the IEF-NP RP HPLC image enables the viewer to perceive more bands than is possible to observe from the whole image. In addition, as shown in Figure 2, the viewer may select a particular band format chromatogram and observe the traditional peak format of the chromatogram in a window to the left of the image. This allows the observer to use the peak format chromatogram to find partially resolved peaks that may not be observable in the band format chromatogram. Five standard protein bands are shown in the left-most column where the masses range from 14.2 kDa up to 67 kDa. As RP HPLC separates proteins by hydrophobicity, these standards are not molecular weight markers as in a traditional 1-D gel. Rather, they are used to indicate the range of protein molecular weights that may be observed. Ten different proteins are labeled on the image although many more proteins were identified as shown in Table 1, below. In some embodiments of the present invention, where it is desired that certain proteins or classes of proteins are to be detected, the starting protein sample may be selectively labeled. After the proteins are passed through the separation step, detection of the proteins can be limited to those that contain the selective label.

### B. Protein Separation by 2-D SDS PAGE

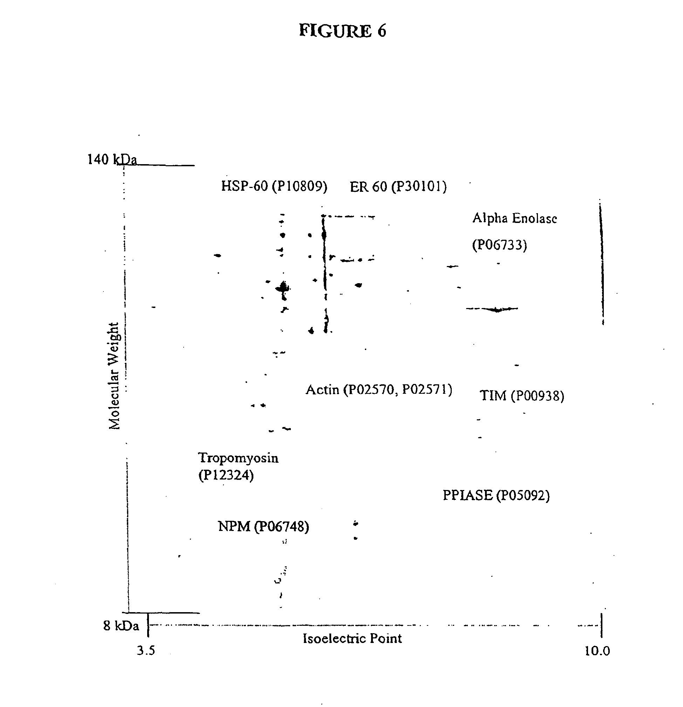

The image in Figure 1 represents the IEF-NP RP HPLC separation of the HEL cell protein lysate and the image in Figure 6 represents the Coomassie blue (CBB) stained 2-D SDS PAGE separation of the same HEL cell line lysate. The pI range for this gel is the same as that used for the Rotofor separation and the molecular weight range is from 8 kDa to 140 kDa. As with the IEF-NP RP HPLC separation a representative sampling of the isolated proteins was identified using enzymatic digestion, MALDI-TOF MS and MSFit methods (*See e.g.,* Rosenfeld et al., Anal. Biochem. 203:173 [1992]). For the target protein mass range of this study (10 kDa - 70 kDa) approximately 188 protein spots are observed on the CBB stained gel, 355 from the CBB stained polyvinylidene difluoride (PVDF) blot, and 652 from the silver stained gel as estimated using BioImage 2D Analyzer Version 6.1 software (Genomic Solutions). The total spot capacity for the 2-D gel separation is estimated to be 2100. The proteins identified from the gel are labeled on the image and also shown in Table 2, below. An image of another 2-D gel separation of HEL cell proteins can be observed via the Swiss-2D PAGE database (*See e.g*., http://www.expasy.ch; Sanchez et al., Electrophoresis 16:1131 [1995]). In addition, it is possible to view the latest protein list for the HEL cell in which 19 protein entries are shown *(See e.g.,* http://www.expasy.ch/cgi-bin/get-ch2d-table.pl).

**Table 1. Thirty Eight Proteins Identified From HEL Cell IEF-NP RP HPLC Separation**

| Rotofor | - | Retention | Enzyme* | MWt I pl: database | Swiss. *NCBlnr* | Protein Name |
|---|---|---|---|---|---|---|
| Fraction | pH | Time (min.) | | calculated | Accession # | |
| 3 | 4.20 | 5.34 | trypsin | 31575.2/4.64 | P06748 | NPM |
| 3 | 4.20 | 6.20 | trypsin | 11665.0/4.41 | P05387 | 60S RIBOSOMAL PROTEIN P2 |
| 3 | 4.20 | 6.91 | trypsin | 16837.7/4.09 | P02593 | CALMODULIN |
| 3 | 4.20 | 10.15 | trypsin | 41737.0 / 5.29 | P02570 | BETA-ACTIN & GAMMA ACTIN |
| 3 | 4.20 | 10.25 | trypsin | 61055.0/5.70 | P10809 | HSP-60 |
| 4 | 4.70 | 5.38 | trypsin | 32575.2./4.64 | P06748 | NPM |
| 4 | 4.70 | 6.24 | trypsin | 35994.6/6.61 | O13011 | ENOYL-COA HYDRATASE |
| 4 | 4.70 | 7.07 | trypsin | 57914.2/7.95 | P14786 | PYRUVATEKINASE, M2 |
| 4 | 4.70 | 10.28 | trypsin | 61055.0/5.70 | P10809 | HSP-60 |
| 5 | 5.40 | 4.93 | trypsin | 22988.1/5.10 | P52566 | RHO GDI 2 |
| 5 | 5.40 | 10.15 | trypsin | 70898.4/5.38 | P11142 | HEAT SHOCK COGNATE 71 KD PROTEIN |
| 8 | 5.60 | 4.99 | trypsin | 22988.1/5.10 | P52566 | RHO GDP-DISSOCIATION INHIBITOR 2 |
| 8 | 5.60 | 7.94 | trypsin | 69224.5/5.49 | P23588 | EIF-4B |
| 8 | 5.60 | 10.35 | trypsin | 49831.3/4.79 | P05217 | TUBUUN BETA-2 CHAIN |
| 9 | 5.80 | 6.90 | trypsin | 56782./5.99 | P30101 | ERP60 |
| 9 | 5.80 | 8.05 | trypsin | 17148.8/5.83 | P15531 | METASTASIS INHIBITION FACTOR NM23 |
| 9 | 5.80 | 8.50 | trypsin | 26669.6/6.45 | P00988 | TRIOSEPHOSPHATE ISOMERASE (TIM) |
| 9 | 5.80 | 10.15 | trypsin | 41737.0/5.29 | P02570 | BETA-ACTIN&GAMMA ACTIN |
| 11 | 6.20 | 5.62 | trypsin | 36926.7 /6.37 | 5542020 | (L32610) ribonucleoprotein |
| 11 | 6.20 | 7.65 | trypsin | 33777.2/6.26 | 4885153 | (X59656)CRKL |
| 11 | 6.20 | 7.91 | trypsin | 22327.3/7.83 | P04792 | HEAT SHOCK 27 |
| 11 | 6.20 | 8.80 | trypsin | 74674.0/8.51 | O92935 | EXOSTOSIN-L |
| 11 | 6.20 | 9.22 | trypsin | 37374.9/5.85 | P19883 | FOLUSTATIN AND 2 PRECURSOR |
| 11 | 6.20 | 10.40 | trypsin | 47033.1/5.30 | 5032183 | cargo selection protein TIP47 |
| 12 | 6.40 | 5.08 | trypsin | 13802.0/6.43 | P49773 | HINT |
| 12 | 6.40 | 5.90 | trypsin | 70021.3/5.56 | P54652 | HEAT SHOCK 70 KD PROTEIN 2 |
| 12 | 6.40 | 7.48 | trypsin | 47169.217.01 | P06733 | ALPHA ENOLASE |
| 12 | 6.40 | 8.12 | trypsin | 26669.6/6.45 | P00938 | TPJOSEPHOSPHATE ISOM ERASE (TIM) |
| 13 | 6.60 | 4.88 | trypsin | 48058.0 / 5.34 | P05783 | KERATIN, TYPE I CYTOSKELETAL 18 |
| 13 | 6.60 | 8.28 | trypsin | 62639.6/6.40 | P31248 | TPANSFORMATION-SENSITIVE PROTEIN |
| 13 | 6.60 | 8.65 | trypsin | 34902.4/7.42 | 4505059 | carcinoma-associated antigen GA733-2 |
| 15 | 7.00 | 4.70 | tryptin | 37429.9 / 8.97 | P22626 | NUCLEAR RIBONUCLEOPROTEINS A2/B1 |
| 15 | 7.00 | 8.70 | trypsin | 11391.6/8.41 | P37802 | SM22-ALPHA HOMOLOG |
| 15 | 7.00 | 7.25 | trypsin | 47169.2/7.01 | P06733 | ALPHA ENOLASE |
| 16 | 7.20 | 5.68 | trypsin. Glu-C(E) | 18012.6/7.68 | P05097 | PPIASE |
| 16 | 7.20 | 6.89 | trypsin | 35940.7/7.18 | P01861 | IG GAMMA-4 CHAIN C REGION |
| 16 | 7.20 | 7.24 | trypsin | 36053.4/8.57 | P04406 | GLYCERALDEHYDE 3-PHOSPHATE |
| 16 | 7.20 | 7.45 | trypsin, Glu-C(E) | 47169.2/7.01 | P06733 | ALPHA ENOLASE |
| 16 | 7.20 | 8.64 | trypsin, Glu-C(E) | 22391.6/8.41 | P37802 | SM22-ALPHA HOMOLOG |
| 19 | 8.00 | 4.88 | trypsin | 38846.0/9.27 | P09651 | NUCLEAR RIBONUCLEOPROTEIN A1 |
| 19 | 9.00 | 5.13 | trypsin | 37429.9/8.97 | P22626 | NUCLEAR RIBONUCLEOPROTEINS A2/B1 |
| 19 | 9.00 | 5.85 | trypsin | 46987.1/7.58 | P13929 | BETA ENOLASE |
| 19 | 8.00 | 7.47 | trypsin | 36053.4/8.57 | P04406 | GLYCERALDEHYDE 3-PHOSPHATE |
| 19 | 9.00 | 8.70 | trypsin | 38604.2/7.58 | P07355 | ANNEXIN II |
| 19 | 9.00 | 9.07 | trypsin | 22391.6/8.41 | P37802 | SM22-ALPHA HOMOLOG |
| 19 | 9.00 | 10.53 | trypsin | 57221.6/9.22 | P26599 | PTB. NUCLEAR RIBONUCLEOPROTON 1 |
| 20 | 9.50 | 4.46 | trypsin, Glu-C(E) | 38846.0/9.26 | P09651 | NUCLEAR RIBONUCLEOPROTEIN A1 |
| 20 | 9.50 | 4.67 | trypsin, Glu-C (E) | 37429.9/8.97 | P22626 | NUCLEAR RIBONUCLEOPROTEINS A2/B I |
| 20 | 9.50 | 6.72 | trypsin, Glu-C(E) | 39420.2/8.30 | P04075 | FRUCTOSE-BISPHOSPHATE ALDOLASE A |
| 20 | 9.50 | 7.06 | trypsin | 36053.4/8.57 | P04406 | GLYCERALDEHYDE 3-PHOSPHATE |
| 20 | 9.50 | 7.39 | trypsin, Glu-C (E) | 47169.2/7.01 | P06733 | ALPHA ENOLASE |
| 20 | 9.50 | 8.52 | trypsin. Glu-C (E) | 22391.6/8.41 | P37802 | SM22-ALPHA HOMOLOG |
| 20 | 9.50 | 10.16 | trypsin | 44728.1/8.30 | P00558 | PHOSPHOOLYCEILATE KINASE 1 |
| 20 | 9.50 | 10.35 | trypsin | 57221.61922 | P26599 | PTB. NUCLEAR RIBONUCLEOPROTEIN 1 |

| | | | | | | |
|---|---|---|---|---|---|---|
| * Note that all proteins labelled only with trypsin were not digested with Glu-C (E) | | | | | | |

**Table 2. Nine Proteins Identified From HEL Cell CBB 2-D Gel**

| Gel Spot I.D. | Enzyme | MWt/pl: database | SwissProt | Protein Name |
|---|---|---|---|---|
| Number | | calculated | Accession # | |
| gl | trypsin | 18012.6/7.68 | P05092 | PPIASE |
| g2 | trypsin | 26669.6/6.45 | P00938 | TRIOSEPHOSPHATE ISOMERASE (TIM) |
| g3 | trypsin | 26669.6 / 6.45 | P00938 | TRIOSEPHOSPHATE ISOMERASE (TIM) |
| g8 | trypsin | 29032.8 /4.75 | P12324 | TROPOMYOSIN, CYTOSKELETAL TYPE (TM30-NM) |
| g10 | trypsin | 32575.2/4.64 | P06748 | NPM |
| g11 | trypsin | 41737.0/5.29 | P02570 | BETA-ACTIN |
| g12 | trypsin | 61055.0/5.70 | P10809 | HSP-60 |
| g13 | trypsin | 56782.715.99 | P30101 | ERP60 |
| g14 | trypsin | 47169.2/7.01 | P06733 | ALPHA ENOLASE |

### C. DW-NP RP HPLC versus 2-D SDS PAGE: Protein Loading and Quantification

Each separation method relies upon orthogonal mechanisms of separation generating a large number of isolated proteins. Protein profiles may be compared in terms of their pattern as well as the relative amounts of isolated proteins. It is shown, however, that the loadability of the liquid phase methods of the present invention greatly surpasses that of the gel phase.

The limit of detection for the gel method when stained with the silver stain is approximately 1 to 10 ng. The Coomassie blue stain can detect 100 ng of protein and the amount of protein in the spot can be quantified over 2.5 orders of magnitude. For the NP RP HPLC of standard proteins used in the methods of the present invention, the limit of detection for the UV detector was 10 ng. The protein in the peak can be quantified from 10 ng up to 20 µg providing 3.1 orders of magnitude. Quantification of an HPLC peak involves integrating the peak to find the area. For the gel, the spots must first be digitized and then this image must be analyzed to determine the integrated optical density of each spot of interest. The sensitivity of the UV detector in embodiments of the present invention utilizing HPLC is competitive with the silver stain and quantification is much simpler. The limits of detection for both the silver stained gel and the HPLC UV peak detection are mass dependent. For the gel, resolution and sensitivity are proportional to the molecular weight of the protein. For IEF-NP RP HPLC, the resolution and sensitivity are inversely proportional to the molecular weight of the protein. The gel appears to provide improved results for both acidic proteins and proteins above 50 kDa whereas IEF-NP RP HPLC performs better with proteins in the basic region and proteins that are below 50 kDa *(See e.g.,* Figure 1 and Figure 6). These results show the complementary nature of these two techniques where the gel and IEF-NP RP HPLC each provide important information of protein content.

In one experiment using the methods of the present invention, 23.5 mg of protein was loaded into the Rotofor, and after a five-hour IEF separation period fractions ranging from 2 to 4 mL were collected into polypropylene microtubes. The amount of protein in the individual fractions ranged from 0.25 mg to 1.05 mg. Summing the amounts of protein in each fraction led to the determination that a total of 10.2 mg of protein was recovered from the Rotofor. This amount can be increased by increasing the amount of non-ionic detergent in the Rotofor buffer above the current 0.1 % level as well as by the addition of thiourea. In contrast, the amount of protein loaded on the 2-D gel in Figure 6 is 200 µg. The amount of protein that actually makes it through the gel and focuses to a spot has not been quantified, relative to the amount of protein that is actually loaded on the gel, though it is known that many hydrophobic proteins are lost during the separation (Herbert, Electrophoresis 20:660 [1999]). The amount of protein that may theoretically be loaded on a gel ranges from 5 µg up to 250 µg whereas for IEF-NP RP HPLC the initial loading of protein may be as high as 1 gram. The amount of protein actually used to produce the separation shown in Figure 1 is only a fraction of the amount initially loaded into the Rotofor. The image in Figure 1 actually represents the separation of a total of 1 to 2 mg of protein though 10.2 mg of protein was recovered from the Rotofor. The loading of the HPLC column being used currently could be increased though the peak capacity may suffer. Alternatively a larger column could be used in series with the smaller column to allow for higher loadability with no loss of separation efficiency *(See e.g.,* Wall et al., Anal. Chem., 71:3894 [1999]).

A 2-D gel provides a two dimensional separation from one initial loading of the cell lysate. The intensities of different spots on the same gel are representative of the relative protein abundances in the original lysate. However, in the IEF-NP RP HPLC methods of the present invention the proteins are loaded for the IEF and the HPLC separations so that the band intensities in the 2-D IEF-NP RP HPLC image depend on the amount of protein loaded to the HPLC from each Rotofor fraction. Since the amount of material in each Rotofor fraction is different, the total area of each chromatogram was scaled to represent the total amount of protein that was recovered for each Rotofor fraction (*See*, Figure 3). The result is that the protein band intensities can be compared both within the Rotofor fraction and between the different fractions.

In some embodiments of the present invention, 2-D gel techniques are used side-by-side with IEF-NP RP HPLC. In embodiments where specific proteins are desired for further characterization, the gel can provide information indicating which fraction obtained with IEF-NP RP HPLC contains the desired protein or proteins.

### D. Isoelectric Focusing: Liquid vs. Gel Phase

The principal concern with liquid phase IEF is that the protein is not isoelectrically focused as effectively as it would be in a gel due to diffusion of the protein in solution. In the case of -enolase, if one compares the liquid and gel phase images, it can be seen that in both cases substantial spreading of the protein occurs over a wide pI range. This range spans from pI 6.5 to pI 9.5 in both the liquid phase and the gel phase. For more acidic proteins such as β-actin, it appears that in the liquid phase the protein is more dispersed in the pI dimension than for the corresponding gel separated protein. Both methods provide a reasonably accurate assessment of the pI of the protein of interest. Referring to Table 1, it can be seen that as the Rotofor fraction pH increases, so generally does the pI of identified proteins therein. The pH of fraction 3 measures 4.2 and the proteins identified from this fraction range in,pI from 4.09 to 5.7. The pH of fraction 9 was 5.8 and the proteins identified from that fraction ranged from 5.29 to 6.45. The pH of fraction 16 was 7.2 and the pI range of proteins found there ranged from 7.01 to 8.93. The pI accuracy therefore ranges from +/- 0.65 to 1.73 pI units. This is comparable to the carrier ampholyte based gel. It should be noted that the pI of a given protein may vary significantly due to post-translational modifications such as phosphorylation and glycosylation, as well as to artifactual modifications such as carbamylation and oxidation.

### E. Second Dimension Liquid Separation

Fraction 16, Figure 4, may be used as an example of the quantification of isolated proteins. For fraction 16, the volume of injection was 160 µL. This means that if the concentration of protein was 201.4 µg/mL then the amount of protein loaded was 32.2 µg. The chromatogram was integrated using Microcal Origin software and the total area was determined to be 97.78. The areas of peaks 16E and 16J were 3.68 and 5.41 respectively. Dividing the peak area by the total area gives the fraction of protein represented by the peak. Therefore, if one assumes 100% protein recovery, the amount of PPIASE (16E, t_{R}= 5.68) in 16 was.(0.0376 * 32.2 µg) 1.21 µg and the amount of - enolase (16J, t_{R} = 7.45) was (0.0553 * 32.3 µg) 1.78 µg. The peak areas were generated by absorbance of 214 nm light at the amide bonds of the proteins and so should offer low selectivity thereby allowing for a good measure of the amount of protein in the peak regardless of the type of protein.

Figure 4 shows how the continuous integration of the chromatogram may be used to estimate the amount of protein isolated in a given peak. The peak area line is simply converted into mass units from which the observer can measure the change in the vertical mass axis that occurs over the width of the peak of interest. If one knows the initial concentration of protein in the cell lysate and the number of cells that were lysed, a quantitative comparison of different cell lysates can be made. This comparison is important to studying changes in protein expression levels due to some disease state or pharmacological treatment. In gel work, a technique used for protein quantification in different samples is to normalize the integrated optical density of the spot of interest to that of standard proteins whose expression levels are thought to be constant. In this way any experimental variation in spot intensity can be corrected. This same method is applied to the IEF-NP RP HPLC image to allow for reliable quantification of proteins of interest such that changes in expression level are quantitatively observed.

The assumption in these experiments is 100% protein recovery. One can determine the actual % recovery of protein and the dependence on elution time. Typical protein recoveries have been shown to range from 70 to 95% in NP RP HPLC (Wall et al., Anal. Chem., 71:3894 [1999]) and so, with a more likely percent recovery of 80%, the amount of PPIASE and -enolase in fraction 16 would be estimated to be 1.0 µg and 1.42 µg, respectively.

### F. Rotofor Fraction Analysis by NP RP HPLC vs. 1-D SDS PAGE

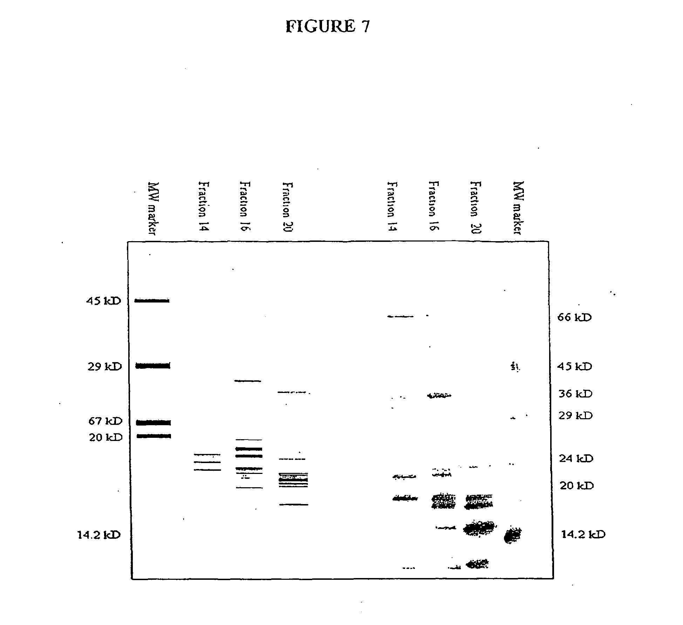

NP RP HPLC provides highly efficient protein separations *(See e.g.,* Chen et al., Rap. Comm. Mass Spec., 12:1994 [1998]; Wall et al., Anal. Chem., 71:3894 [1999]; and Chong et al., Rap. Comm. Mass Spec., 13:1808 [1999]), and is a far easier method to automate as compared to gels in terms of injection, data processing and protein collection. In addition the NP RP HPLC separations provided by the present invention are 70 times faster than the equivalent separation by 1-D SDS-PAGE, which requires 14 hours. In the experiments described above, the NP RP HPLC method has greater resolving power generating 35 bands where the 1-D gel generates only 26 bands. A direct comparison of the two methods, as shown in Figure 7, reveals that the NP RP HPLC bands are much narrower than those of the 1-D SDS PAGE over a similar molecular weight range. Also it is clear that as molecular weight decreases, the 1-D gel bandwidth increases substantially. In NP RP HPLC the opposite trend occurs where the lower molecular weight proteins show improved resolution and sensitivity. This image may appear to show that the NP RP HPLC separation fails with larger proteins, as there are few bands in the upper region of the image. However, this is not the case as it is important to remember that the vertical dimension for NP RP HPLC is not protein molecular weight but rather protein hydrophobicity. This is evidenced by the observation of the elution of bovine serum albumin (66 kDa), a relatively hydrophilic protein, half way up an image.

### G. Elution Time Prediction for Known Target Protein

One of the advantages of the 2-D gel is that the vertical coordinate of the gel may be used to estimate the molecular weight of the protein with a +/- 10% error. The position of a protein of interest can therefore be estimated before the protein is identified from the gel. In an attempt to correlate elution time in the methods of the present invention with the mass of the protein, a linear fit to a plot of percent acetonitrile at time of elution (%B) versus the log(MWt)/protein polar ratio was generated. The polar ratio (PR) is the number of polar amino acids divided by the total number of amino acids in the protein and the molecular weight is in kDa. The proteins used for this plot were four of the standards listed in Figure 1 as well as a sampling of six of the proteins from Table 1 (HSP60, β-actin, TIM, -enolase, PPIASE and glyceraldehyde-3-phosphate). The resulting equation (equation 1: %B/100 = 0.079805*(logMWt)/PR + 0.077686, (R = 0.9677, SD = 0.014722, N = 7)) is used to predict the elution time of target proteins. For HSP60, β-actin and -enolase the experimental elution times were 10.28, 10.15 and 7.25 respectively. The predicted elution times were 10.20, 10.13 and 9.78. In the cases of HSP60 and β-actin the prediction works well, whereas for -enolase the prediction is not as good. While not precise, this prediction does give some idea of when a protein will elute such that a given target protein, for which the molecular weight and hydrophobicity are known, can be found more readily.

### H. Protein Identification by Enzymatic Digestion, MALDI-TOF MS and MSFit Database Searching

The proteins that were identified from a representative sampling of the bands from the IEF-NP RP HPLC separation are listed in Table 1. A sampling of approximately 80 proteins from 12 of the Rotofor fractions were digested and their peptide mass maps successfully obtained by MALDI-TOF MS. Of these 80, 38 different proteins were identified. In this case, identifying roughly 50% of the proteins searched is to be expected as not all the proteins are in the available databases. Similar results were observed for proteins analyzed from 2-D gels of the HEL cell samples. The current table in Swiss-2D PAGE lists 19 protein entries for the HEL cell. Of these 19 proteins, five were identified from the IEF-NP RP HPLC separation. In the gel, these same five proteins were also identified.

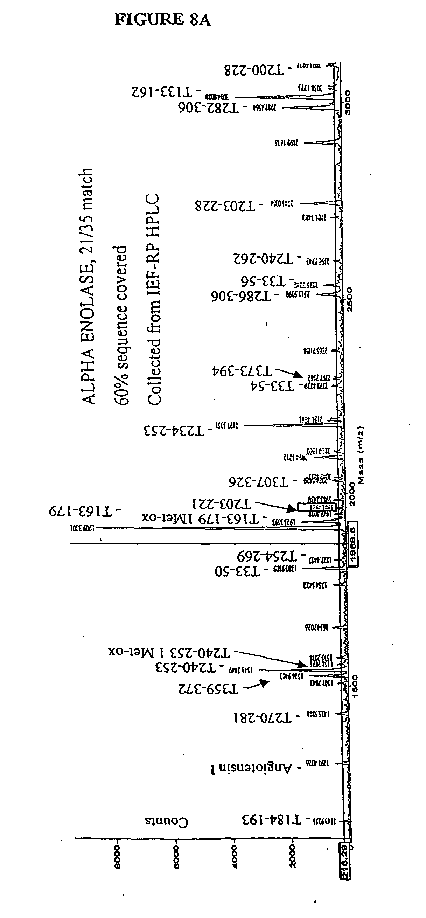

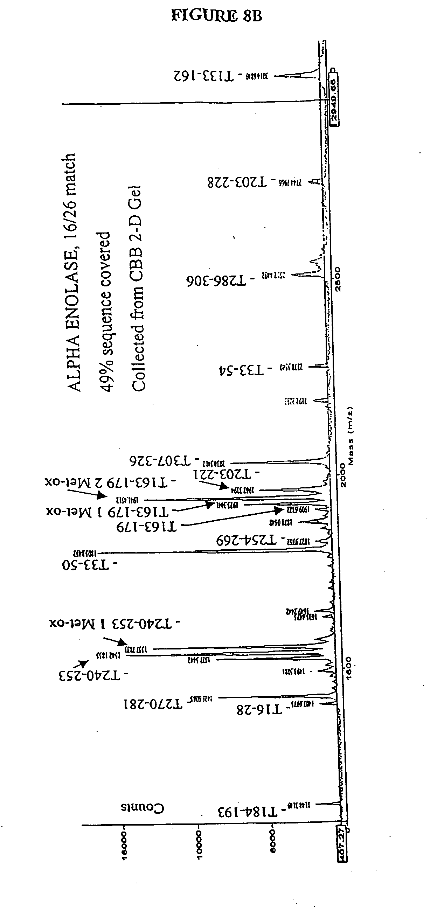

In general, it appears that the gel MSFit results are better than those from the liquid phase. This can be attributed to the fact that the gel proteins were reduced and alkylated with DTE and iodoacetamide respectively prior to the running of the second dimension. This step would help insure that all disulfide bonds are broken and optimal proteolysis is produced. Thus, this derivatization step can be added to the IEF-NP RP HPLC method, by performing the reduction and alkylation step prior to NP RP HPLC or during cell lysis. Nevertheless, in some cases the IEF-NP RP HPLC digestions surpassed those from the gel in coverage and quality. This is evidenced in Figure 8, which shows a direct comparison of the MALDI-TOF MS for -enolase as isolated via the IEF-NP RP HPLC method and the gel method. These mass spectra were calibrated externally at first and the mass profiles used to search the Swiss protein database with a mass accuracy of 400 ppm. These searches gave strong hits to -enolase for both the gel and the liquid protein digests. Each mass spectrum was then recalibrated internally using matched peptide peaks from the initial externally calibrated match. The new peak table was then used to search the same Swiss protein database but with 200 ppm mass accuracy. Figure 8 clearly shows that the digestion from the liquid phase is improved compared to that from the gel. The IEF-NP RP HPLC mass spectrum matches to 60% of the protein sequence whereas that from the gel matches to 49%. Achieving a match to 60% of the sequence of a 47 kDa protein is very unusual for MALDI-TOF MS analysis and represents a significant improvement over gel digests. Although the present invention is not limited to any particular mechanism, the increase in sequence coverage may be due to the fact that the protein is digested in the liquid phase, is relatively pure, and because the peptides are not lost due to being embedded inside the gel piece. Also if one observes the level of methionine oxidation in the peak that matches to T163-179, it is clear that the protein isolated by IEF-NP RP HPLC is far less oxidized than that from the gel.

Many of the NP RP HPLC chromatograms contain some peaks that are not fully resolved to baseline. This need not be a problem as partially resolved proteins can still be effectively identified using MALDI-TOF MS analysis. In Rotofor fraction 3 there are peaks at 10.15 minutes and 10.25 minutes (See, Table 1). These peaks are only resolved to 50% above the baseline and yet it is clear that the peak eluting at 10.15 minutes is β-actin and the peak eluting at 10.25 minutes is HSP-60. Note that the predicted elution times for these proteins are 10.13 and 10.20 minutes respectively. As proteins can be identified from partially resolved peaks, faster separations with more rapid gradients are possible. The reproducibility of the pattern of bands can be determined by looking at the retention times for particular proteins as observed from different Rotofor fractions. β-actin elutes at 10.1 minutes in both fractions 3 and 9; -enolase elutes at 7.25, 7.45 and 7.39 minutes in fractions 12, 16 and 20 respectively; and HSP-60 elutes at 10.28 and 10.25 minutes in fractions 3 and 4 respectively. Clearly, with +/- 0.1 minutes variation in the retention times, these separations are quite reproducible from run to run.

Thus, the methods of the present invention have been shown to provide advantageous methods for the reproducible separation of large numbers of proteins. In the human erythroleukemia cell lysate example, the methods are capable of resolving 700 bands with a rapid gradient, and 1000 bands with a longer gradient. There were 38 different proteins tentatively identified, by MALDI-TOF MS and MSFit database searching, after analysis of a fraction of these bands. This compares favorably with the 19 different proteins that have been identified to date from the 2-D gel. Some of the proteins found in the human erythroleukemia cell lysate; including -enolase (Rasmussen et al., Electrophoresis 19:818 [1998] and Mohammad et al., Enz. Prot., 48:37 [1994]), glyceraldehyde-3-phosphate dehydrogenase (Bini et al., Electrophoresis 18:2832 [1997] and Sirover, Biochim. Biophys. Acta 1432:159 [1999]), NPM (Redner et al., Blood 87:882 [1996]), CRKL (ten Hoeve et al., Oncogene 8:2469 [1993]), and heat shock protein (HS27) (Fuqua et al., Cancer Research 49:4126 [1989]), have been linked to various forms of cancer. NPM and CRKL have been linked specifically to leukemias.

The proteins identified in one exemplary experiment ranged from 12 kDa up to 75 kDa (although broader ranges are contemplated by the present invention); this range may include many of the proteins of interest to current research involving protein profiling, identification and correlation to some disease state or cell treatment. In sharp contrast to 2-D gels, this method is well-suited to automation. Mass spectrometric methods can be applied, such as ESI-MS and MALDI-TOF MS, to the detection of whole proteins and protein digests. Most importantly, the methods of the present invention provide an alternative 2-D protein map to the traditional 2-D gel and appears to improve results for lower mass proteins and more basic proteins. A key advantage of the liquid 2-D separation is that the end product is a purified protein in the liquid phase. Also, since the initial protein load can be fifty times that of the gel, the amount of a target protein that may be isolated by one IEF-NP RP HPLC separation is potentially fifty times higher than that obtainable from a 2-D gel separation. Additionally, in the case that the investigator is interested in specific proteins where the pI is known, this method may be used to isolate and identify the target protein in less than 24 hours, since only the fraction of interest need be analyzed via the second dimension separation. The gel-based method would require three days to achieve the same result.

### I. Identification of Novel Tumor Antigens

There is substantial interest in identifying tumor proteins that are immunogenic. Autoantibodies to tumor antigens and the antigens themselves represent two types of cancer markers that can be assayed in patient serum and other biological fluids. IEF-NP RP HPLC-MS has been implemented for the identification of tumor proteins that elicit a humoral response in patients with cancers. The identification of proteins that specifically react with sera from cancer patients was demonstrated using this approach. Solubilized proteins from a tumoral cell line are subjected to IEF-NP RP HPLC-MS. Individual fractions defined on the basis of pI range are subjected simultaneously to one-dimensional electrophoresis as well as to HPLC. Sera from cancer patients are reacted with Western blots of one-dimensional electrophoresis fractions. One band which reacted specifically with sera from lung cancer patients and not from controls was found to contain both Annexin II and aldoketoreductase. The ability to subfractionate further proteins contained in this fraction by HPLC led to the identification of Annexin II as the tumor antigen that elicited a humoral response in lung cancer patients.

### J. Comparative Analysis

As is clear from the above description, the methods of the present invention offer the opportunity to compare protein profiles between two or more samples (e.g., cancer vs. control cells, undifferentiated vs. differentiated cells, treated vs. untreated cells). In one embodiment of the present invention, the two samples to be compared are run in parallel. The data generated from each of the samples is compared to determine differences in protein expression between the samples. The profile for any given cell type may be used as a standard for determining the identity of future unknown samples. Additionally, one or more proteins of interest in the expression pattern may be further characterized (e.g., to determine its identity). In an alternative embodiment, the proteins from the samples are run simultaneously. In these embodiments, the proteins from each sample are separately labeled so that, during the analysis stage, the protein expression patterns from each sample are distinguished and displayed. The use of selective labeling can also be used to analyze subsets of the total protein population, as desired.

As is clear from the above description, the methods of the present invention provide a range of novel features that provide improved methods for analyzing protein expression patterns. For example, the present invention provides methods that combine IEF, resulting in pI-focused proteins in liquid phase fractions, with nonporous RP HPLC to produce 2-dimensional liquid phase protein maps. The data generated from such methods may be displayed in novel and useful formats such as viewing a collection of different pI NP RP HPLC chromatograms in one 2-D image displaying the chromatograms in a top view protein band format, not the traditional side view peak format. As shown in Figure 2, the side view peak format is shown to the left and the top view band format is shown to the right. The present invention also provides detergents that are compatible with automated systems employing multi-phase separation and detection steps.

The present invention provides additional characterization steps, including the identification of proteins separated by IEF-NP RP HPLC using enzymatic digestions and mass spectrometric analysis of the resulting peptide mass fingerprints. Proteins may be detected to determine their molecular weights by analyzing the effluent from the HPLC with either off-line collection to a MALDI plate (Perseptive) or on-line analysis using orthogonal extraction time-of-flight. The data generated from such methods may be displayed in novel and useful formats such as using the data from the MALDI or LCT generated protein molecular weights to generate total ion chromatograms (TIC) that would be virtually identical to the original UV-absorbance chromatograms. The signal of these chromatograms would be based on the number of ions generated from the HPLC effluent of a given group of pI-focused proteins, not by absorption of light. These chromatograms are plotted in the same 2-D top view band format as mentioned above. These methods allow one to fully integrate and deconvolute each of the TIC's generated to display complete mass spectra of each collection of pI-focused proteins. The methods also allow the display of all the integrated TIC's in one 2-D image where the vertical dimension is in terms of protein molecular weight and the horizontal dimension is in terms of protein pI. The protein mass spectra appears as bands as they are also viewed from the top. This image would therefore also contain quantitative information (in the case of the LCT) and so the bands would vary in intensity depending on the amount of protein present.

The liquid phase methods for protein mass mapping would also allow for collection of protein fractions to microtubes such that the proteins could be digested and the peptide mass maps analyzed to determine the identity of said proteins simultaneously. Laser induced fluorescence (LIF) detection schemes are used in conjunction with this method to increase the overall sensitivity by three orders of magnitude. The liquid phase LIF detector provides more sensitive fluorescence detection than in the gel as there would be no gel background fluorescence. This LIF detection method could be used in a number of ways including, but not limited to:
1) Combining equal amounts of two cell lysates that have each been previously stained with a different fluorescent dye followed by use of a dual fluorescence detector to simultaneously detect the same proteins from two different cell lysates. This would allow for very accurate comparisons of the relative amounts of proteins found for different cell lines or tissues; and
2) Using a fluorescently tagged antibody to label specific target proteins in a cell lysate such that they can be targeted for thorough analysis without looking at all the other proteins.

The methods of the present invention also offer an efficient system for combining with other analysis techniques to obtain a thorough characterization of a given cell, tissue, or the like. For example, the methods of the present invention may be used in conjunction with genetic profiling technologies (e.g., gene chip or hybridization based nucleic acid diagnostics) to provide a fuller understanding of the genes present in a sample, the expression level of the genes, and the presence of protein (*e.g.,* active protein) associated with the sample.

### III. Improved Elution Techniques Using Chromatofocusing

As described above, the present invention provides novel liquid chromatographic methods involving a 2-column 2-D separation of proteins from whole cell lysates followed by on-line mass mapping with by mass spectrometry (*e.g.*, using ESI-oaTOF MS as described in detail below). It is a 3-D protein analysis system as proteins are separated based upon, for example, their isoelectric points (pI) in the first LC dimension.

The present invention further provides novel techniques for eluting proteins from a separation apparatus (e.g., the first phase separation apparatus). The proteins eluted from the first dimension are peeled off from the column according to their pH, either one pH unit or fraction thereof, at a time-referred to as chromatofocusing (CF). These focused liquid fractions are then separated according to their hydrophobicity and size (or other desired properties) in the second dimension. Liquid fractions from, NP-RP-HPLC can be conveniently analyzed directly on-line using mass spectrometry (e.g., ESI-oaTOF) to obtain their molecular weight and relative abundance, which provides a third dimension. As a result, a virtual 2-D protein image is created and is analogous to a 2-D gel image. Furthermore, this 2-D protein image includes vital information such as the pI, hydrophobicity, molecular weight, and relative abundance. This Protein Peeling 2-D LC-MS method is a practical alternative to 2-D gels in order to study protein expression between normal and disease whole cell lysates, for example. This whole system can be fully automated and integrated into a single unit for rapid proteome analysis, providing a more accurate and less expensive automation technology compared to automation technologies for use with 2-D gels.

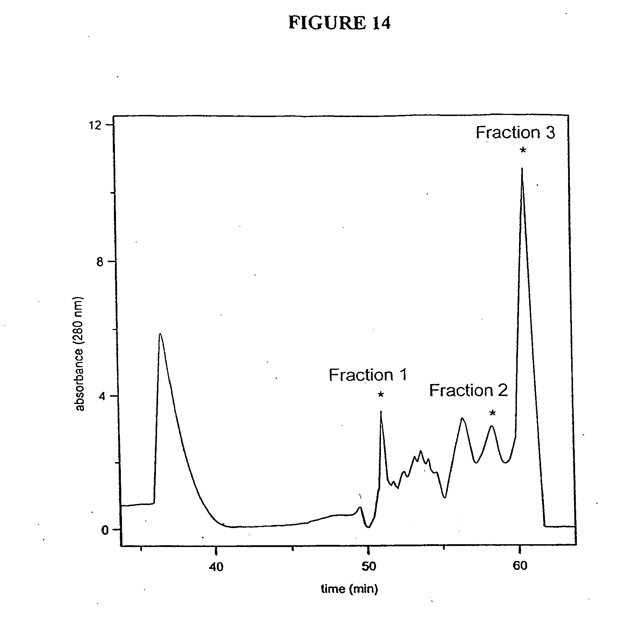

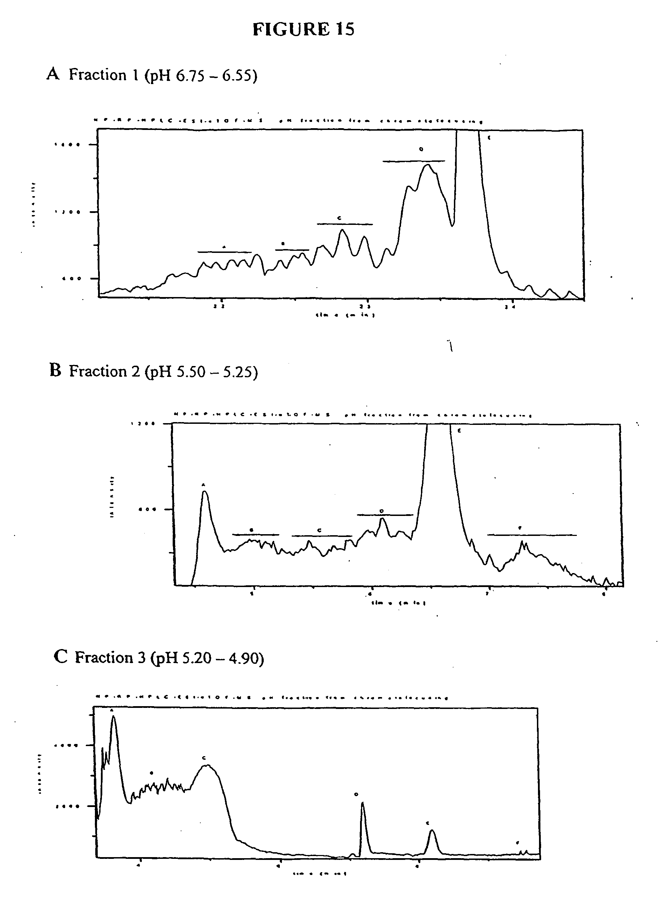

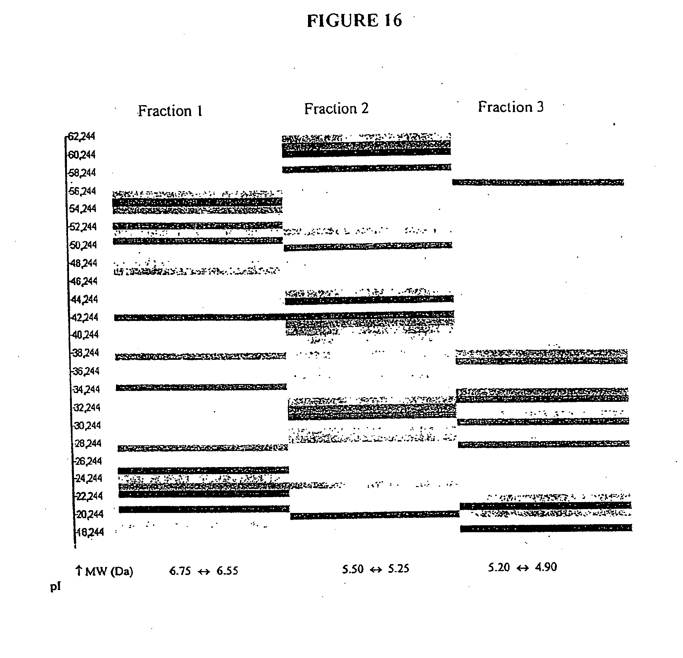

An exemplary embodiment of the chromatofocusing techniques of the present invention are provided in Example 7. Data from these experiments is shown in Figures 14-16. Figure 14 shows the CF profile of MCF-10A whole cell lysate (pH 7 to 4). Fractions 1 to 3 were further analyzed with NP-RP-HPLC-ESI-oaTOF MS (described in detail below). Figures 15A-C show the NP-RP-HPLC-ESI-oaTOF TIC (total ion count) profile of the three fractions from Figure 14: (A) fraction 1 (pH 6.75 - 6.55); (B) fraction 2 (pH 5.50 - 5.25); and (C) fraction 3 (pH 5.20 - 4.90). By integrating and deconvoluting the TIC profiles with the MaxEntl software (described in detail below), the mass spectra for all three fractions are displayed in a 2-D format as shown in Figure 16. Figure 16 shows the integrated TIC in one 2-D protein map where the vertical column is the molecular weight while the horizontal dimension is the protein pI point. This map also contains the relative abundance information whereby the bands vary in intensity (shades of gray) depending on the amount of the protein present.

The data generated by CF-NP-RP-HPLC-ESI-oaTOF MS can be presented as 2-D maps or 2-D images much like the traditional 2-D gel images. For example, in some embodiments, the chromatograms, TICS, integrated and deconvoluted mass spectra are converted into the ASCII format before being plotted vertically, using a 256-step gray scale, such that peaks are represented as darkened bands against a white background. This scale comes in a variety of color formats. Therefore, this 2-D map provides vital information on pI, hydrophobicity, molecular weight as well as the relative abundance of separated proteins. This map can also be adjusted by zoom into a specific area of interest, for a more detailed image of all the bands therein. All the information gathered from this 2-D map can be used to examine protein expression in a cell system due to the disease state, pharmaceutical treatment or environmental change. Since the image is automatically digitized, it can be easily stored and the bands can be hyperlinked to other experimental results or related data. As a result, all the information is available from the original image.

The use of chromatofocusing with the separation, analysis, and display methods of the present invention provide a number of important advantages not previously available. For example, by combining chromatofocusing with a second separation phase (e.g., NP-RP-HPLC) and mass spectrometry analysis, a 2-D liquid phase protein map is generated which is analogous to a 2-D geL In preferred embodiments, this is a multi-dimensional liquid chromatography (LC) whereby both chromatographic techniques are performed on-line (*i.e.*, in an automated fashion) between two or multiple LC units with a switching valve to deliver fractions from CF to, for example, NP-RP-BPLC; Proteins are peeled off the CF column according to their pH, one pH unit or fraction thereof, at a time. This peeling feature allows for further focusing of the protein bands at their respective pI regions. The protein concentration of each pI band is thus enhanced during elution. As with the method described above, buffers can be used that are compatible with each step of the process. For example, in some embodiments, the sample preparation and CF separation involves the use of guanidine-hydrochloride and a nonionic detergent (e.g., n-octyl -D-glucopyranoside) that is compatible with the NP-RP-HPLC and ESI-oaTOF MS.

The present invention may be used in developing protein microarrays. The present invention provides a new method for preparing large numbers of intact proteins from cell lysates for microchip or other solid surface array analysis. This method involves a 2-D (or more) fractionation of proteins from a cell lysate. The 2-D fractionation of proteins from a cell lysate first involves the separation of intact proteins from cell lystates using chromatofocusing. Next, each fraction is further purified with non-porous silica RP-HPLC. This preferred embodiment yields a 2D-liquid phase fractionation of the proteins from a cell lysate, at a relatively high purity. In addition, this preferred embodiment permits the spotting onto a microarray of thousands of isolated proteins in a liquid phase. An exemplary embodiment of developing protein microarrays with chromatofocusing and HPLC techniques is provided in Example 11.

Microarrayed proteins find many uses. For example, the present invention may be used in studying a humoral response. The present invention provides a new method for identifying sero-diagnostic markers for disease (e.g., cancer). The present invention provides a new method for identifying sero-diagnostic markers for prostate cancer. The fractionated proteins can be spotted on a nitrocellulose slide or any other suitable surface and used in the study of a humoral response by exposing it to sera from cancer patients and normal individuals (or from any two samples to be compared). This method allows for comprehensive analysis of the cancer proteome using very small amounts of analyte obtained by fractionation. Importantly, this method allows for use of post-translationally modified proteins as baits for detection of humoral response. Proteins eliciting an immune response are identified using, for example, the molecular mass and peptide sequence data obtained using mass spectrometric analysis of liquid fractions. As such, this method further may be used as a diagnostic agent in the diagnosis of disease (e.g., cancer). An exemplary embodiment of studying a cancer humoral response with chromatofocusing and HPLC techniques is provided in Example 11.

### IV. Mass Spectroscopic Analysis and 2-D Display Systems and Methods

In some preferred embodiments of the present invention, separated proteins are analyzed by mass spectrometry to facilitate the generation of detailed and informative 2-D protein maps. The present invention is not limited by the nature of the mass spectrometry technique utilized for such analysis. For example, techniques that find use with the present invention include, but are not limited to, ion trap mass spectrometry, ion trap/time-of-flight mass spectrometry, quadrupole and triple quadrupole mass spectrometry, Fourier Transform (ICR) mass spectrometry, and magnetic sector mass spectrometry. The following description of mass spectroscopic analysis and 2-D protein display is illustrated with ESI oa TOF mass spectrometry. Those skilled in the art will appreciate the applicability of other mass spectroscopic techniques to such methods.

In some embodiments of the present invention, ESI oa TOF mass spectrometry is used following two dimensional protein separation to provide an accurate protein separation map. For example, in one embodiments of the present invention, proteins were analyzed from human erythroleukemia (HEL) cells. The human erythroleukemia (HEL) cell line was obtained from the Department of Pediatrics at The University of Michigan. HEL cells were cultured according to the methods described in Example 1. A preparative scale Rotofor (Biorad) was used in the first dimension separation. In this experiment, 20 mg of protein was loaded. The proteins were separated by isoelectric focusing over a 5 hour period with slight modifications to the Rotofor methods described elsewhere herein. The separation temperature was 10°C, and the separation buffer contained 0.5 % n-octyl -D-glucopyranoside (OG) (Sigma), 6 M urea (ICN), 2 M thiourea (ICN), 2 % -mercaptoethanol (Biorad) and 2.5 % Biolyte ampholytes, pH 3.5-10 (Biorad).

The procedure used for running the Rotofor (Rotofor Purification System, Biorad) was a modified version of the standard procedure described in the manual from Biorad. The starting power, voltage and current were 12 W, 400 V and 36 mA respectively. The ending power, voltage and current were 12 W, 1000 V and 5 mA respectively. The 20 fractions contained in the Rotofor were collected simultaneously into separate vials using a vacuum source attached by plastic tubing to an array of 20 needles, which were punched through a septum. The Rotofor fractions were aliquotted in 400 µL amounts into polypropylene micro-centrifuge tubes and stored at -80°C for further analysis as desired. The pH of the fractions was determined using pH indicator paper (Type CF, Whatman). Fractions from the Rotofor were quantified using a Bradford assay *(See e.g.,* Wall et al., Anal. Chem., 72:1099 [2000]).

For NPS RP HPLC, separations were performed at a flow rate of 0.4 mL per minute on an analytical (3.0 * 33 mm) NPS RP HPLC column containing 1.5 µm C18 (ODSI) non-porous silica beads (Eichrom Technologies). The use of the 3 mm column provided more than sufficient sensitivity with the use of the LCT as well as reduced solvent consumption. The column was placed in a column heater (Timberline, Boulder CO) and maintained at 65°C. The separations were performed using water/acetonitrile (0.1 % TFA, 0.3% formic acid) gradients. The gradient profile used was as follows: 1) 0 to 20 % acetonitrile (solvent B) in 1 minutes; 2) 20 to 30 % B in 2 minutes; 3) 30 to 54 % B in 8 minutes; 4) 54 to 65% B in 1 minute; 5) 65 to 100 % B in 1 minute; 6) 100 % B in 3 minutes; 7) 100 to 5 % B in 1 minute. The effective start point of this profile was one minute into the gradient due to a one-minute dwell time. The acetonitrile was 99.93 +% HPLC grade (Sigma), the TFA was from 1 mL sealed glass ampules (Sigma) and the formic acid was ACS grade (Sigma). The non-ionic detergent used was n-octyl - -D-galactopyranoside (OG) (Sigma). The HPLC instrument used was a Beckman model 127s/166 and the peaks were detected on-line by a commercial ESI oa TOF/MS (LCT, Micromass, Manchester U.K.). In preferred embodiments, a detergent is used throughout the separation and detection steps that is compatible with the steps of RP HPLC and ESI oa TOF/MS (e.g., detergents of the formula n-octyl (SUGAR) pyranoside).

The ESI oa TOF/MS analyses were performed on a Micromass LCT equipped with a reflectron, a 0.5 meter flight tube and a dual micro-channel plate detector. The instrument produced protein mass spectra with a mass resolution of 5000 (FWHM). The flow from the HPLC column eluent was split to the ESI stainless steel capillary at a 1:1 ratio leaving a flow to the mass spectrometer of 0.2 mL/minute. The source temperature was held at 150°C, the desolvation temperature was 400°C, the nebulizer gas (N₂) was left at 50% maximum flow and the desolvation gas was held at 600 L/minute. The capillary voltage was held at +2500 V and the sample cone voltage was held at +45 V. The extraction cone was held at +3 V. The RF voltage was set at 1000 V with the first hexapole being biased to a positive DC offset of +7 V and the second hexapole being biased to a negative DC offset of -2 V. The detector voltage was held at 2900 V. Data was acquired for a maximum mass/charge range of 5000 resulting in a pusher cycle time of 90 µs. The data was stored to the ECP at a rate of 1 Hz and then transferred from this data-collecting computer to the main data analysis computer for generation of the data files and TIC.

Software used to analyze the mass spectra was the MaxEnt (version 1) software and Mass Lynx version 3.4 (Micromass). Typical deconvolution was performed with a wide target mass range, 1 Dalton resolution, 0.75 Da peak width and 60% peak height values. All deconvoluted mass spectra from a given TIC were added together to produce one mass spectrum for each TIC. The TIC mass spectra from each of the Rotofor fractions were then input to the 2D mapping software (available from Dr. Stephen J. Parus, University of Michigan, Department of Chemistry, 930 N. University Ave., Ann Arbor, MI 48109-1055).

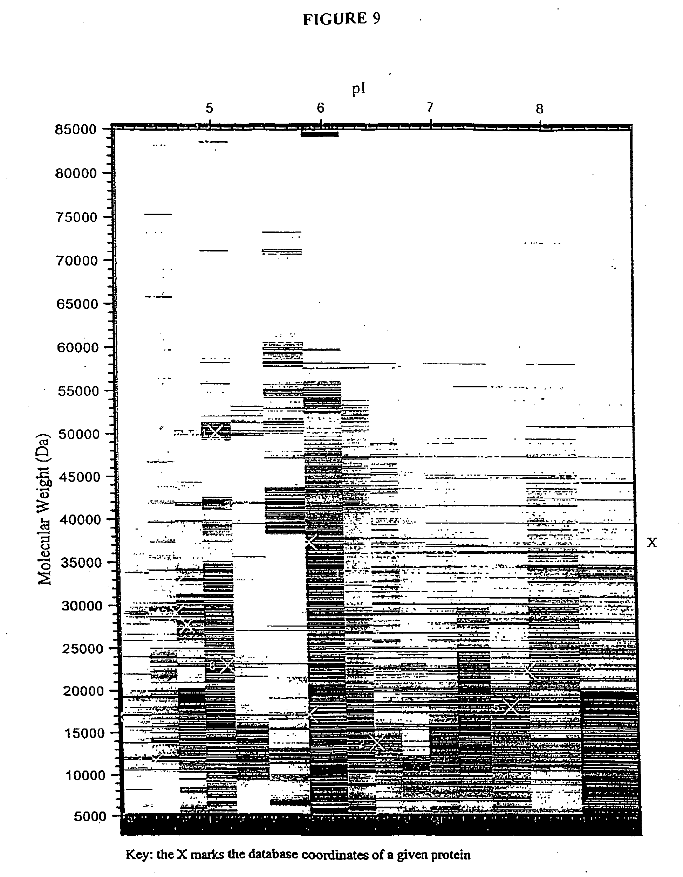

The 2-D image in Figure 9 shows protein molecular weight in the vertical dimension and protein pI in the horizontal dimension. Individual proteins are represented as bands within the grayscale image. Protein identities were matched to this image by overlaying a virtual map of all proteins previously identified via the NPS RP HPLC separation method described above and digest analysis with MSFit database searching.

The experimental mass values were typically within 1 to 3 parts per thousand of the value recorded in the SWISS-PROT database. The pI could be estimated to within 0.01 to 0.5 pI units using intensity profiling as described below. Each vertical lane represents, in band format, all proteins observed via LCT mass spectral detection from the NPS RP HPLC analysis of that particular Rotofor fraction. The NPS RP HPLC separations were performed on from 17 to 60 µg of protein per Rotofor fraction. The bands in the image vary in gray scale intensity according to the intensity of the source molecular weight peaks. This image has been magnified in the intensity dimension by allowing virtual saturation of the signal of the more abundant proteins. The magnification factor is 27X or 53615/2000 (max intensity/magnification intensity). The intensity has a linear dynamic range of at least 3 orders of magnitude. Some of the same protein patterns can be seen in both the liquid phase separation and a 2D gel image from Swiss-Prot (http://expasy.cbr.nrc.ca/ch2dothergifs/publi/elc.gif). Five of the nineteen proteins identified in the 2D gel image also were found in the liquid phase separation. When comparing these images it must be kept in mind that the mass scale is linear from the liquid phase separation and logarithmic in the gel phase separation.

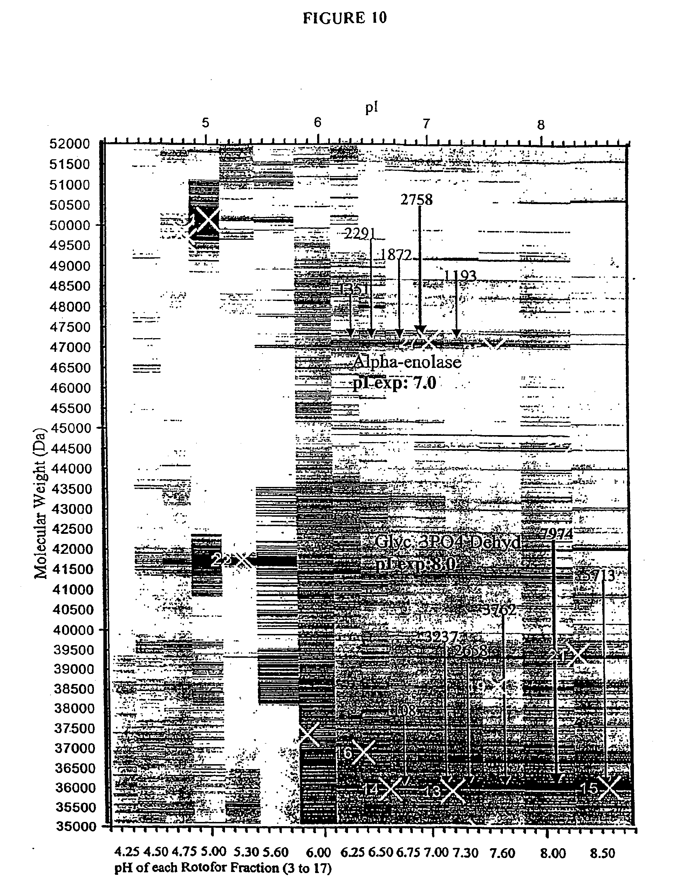

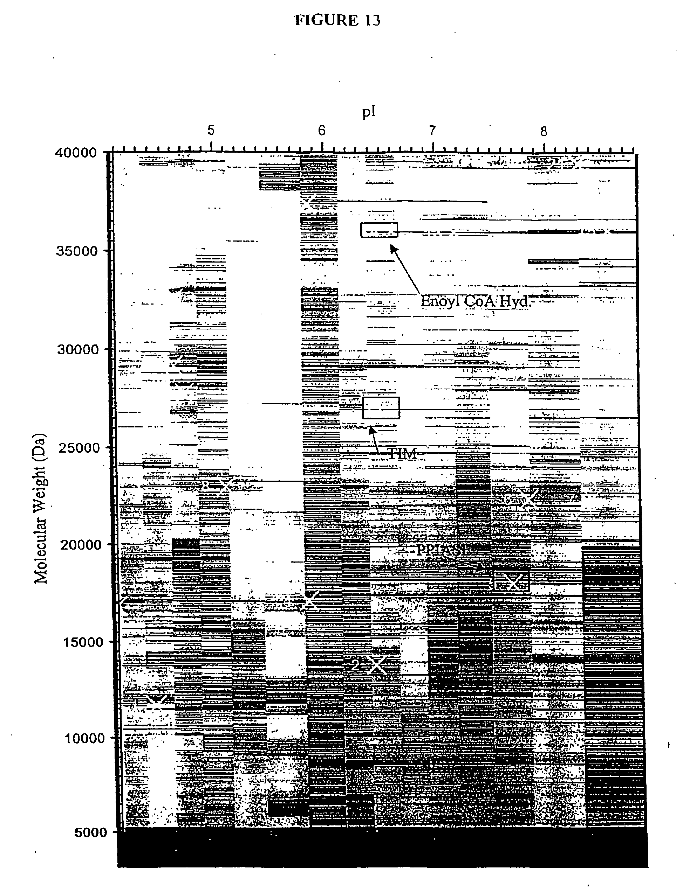

The pI of proteins isolated in the 3D liquid separation method can be estimated by observing the intensity of a given protein peak over a range of pI fractions. As a protein may spread anywhere from 2 to 6 pI fractions due to diffusion and basic cathodic drift, it should be most abundant in that fraction that is closest to its own pI. This can be observed in the zoom image of Figure 10 *(See also,* zoom image of Figure 13). Using this approach, the pI of alpha-enolase is estimated to be 7.0 (database value of 7.01), and the pI of glyceraldehyde 3-PO₄ dehydrogenase is estimated to be 8.0 (database value of 8.57). This acidic shift may be due to a post-translational modification such as phosphorylation or glycosylation.

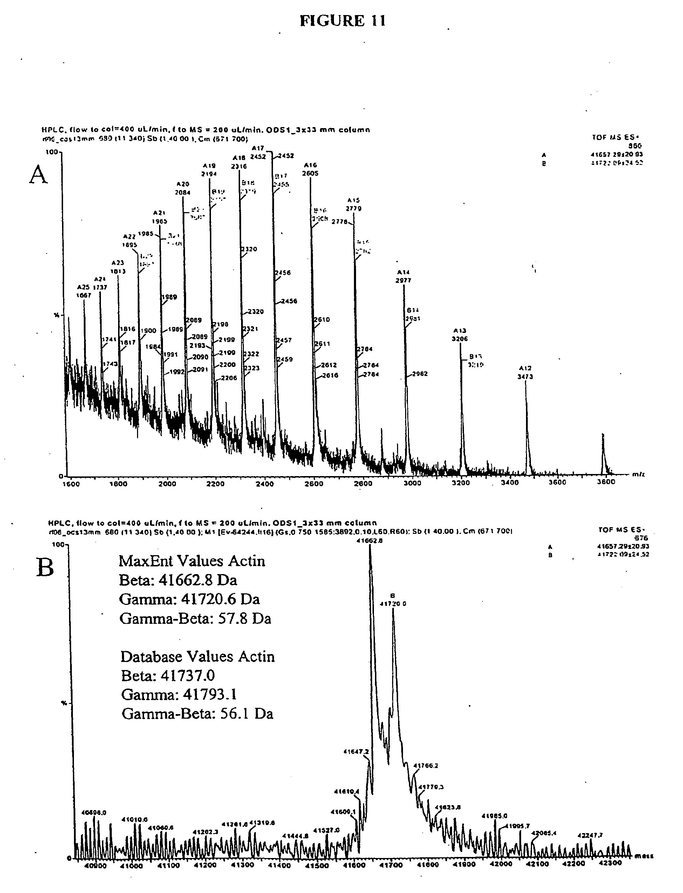

The protein molecular weights were determined by MaxEnt deconvolution of multiply charged protein umbrella mass spectra that were obtained by combining anywhere from 10 to 60 seconds of data from the initial total ion chromatogram (TIC). The umbrella for beta and gamma actin is shown in Figure 11A, each form of actin being labeled with the charge state. Figure 11B shows the resulting molecular weight mass spectrum for actin where the two forms of actin are separated. Note that the two forms of actin are clearly resolved from one another unlike in gel images where the actin spot always represents the co-migration of beta and gamma actin. A useful feature of the liquid phase method of the present invention is the capability of the high resolution mass spectrometry to quantitate which allows the observer to record relative levels of each form of a given protein. Consequently, it is contemplated that one cam determine the relative abundances of the phosphorylated and non-phosphorylated forms of a given protein. In addition, post-translational modifications such as phosphorylation can be found by searching the data for intervals of some integer value times 80 Da.

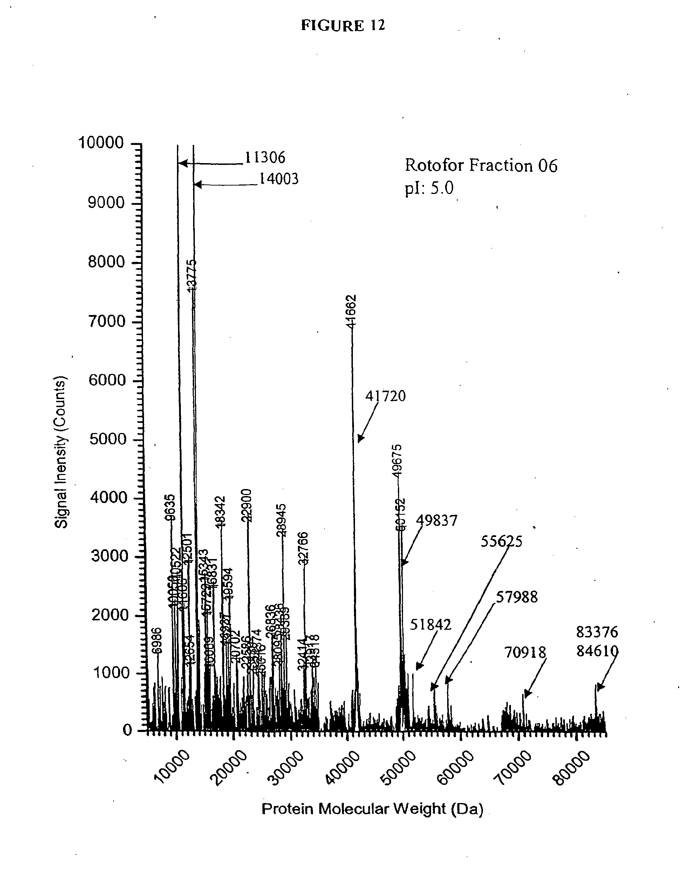

Figure 12 shows the traditional peak view format of one of the Rotofor fraction's combined molecular weight mass spectra. All proteins were deconvoluted and then added together into one mass spectrum. There are 44 unique protein molecular weights observed in this mass spectrum. Assuming similar numbers of unique masses in all 15 of the Rotofor fractions analyzed herein, and accounting for longitudinal diffusion between fractions, it is estimated that approximately 220 unique protein masses in the image from a pI of 4.1 to a pI of 8.75. The Rotofor produces 20 fractions, though only 15 were analyzed in this work, so that around 300 unique masses should be observed in the full analysis of all Rotofor fractions. It is contemplated that lower level proteins not obtained in the above experiment can be obtained using improved HPLC gradients, 53 mm long columns and more detailed MaxEnt analyses. Using such methods, it is contemplated that the number of unique masses will be around 750.

As shown in the above experiments, the 2D protein image from the IEF-NPS RP HPLC-ESI oa TOF/MS separation of the human erythroleukemia cell lysate provides high mass resolution and high accuracy imaging of the proteins. The mass resolution allows the image to show very different forms of the same protein that have small differences in mass. With a mass resolution of 5000 Da, a 50000 Da protein can be resolved from a 50010 Da protein. Clearly, single phosphorylations on entire proteins can be observed with this level of resolution. Quantitative comparison between 2-D images can be achieved by spiking samples with known amounts of standard proteins and normalizing images through landmark proteins. Thus, the observer can detect significant abundance changes in the protein profiles of different samples. The differences can then be targeted for more detailed analysis. For example, protein bands on the image can be hyper-linked to other experimental results, obtained via analysis of that band, such as peptide mass fingerprints and MSFit search results. Thus all information obtained about a given 2-D image, including detailed mass spectra, data analyses and complementary experiments (immuno-affinity, peptide sequencing) can be accessed from the original image.

Having identified and characterized the proteins that have changed in abundance due to some disease state or drug treatment, it is possible to identify biomarkers for disease states as well as drug targets for pharmaceutical agents and monitor the presence of, or change in, such markers in a particular biological sample (e.g., tissue samples with and without exposure to a candidate drug). Indeed, drug screening and diagnostic techniques can be automated using the systems and methods of the present invention, wherein cells (e.g., experimental and control cells) are cultured, treated, and lysed using robotics and wherein the lysate is fed into the automated separation and analysis systems of the present invention.

As is clear from the above description, the methods of the present invention provide a range of novel features that provide improved methods for analyzing protein expression patterns. For example, the present invention provides a combination of IEF, resulting in pI-focused proteins in liquid phase fractions, with nonporous RP HPLC and ESI oa TOF/MS to produce a 2-dimensional liquid phase protein map image analogous to that of a 2-D gel. These methods allow the identification of proteins separated by IEF-NPS RP HPLC using enzymatic digestions and mass spectrometric analysis of the resulting peptide mass fingerprints and correlation of this data with the pI and molecular of the protein found via the whole protein 3-D separation method. In some improved display embodiments of the present invention, one can view a collection of different IEF-NPS RP HPLC-ESI oa TOF/MS chromatograms in one 2-D image displaying the mass spectra in a top view protein band format, not the traditional side view peak format. The methods also allow the detection of proteins and determination of their molecular weights by analyzing the eluent from the HPLC with computational (*e.g.,* on-line) analysis using ESI oa TOF/MS.

The IEFF-NPS RP HPLC-ESI oa TOF/MS method also allows one to fully integrate and deconvolute each of the TIC's generated to display complete mass spectra of each collection of pI-focused proteins. The method also allows the display of all the integrated TIC's in one 2-D image where the vertical dimension is in terms of protein molecular weight and the horizontal dimension is in terms ofprotein pL In such displays, the protein mass spectra appear as bands as they will also be viewed from the top. This image would therefore also contain relative quantitative information wherein the bands vary in intensity depending on the amount of protein present. The use of liquid phase separation techniques with the method allows for collection of protein fractions to micro-tubes or 96-well plates such that the proteins could be digested and the peptide mass maps analyzed to determine the identity of said proteins simultaneously.

### V. Automated 3D HPLC/MC Methods for Rapid Protein Characterization

The method set present invention are useful for an automated system for the separation and identification of protein samples based on multiple physical properties. Accordingly, the protein separation and analysis techniques described in the preceding sections may be automated into one integrated, on-line system. Protein samples are separated in a first phase and a second orthogonal phase, followed by mass spectroscopy analysis. All of the step may be automated and coordinated through an automated sample handler and a centralized control network.

Accordingly, the entire separation and characterization process can be controlled through one centralized control network. The network is integrated with all of the apparatus and software used for the automated process. The centralized control network may include a computer system. The use of a centralized control network allows for the entire separation and characterization process to be controlled from one computer terminal by one operator. The network directs sample through the appropriate separation phases. The network then controls the transfer of protein information to analysis software. The analysis software is integrated into the network and can be programmed to generate a customized report based on the information required by the user.

### A. Protein Separation

As described above, the present invention provides methods for the separation of protein samples in two phases. The methods may be orthogonal, and thus allow for the generation of a two-dimensional map. In some preferred embodiments, the present invention further provides methods of automating the two phase separation.

### 1. Separation in a First Phase

The automated separation methods of the present invention may be used on any suitable protein sample. As discussed above, in some embodiments, the sample is solubilized in a buffer comprising a compound of the formula n-octyl SUGAR pyranoside (e.g., including, but not limited to, n-octyl -D-glucopyransoside and n-octyl -D-galactopyransoside).

The first dimension of the automated separation process separates proteins based on a first physical property. Proteins are separated by charge (e.g., ion exchange chromatography). Cation exchange chromatography is used to separate positive proteins and anion exchange chromatography is used to separate negatively charged proteins. However, the first dimension may employ any number of separation techniques including, but not limited to, ion exclusion, isoelectric focusing, normal/reversed phase partition, size exclusion, ligand exchange, liquid/gel phase isoelectric focusing, and adsorption chromatography.

In some preferred embodiments, the first separation phase is conducted in the liquid phase. In some embodiments, the first phase is ion exchange. In such embodiments, it is preferred that samples are de-salted prior to the second separation phase. In some embodiments, desalting is performed on an automated solid phase extraction (SPE) system. In some embodiments, both the ion exchange and the desalting are performed on the same automated SPE system. In other embodiments, the ion exchange is performed on a column and the eluate is directed into the automated SPE system.

In some embodiments, if proteins are present in small amounts, samples can be loaded onto the SPE columns multiple times in order to obtain a sufficient amount for analysis. Thus, the present invention has the added advantage of allowing the identification of proteins with a low level of expression.

### 2. Automated Sample Handling

As described in the preceding section, samples may be processed using an automated sample handling system. An on-line automated, SPE system may be utilized (*e.g.,* including, but not limited to, the Prospekt automated SPE system; Spark Holland Instrumenten, The Netherlands). The advantage of on-line SPE is the direct elution of the extract from the SPE cartridge into the second phase (*e.g.,* LC system) by the LC mobile phase. Several laborious handling steps are thus omitted, making on-line SPE much more efficient and providing superior analytical results. The superior analytical performance of on-line SPE is derived from the elimination of eluate collection, evaporation, reconstitution and injection, thus eliminating several major error sources. In addition, on-line elution transfers 100% of the purified analytes from the extraction cartridge into the LC (*e.g.,* HPLC). This provides maximum precision and sensitivity, as well as reduced costs, thus saving solvents, glassware, and labor time. In addition, samples and SPE cartridges are processed in a completely closed system, making sample tracking easy and protecting samples against light and air. It also protects the operator from contact with hazardous samples or solvents. Furthermore, less handling means fewer failures and high pressure solvent control for SPE makes the process independent of cartridge back pressure.

### 3. Separation in a Second Phase

Following the first separation phase, products of the separation step are fed directly into a second liquid phase separation step. The second dimension separates proteins based on a second physical property (*i.e.,* a different property than the first physical property) and is preferably conducted in the liquid phase (*e.g.,* liquid-phase size exclusion). Proteins are separated by hydrophobicity using non-porous reversed phase HPLC *(See e.g.,* Liang et al., Rap. Comm. Mass Spec., 10:1219 [1996]; Griffin et al., Rap. Comm. Mass Spec., 9:1546 [1995]; Opiteck et al., Anal. Biochem. 258:344 [1998]; Nilsson et al., Rap. Comm. Mass Spec., 11:610 [1997]; Chen et al., Rap. Comm. Mass Spec., 12:1994 [1998]; Wall et al., Anal. Chem.; 71:3894 [1999]; Chong et al.,Rap. Comm. Mass Spec., 13:1808 [1999]).

This method provides for exceptionally fast and reproducible high-resolution separations of proteins according to their hydrophobicity and molecular weight. The non-porous (NP) silica packing material used in these reverse phase (RP) separations eliminates problems associated with porosity and low recovery of larger proteins, as well as reducing analysis times by as much as one third.

An automated on-line sample handling system may be utilized and fully integrates the second separation phase with the first separation step. The sample flows directly from the first phase (*e.g.,* ion exchange) through a desalting step (*e.g.,* SPE) to the second phase (*e.g.,* NP-RP HPLC). The HPLC column may be integrated into the automated sample handling system (*e.g.,* those utilizing the Prospekt system). For example, a multi valve system can be utilized where valve-switching is used to bring the extraction cartridge into the HPLC system. A sample is passed through the second phase separation step (*e.g.,* NP-RP HPLC) greater than one time (e.g., twice) in order to improve selectivity and resolution. For example, in some embodiments, two different NP-RP-HPLC columns are utilized in tandem. The automation of protein separation increases efficiency and speed as well as decreases sample loss or potential contamination that may occur through handling.

### B. Protein Identification by Mass Spectroscopy

Following separation in the first and second phase, the automated sample handling system transfers samples to the mass spectroscopy step. The present invention is not limited to any one mass spectroscopy technique. Indeed, a variety of techniques are contemplated. For example, techniques that find use with the present invention include, but are not limited to, ion trap mass spectrometry, ion trap/time-of-flight mass spectrometry, quadrupole and triple quadrupole'mass spectrometry, Fourier Transform (ICR) mass spectrometry, and magnetic sector mass spectrometry. In preferred embodiments, the MS analysis is automated and is performed on-line. In some embodiments, the eluent from the second separation phase is split into two fractions. A fraction of the effluent is used to determine molecular weight by either MALDI-TOF-MS or ESI oa TOF (LCT, Micromass) (*See e.g.,* U.S. Pat. No. 6,002,127). The remainder of the eluent is used to determine the identity of the proteins via digestion of the proteins and analysis of the peptide mass map fingerprints by either MALDI-TOF-MS or ESI oa TOF. The molecular weight 2-D protein map is matched to the appropriate digest fingerprint by correlating the molecular weight total ion chromatograms (TIC's) with the UV-chromatograms and by calculation of the various delay times involved. The UV-chromatograms are automatically labeled with the digest fingerprint fraction number. The resulting molecular weight and digest mass fingerprint data can then be used to search for the protein identity via web-based programs like MSFit (UCSF).

A detailed discussion of the use of 3-D maps generated by the automated separation process of the present invention to identify and characterize proteins is provided in the above sections. In some embodiments, the present invention provides a 3-D map in which the first dimension represents a first physical property (*e.g.,* charge or isoelectric point), the second dimension represents a second physical property (*e.g.,* hydrophobicity or molecular weight), and the third dimension represents the molecular weight and relative abundance of proteins present in the sample. In some embodiments, the data from the 3-D protein map is used to search protein data bases in order to determine the identity of the proteins.

Sample analysis can be automated and integrated with the centralized control network. For example, mass spectroscopy data is transferred to an integrated computer system containing software for the generation of 3-D protein maps. The integrated computer system is also capable of searching databases and generating a report. The report is provided to the operator in a format that is customized to the particular application. For example, if an experiment was designed to identify unknown components of a solution, the report identifies components of the 3-D map as particular proteins. Conversely, if an experiment is designed to compare the protein expression profiles of two samples, the report may identify proteins that are present in one sample and absent in another or are present at different abundances between the two samples.

### C. Automated Protein Separation and Characterization in Practice

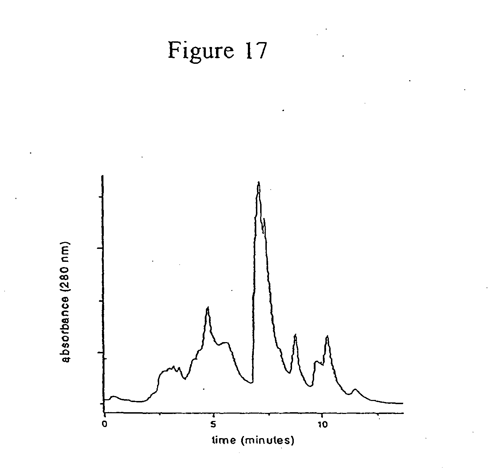

Illustrative Example 8 describes one particular embodiment of the present invention where an automated on-line Prospekt system was used to separate a protein sample based on charge and hydrophobicity. Siberian Permafrost whole cell lysate was first separated using a mini MonoQ anion exchange column. A graph of the Mini Q column eluent is shown in Figure 17. Fractions (1 minute each) from the anion exchange column gradient were fed directly into the second step using the automated Prospekt system. The Prospekt then trapped the fractions on 10 C4 SPE cartridges. Each cartridge was washed with the reverse-phase HPLC starting buffer to remove residual salt. The Prospekt system integrates the HPLC and SPE steps with a multi valve switching system. Following the wash step, the eluent from the SPE cartridge was directly transferred to the NP-RP HPLC column.

The fractions were separated using a tandem column method. A gradient was applied to the HPLC column. The HPLC column was then switched back to the initial buffer and allowed to equilibrate. The eluent from the first gradient is then passed through a second (different) HPLC column. The use of a second tandem column increases resolution and selectivity. This step is repeated for each of the SPE cartridges (each representing one anion exchange fraction).

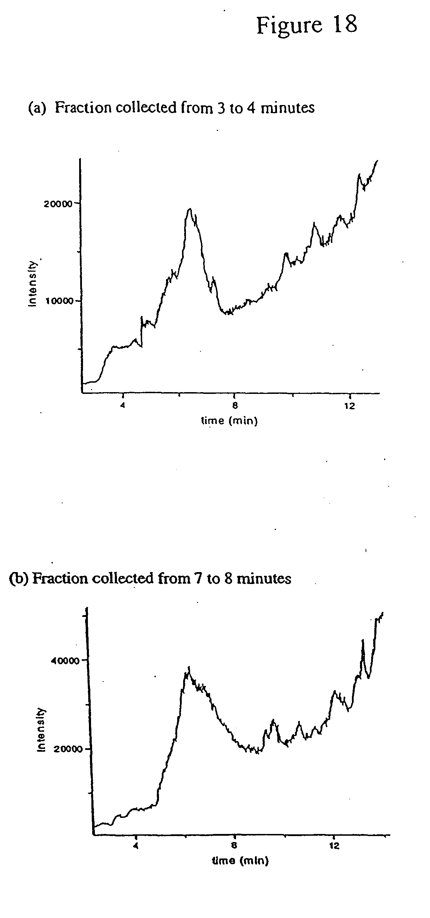

Following separation by NP-RP-HPLC, protein fractions were analyzed online by MS to determine their molecular weight and abundance. The eluent from the column was split into two fractions. One fraction is digested enzymatically before MS. Both the digested and non-digested sample were analyzed by ESI oa TOF TIC (total ion count) mass spectroscopy. Total ion count profiles are shown in Figures 18A and 18B.

### EXPERIMENTAL

The following examples serve to illustrate certain preferred embodiments and aspects of the present invention.

### EXAMPLE 1

### HEL Cell Sample Preparation

The human erythroleukemia (HEL) cell line was obtained from the Department of Pediatrics at The University of Michigan. HEL cells were cultured (7% CO₂, 37°C) in RPMI-1640 medium (Gibco) containing 4 mM glutamine, 2 mM pyruvate, 10 % fetal bovine serum (Gibco), penicillin (100 units per mL), streptomycin (100 units per mL) and 250 mg of hygromycin (Sigma). The HEL cell pellets were washed in sterile PBS, and then stored at 80°C. The cell pellets were then re-suspended in 0.1% n-octyl B-D-galactopyranoside (OG) (Sigma) and 8 M urea (Sigma) and vortexed for 2 minutes to effect cell disruption and protein solubilization. The whole cell protein extract was then diluted to 55 mL with the Rotofor buffer and introduced into the Rotofor separation chamber (Biorad).

### EXAMPLE 2

### 1-D Gel and SDS PAGE Separation

HEL cell proteins, resolved by Rotofor separation into discrete pI ranges, were further resolved according to their apparent molecular weight by SDS-PAGE. This procedure takes approximately 14 hours to complete. Samples of rotofor fractions were suspended in an equal volume of sample buffer (125 mM Tris (pH 6.8) containing 1% SDS, 10% glycerol, 1% dithiothreitol and bromophenol blue) and boiled for 5 min. They were then loaded onto 10% acrylamide gels. The samples were electrophoresed at 40 volts until the dye front reached the opposite end of the gel. The resolved proteins were visualized by silver staining. The gels were fixed overnight in 50% ethanol containing 5% glacial acetic acid, then washed successively (for 2 hours each) in 25% ethanol containing 5% glacial acetic acid, 5% glacial acetic acid, and 1% glacial acetic acid. The gels were impregnated with 0.2% silver nitrate for 25 min. and were developed in 3% sodium carbonate containing 0.4% formaldehyde for 10 min. Color development was terminated by impregnating the gels with 1% glacial acetic acid, after which the gels were digitized.

### EXAMPLE 3

### 2-D PAGE

In order to prepare protein extracts from the HEL cells, the harvested cell pellets were lysed by addition of three volumes of solubilization buffer consisting of 8 M urea, 2% NP-40,2% carrier ampholytes (pH 3.5 to 10),2% β-mercaptoethanol and 10 mM PMSF, after which the buffer containing the cell extracts was transferred into microcentrifuge tubes and stored at 80°C until use.

Extracts of the cultured HEL cells were separated in two dimensions as previously described by Chen *et al.* (Chen et al., Rap. Comm. Mass Spec. 13:1907 [1999]) with some modifications as described below. Subsequent to cellular lysis in solubilization buffer, the cell lysates from approximately 2.5 x 10⁶ cells were applied to isoelectric focusing gels. Isoelectric focusing was conducted using pH 3.5 to 10 carrier ampholytes (Biorad) at 700 V for 16 h, followed by 1000 V for an additional 2 hours. The first dimension tube gel was soaked in a solution of 2 mg/mL of dithioerythritol (DTE) for 10 minutes, and then soaked in a solution of 20 mg/mL of iodoacetamide (Sigma) for 10 minutes, both at room temperature. The first-dimension tube gel was loaded onto a cassette containing the second dimension gel, after equilibration in second-dimension sample buffer (125 mM Tris (pH 6.8), containing 10% glycerol, 2% SDS, 1% dithioerythritol and bromophenol blue). For the second-dimension separation, an acrylamide gradient of 11.5% to 14% was used, and the samples were electrophoresed until the dye front reached the opposite end of the gel. The separated proteins were transferred to an Immobilon-P PVDF membrane. Protein patterns in some gels were visualized by silver staining or by Coomassie blue staining, and on Immobilon-P membranes by Coomassie blue staining of the membranes.

### EXAMPLE 4

### Rotofor Isoelectric Focusing

A preparative scale Rotofor (Biorad) was used in the first dimension separation. This device separated the proteins in liquid phase according to their pI, and is capable of being loaded with up to a gram of protein, with the total buffer volume being 55 mL. Alternatively, for analysis of smaller quantities of protein, a mini-Rotofor with a reduced volume can be used. These proteins were separated by isoelectric focusing over a 5 hour period where the separation temperature was 10 °C and the separation buffer contained 0.1 % n-octyl β-D-galactopyranoside (OG) (Sigma), 8 M urea (ICN), 2 % β-mercaptoethanol (Biorad) and 2.5 % Biolyte ampholytes, pH 3.5-10 (Biorad). The procedure used for running the Rotofor (Rotofor Purification System, Biorad) was of the standard procedure described in the manual from Biorad as modified herein. The 20 fractions contained in the Rotofor were collected simultaneously, into separate vials using a vacuum source attached by plastic tubing to an array of 20 needles, which were punched through a septum. The Rotofor fractions were aliquotted into 400 µL amounts in polypropylene microcentrifuge tubes and could be stored at 80°C for further analysis if necessary. An advantage of gel methods is the ability to store proteins stably in gels at 4 °C for further use. The concentration of protein in each fraction was determined via the Biorad Bradford based protein assay. The pH of the fractions was determined using pH indicator paper (Type CF, Whatman).

### EXAMPLE 5

### NP RP HPLC

Separations were performed at a flow rate of 1.0 mL/minute on an analytical (4.6 *14 mm) NP RP HPLC column containing 1.5 µm C18 (ODSI) non-porous silica beads (Micra Scientific Inc.). The column was placed in a Timberline column heater and maintained at 65°C. The separations were performed using water/acetonitrile (0.1% TFA, 0.05 % OG) gradients. The gradient profile used was as follows: 1) 0 to 25% acetonitrile (solvent B) in 2 minutes; 2) 25 to 35% B in 2 minutes; 3) 35 to 45% B in 5 minutes; 4) 45 to 65% B in 1 minute; 5) 65 to 100% B in 1 minute; 6) 100% B in 3 minutes; 7) 100 to 5% B in 1 minute. The start point of this profile was one minute into the gradient due to a one-minute dwell time. The acetonitrile was 99.93+% HPLC grade (Sigma) and the TFA were from 1 mL sealed glass ampules (Sigma). The non-ionic detergent used was n-octyl β-D-galactopyranoside (OG) (Sigma). The HPLC instrument used was a Beckman model 127s/166. Peaks were detected by absorbance of radiation at 214 nm in a 15 µL analytical flow cell.

Protein standards (Sigma) used as MW protein markers and for correlation of retention time, molecular weight and hydrophobicity were bovine serum albumin (66 kDa), carbonic anhydrase (29 kDa), ovalbumin (45 kDa), lysozyme (14.4 kDa), trypsin inhibitor (20 kDa) and -lactalbumin (14.2 kDa).

### EXAMPLE 6

### MALDI-TOF MS of NP RP HPLC Isolated Proteins

The MALDI-TOF MS analyses were performed on a Perseptive Voyager Biospectrometry Workstation equipped with delayed extraction technology, a one-meter flight tube and a high current detector. The N₂ laser provided light at 337 nm for laser desorption and ionization. MALDI-TOF MS was used to determine masses of peptides from protein digests using a modified (described herein) version of the two layer dried droplet method of Dai *et al.* (Dai et al., Anal. Chem., 71:1087 [1999]). The MALDI matrix -cyano-4-hydroxy-cinnamic acid ( -CHCA) (Sigma Chemical Corp., St Louis, MO, USA) was prepared in a saturated solution of acetone (1% TFA). This solution was diluted 8-fold in the same acetone solution (1% TFA) and then added to the sample droplet in a 1:2 ratio (v:v). The mixed droplet was then allowed to air dry on the MALDI plate prior to introduction into the MALDI TOF instrument for molecular weight analyses.

The proteins were collected into 1.5 mL polypropylene micro-tubes containing 20 µL of 0.8 % OG in 50 % ethanol. In preparation for enzymatic digestion the acetonitrile was removed via speedvac at 45°C for 30 minutes. A solution of 200 mM NH₄HCO₃ (ICN) / 1mM β-mercaptoethanol was then added in a 1 to 2 ratio to the remaining solution in the tubes, resulting in a solution of 50 to 100 mM NH₄HCO₃ with a total volume of approximately 150 µL. Subsequently 0.25 µg of enzyme was added to this solution and then the mixture was vortexed and placed in a 37°C warm room for 24 hours. The enzymes used were either trypsin (Promega, TPCK treated), which cleaves at the carboxy side of the arginine and lysine residues, or Glu-C (Promega), which in 50 - 100 mM NH₄HCO₃ solution cleaves at the carboxy side of the glutamic acid residues.

The digest solutions were typically 100 µL in volume and 30 to 50 µL of this solution was desalted and concentrated to a final volume of 5 µL using Zip-Tips (Millipore) with 2 µL C18 resin beds. The purified peptide solution was then used to spot onto the MALDI plate for subsequent MALDI-TOF MS analysis. All spectra were obtained with 128 averages and internally or externally calibrated using the PerSeptive standard peptide mixture containing angiotensin I, ACTH(1-17), ACTH(18-39) and ACTH(7-38) (PerSeptive Biosystems).

These digests were then used to aid in the identification of the proteins by MALDI-TOF MS analysis and MSFit database searching (Wall et al., Anal. Chem., 71:3894 [1999]). The peptide mass maps were searched against the Swiss and NCBInr protein databases using MSFit allowing for 2 missed cleavages. The molecular weight ranged from 5 kDa to 70 kDa and the pI ranged over the full pI range. Externally calibrated peptide masses were searched with 400 ppm mass accuracy and internally calibrated peptide masses were searched with 200 ppm mass accuracy.

### EXAMPLE 7

### Chromatofocusing

In one exemplary embodiment of the chromatic focusing techniques of the present invention, proteins are extracted from cells using a chemical lysis procedure. The lysis buffer consists of 6M guanidine-hydrochloride, 20 mM n-octyl -D-glucopyranoside and 50 mM Tris. Cells are vortexed rigorously and kept overnight at 20 °C. They are subsequently centrifuged at 17,000 rpm for 20 min. The supernatant is removed from the cell debris and re-centrifuged at high speed to further remove any particulate. For the best reproducible results, lysate is best used within 48 hrs. Buffers for this CF are (A) Imidazole-HAC, 0.1% guanidine-hydrochloride, 0.05% n-octyl -D-glucopyranoside, pH 7.2, and (B) Polybuffer 74 (diluted 1:10), 0.1% guanidine-hydrochloride, 0.05% n-octyl -D-glucopyranoside, pH 4. The CF column in this example is Mono P HR 5/20 (Amersham Pharmacia, Uppsala, Sweden) with a flowrate of 1 mL/min at room temperature. Prior to injection lysate is equilibrated with buffer A with a loading time of 20 min. The sample loadability for this CF column is 10 mg of protein. The separation profile is monitored at 280 nm while the pH gradient is monitored using a pH flowcell meter, also from Amersham Pharmacia.

The CF column is equilibrated with buffer A to define the upper pH range (7 in this case) of the pH gradient. The second focusing buffer B is then applied to elute bound proteins, in the order of their isoelectric (*pI*) points. The pH of buffer B is 4, which defines the lower limit of the pH gradient. The pH gradient is formed as the eluting buffer B titrates the buffering groups on the ion-exchanger.

The pI-focused liquid fractions from CF are analyzed in the second dimension using NP-RP-HPLC. Non-porous RP-HPLC columns (Eichrom Technologies, Darien, IL, USA) are used as the second orthogonal separation dimension after CF in order to obtain a 2-D protein map that is capable of competing with 2-D gel. These columns are excellent for protein separation due to their high protein recovery, speed and efficiency. To achieve optimal protein separation, the columns should be kept at a high temperature (*e.g.,* 60°C). This elevated temperature also improves selectivity. Selectivity as well as resolution can also be enhanced by using multiple NP columns in series. RP-HPLC columns packed with non-porous silica beads (Eichrom Technologies) such as ODS1, 2 and 3 are all well suited for these tasks.

Proteins that elute from NP-RP-HPLC separation can be directly analyzed by MS to determine their molecular weight, identity and relative abundance. In this case the eluted proteins are sized simultaneously by ESI-oaTOF MS (LCT, Micromass, Manchester, UK). The other part of the eluted proteins from the split valve can be collected using a fraction collector for enzymatic digestion to obtain peptide maps with a MALDI-TOF MS, ESI-QIT-reTOF MS, or ESI-oaTOF MS (LCT). Information such as the molecular weight, *pI* and peptide map of a protein can then be entered into a web-based protein database program such as MS-Fit (e.g., http://prospector.ucsf.edu) for protein identification.

### Example 8

### Automated 3-D IE NP-RP-HPLC-ESI-oa TOF MS

This example describes an automated system for protein separation and identification based on charge, hydrophobicity, and mass. Protein samples are separated based on charge using an ion exchange (IE) column. Protein fractions are then trapped on a solid phase extraction (SPE) column for desalting using an automated Prospekt system. The Prospeckt system then directs the protein fractions to a nonporous-reverse phase HPLC column (NP-RP-HPLC). The samples are then identified using ESI oa TOF mass spectroscopy.

### A. Protein Separation and Trapping by SPE

Siberian Permafrost whole cell lysate of sample 23-9-25 (obtained from Jim Tiendje, Department of Microbial Ecology, Michigan State University) was lysed using a chemical lysis procedure. The lysis buffer contained 6M guanidine-HCL, 20 mM n-octyl -D-glucopyransoside and 50 mM Tris. The cells were vortexed vigorously and stored overnight at 0°C. The cells were then centrifuged at 17,000 rpm for 20 minutes. The supernatant was removed from the cellular material and then mixed 1:1 with an equilibration buffer for IE (10 mM KH₂PO₄, 5% MeOH, 0.1 % n-octyl -D glucopyranoside, pH 8). The sample was then injected into a Mini Q anion exchange column (Amersham Pharmacia, Uppsala, Sweden) with a flow rate of 1 ml/min at 27°C. Equilibration buffer was run through the column for 3 minutes, followed by a 0% to 100% gradient of buffer B (10 mM KH₂PO₄, 5%MeOH, 0.1 % n-octyl -D glucopyranoside, 1M NaCl, pH 7) in 15 minutes. A graph of the Mini Q column eluent is shown in Figure 17.

Fractions (1 minute each) are each collected on a separate solid phase extraction (SPE) cartridge by directing the eluent from the IE through 10 C4 SPE cartridges. A Prospekt on-line automated SPE system (Spark Holland Instrumenten, The Netherlands) was utilized for the SPE, HPLC, and MS phases.

### B. Protein Purification and Separation by NP-RP-HPLC

The initial mobile phase buffer for the RP analysis was 5 % buffer B (0.1% TFA in ACN) in buffer A (0.1% TFA in H₂O). This solution was directed through the SPE cartridge until all the residual salt from the anion exchange mobile phase was removed. The eluent from the SPE cartridge was next directed by the Prospekt system directly to a HPLC for the second orthogonal separation phase.

Non Porous-RP columns (Eichrom Technologies, Darien, IL) were used as the second separation phase. A tandem column method was employed. ODSIIIE and ODSI NP RP HPLC columns (Eichrom Technologies, Darien, IL) contained 1.5 µm C18 (ODSI) non-porous silica beads. Column dimensions were 4.6 * 33 mm (ODSIIIE) and 4.6 * 14 mm (ODSI). The columns were maintained at 60°C to improve selectivity. A flow rate of 0.5 mL/min at a pressure of 5000 psi was maintained. The columns were loaded, equilibrated in the initial buffer, and the gradient was started. A gradient of buffer B (0.1 % TFA in ACN) was performed as follows: 5% B for 1.5 min, 5% B to 20% B in 2 min, 20% B to 35% B in 5 min, 35% B to 60% B in 15 min, 60% B to 100% B in 5 minutes. The eluent from the first HPLC column (ODSI) was directed into the second HPLC column (ODSIIIE).

Following the gradient, the initial mobile phase buffer was run through the RP column until a stable baseline is realized. The HPLC step was repeated for each of the SPE columns (each of which contained a 1 minute fraction from the anion exchange column).

### C. Protein Identification by Mass Spectroscopy

Following separation by NP-RP-HPLC, protein fractions were analyzed online by MS to determine their molecular weight and abundance. Samples were analyzed by ESI oa TOF TIC (total ion count) mass spectroscopy. Mass spectroscopy conditions were as follows: capillary 2900V, sample cone 45V, extraction cone 3V, RF lens 1000V, desolution temp or 350°C, and source temp of 120°C.

Results of the ESI oa TOF TIC analysis are shown in Figures 18A and B. Figure 18A shows the total ion profile of the fraction collected from 3 to 4 of the MiniQ column; figure 18B shows the total ion profile of the fraction collected from 7 to 8 minutes.

### Example 9

### Automated Protein Separation and CE-MS/MS Analysis

This method identifies individual proteins in a mixture. They are initially physically separated from each other and then each is digested into a set of peptides. Those peptide mixtures are each analyzed by mass spectrometry techniques to obtain the protein identification. The physical separation process is a sequence of two methods (two dimensions). The first is separation by protein pI (pH). The second is a chromatographic separation.

Several methods are available to accomplish the separation by pI. The commercial Rotofor system is an all liquid phase method which uses an electric field with carrier ampholyte to build up a pH gradient. The commercial IsoPrime system uses membranes to build up the pH gradient. Another approach is to utilize carrier ampholye to establish a pH gradient on the stationary phase of a chromatography column and then separate the proteins by elution off the column with a polybuffer. While separation of proteins does occur in these methods, there are still many proteins within a given pI range. In some embodiments, they are then separated from each other by another method (the second dimension). Proteins are collected from first two pI separation methods by collecting the regions that physically exist.

In the latter chromatography method, the proteins are collected into individual tubes as they elute off the column. It is desirable to collect a small pH range (typically 0.2 pH units) into each tube. pH is continuously monitored using a pH electrode placed at the output of the column. The electrode's voltage is digitized and measured by custom software developed to monitor and record the pH and control the tube changes based on user specified pH ranges. A custom tube holder and mechanical changing system has been developed. Up to 100 tubes with tops can be held on a one square foot plate. The chromatography column output tubing is held above the tubes and its eluent drips down into a specific tube. A stepper motor attached to the plate tube holder indexes the desired tube under the column output upon commands from the pH monitoring software. Alternatively, the software sends control signals to commercial fraction collectors. This provides an automated and reproducible first dimension collection scheme.

Each small pH range will still contain many proteins. They are further separated in the second dimension using chromatography methods. As they elute off the column, their presence is detected by ultraviolet light absorption. The separated proteins are collected into individual tubes using the same custom designed collector described above for pH elution or using a commercial fraction collector. The custom pH change software also monitors and records the UV absorbance spectra. When the user visually determines that a protein is eluting (typically by observing the emergence of a peak in the UV spectra), they use the software to send a signal to change collection tubes. The software records the UV spectra and simultaneously an indication of into which tube the protein was collected. Methods are available for automatic peak detection and hence automation of this second dimension separation and collection.

In some cases (in order to compare protein mixtures from two cell lines for example), the first dimension separation by pH may be collected not at fixed pH increment ranges, but rather based on UV absorption of the eluting proteins. The fractions are collected on the basis of the presence of proteins in the eluent instead of at equally spaced pH changes. The pH range of each collected fraction is recorded by the custom software. The same ranges are then chosen as fraction collector trigger points for another protein mixture first dimension separation by pH gradient. The pH is monitored and when it exceeds the next range collected in the previous separation, the fraction collector is triggered to change tubes. Thus the second sample's fractionation occurs with pH ranges identical to the first sample.

The first dimension separation does not have to result in collection into individual tubes and subsequent injection of those individual fractions onto another column for second dimension separation. In some embodiments, the two separations are directly coupled in an automated process. A defined portion or pH range of the eluent from the first column is held up or loaded onto a blocking column. When the desired pH range has been loaded, computer controlled valves are used to switch the first dimension pH separation eluent onto another blocking column to begin loading the next pH range. The first blocking column is switched to a buffer that elutes its collected proteins onto a column used for second dimension separation. Thus by using several columns and appropriate valve switching, the entire two-dimensional physical separation process is automated.

The individual proteins now mostly physically separated from each other are analyzed by mass spectral methods to determine their identification. Each final protein fraction is directed to an LCT commercial mass spectrometry system to obtain its intact molecular weight. This will not be able to indicate however if the protein has been modified.

The protein is digested by enzymatic cleavage into a mixture of small peptides. This mixture is then analyzed by MALDI mass spectrometry to obtain a peptide mass fingerprint. No fragmentation occurs. When combined with the intact molecular weight information from the LCT data, accurate protein identification can be made with high confidence.

Alternatively, in preferred embodiments, the peptide digest is separated by capillary electrophoresis and each peptide identified by on-line by mass spectrometry (CE/MS). Each peptide is fragmented and its amino acid sequence determined. This leads to very accurate identification of the protein with high confidence.

There will be 15 fractions if separated into a range of 0.2 pH each over a total of 3 pH units in the first dimension. With 75 proteins in each fraction there are 1125 proteins to be digested and analyzed by CE/MS. Custom software has been developed to automate the CE/MS process. It controls a sample injector, high voltage power supply for CE sample loading and running, CE column/electrode mechanical positioning for loading and running and overall system timing. Data from the time of flight mass spectrometer is digitized and recorded by computer with the custom software. The software provides representation of the data as a two dimensional image for visual identification of important mass spectral peak locations.

In some embodiments, CE/MS/MS is performed. The desired ion of interest is first isolated and then fragmented. The ions in the eluting peak are confined to an ion trap and a waveform consisting of all frequencies except for the frequency resonance with the desired ion is applied to the cell. This notched waveform excludes all interferences from the ion trap. Then, a low amplitude frequency that is resonant with the desired ion is applied to partially fragment it. When subsequently ejected from the ion trap, both parent and fragment ion information is obtained free of background. In order to know the appropriate frequency to apply to the ion trap, the mass-to-charge ratio of the desired ion must be known. Custom software has been developed to determine this in real time as a peak begins to elute. The notched waveform is immediately calculated and applied to the ion trap so that background-free spectra can be acquired over as much of the peak as possible. This capability avoids the necessity of performing two injections with the first done to obtain the desired ion-of-interest information. This more than doubles the throughput. The amplitude of the frequency resonant with the desired ion-of-interest is feedback adjusted in real time from spectra to spectra to optimize the parent to fragment ion ratio.

### Example 10

### CE-MS/MS Analysis of CaldCL1

This Example describes the analysis of CaldCL1, a fully malignant breast cancer cell line. Cells were separated by rotofor and chromatography, followed by tryptic digestion for CE-MS/MS analysis through database searching. The high efficiency of this method was demonstrated and discussed based on increased peptide coverage yield compared to other mass spectrometric methods.

### A. Methods

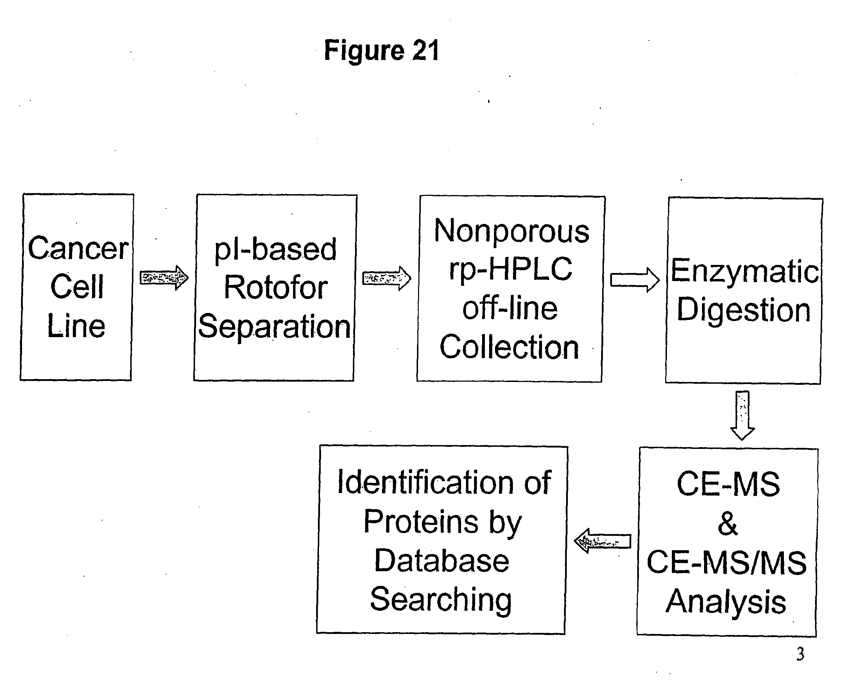

Figure 21 shows an overview of the method utilized. A Mini-Rotofor (17 mL capacity) in pH range of 4 to 9 was utilized for the first separation phase. The ampholytes used were 1 mL of 40% Bio-Lyte 3-10. The rotofor was run at 12 W for 3.5 hrs. A MICRA-Platinum nonporous reversed phase C18 column (1.5 µm, 33 x 4.6 mm) from Eichrom was next used to separate the mixture in a second phase.
The HPLC fractions were next digested and processed for CE-MS/MS. Briefly, a protein to enzyme ratio of approximately 1:30 to 1:40 was used. 4 µL of 0.5 µg/mL trypsin was added with 40 µL of 50 mM NaHCO₃ digestion buffer at pH 7.8. The digestion mixture was incubated at 37°C for approximately 18 hrs. The digested sample was then dried down by speed vacuuming and reconstituted with 1 ml of DI water and stored at -20°C until analysis.

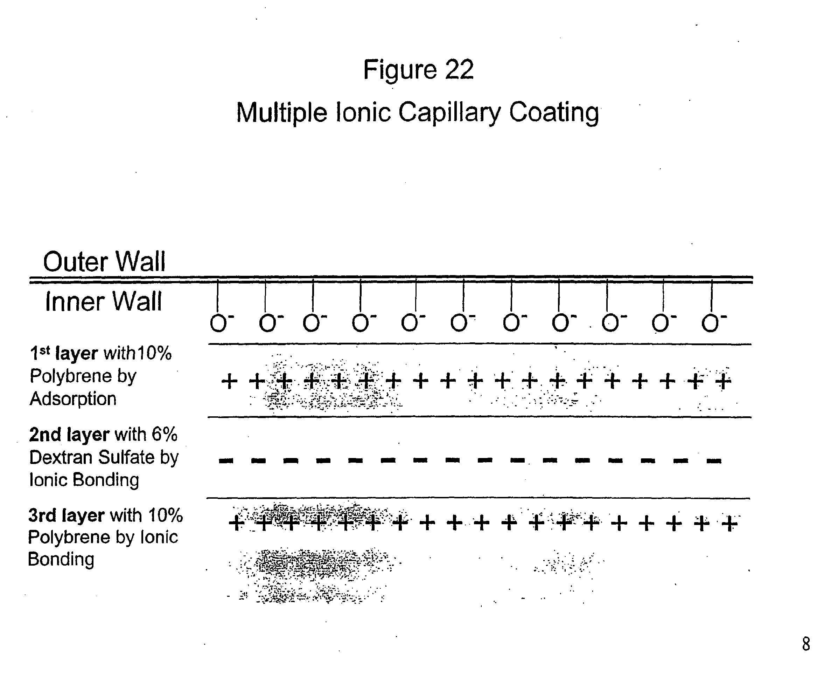

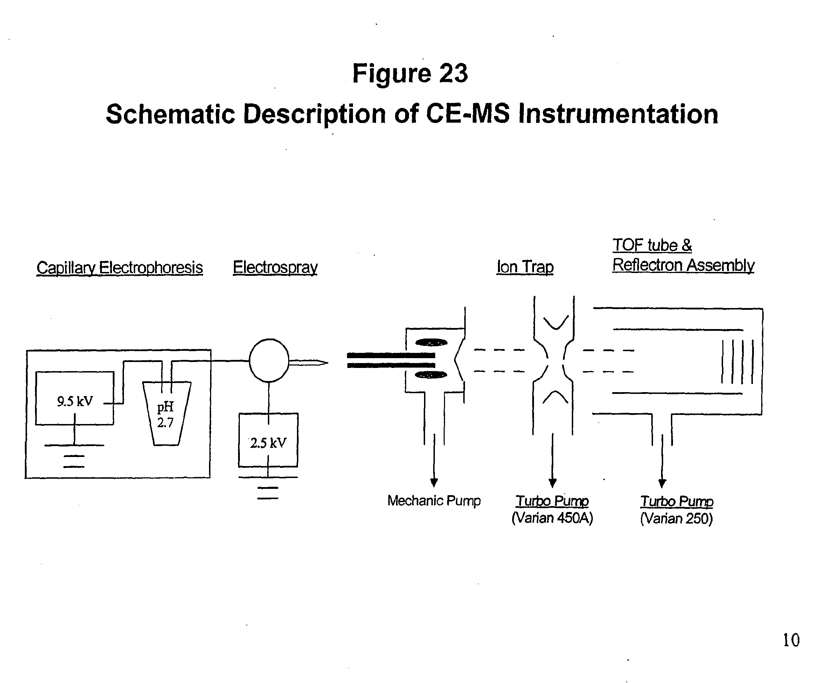

For CE analysis, a capillary column with dimensions of 50 cm long, 150 µm OD/50 µm ID was utilized. The running buffer solution was 50mM ammonium formate at pH 2.7. An electrokinetic injection at 1 to 3 kV for 10 to 60 sec, followed by a voltage gradient of approximately 240 V/cm during the run was used. A positively charged inner wall with multiple coating procedure was utilized (See Figure 22). A capillary tip coated with silver for sheathless ESI was also utilized. The multiple coating of the capillary column was performed as follows. Each step was performed under N₂ (g), followed by DI water rinse for 5 min. The steps were:
1. 1 M NaOH rinse for 30 min
2. 10% polybrene (w/v) / 3% ethylene glycol (v/v) rinse for 30 min
3. 6% dextran sulfate rinse for 30 min
4. 10% polybrene (w/v) / 3% ethylene glycol (v/v) rinse for 30 min
Figure 23 shows a flow chart of the CE-MS/MS instrumentation and procedures utilized.

### B. Results

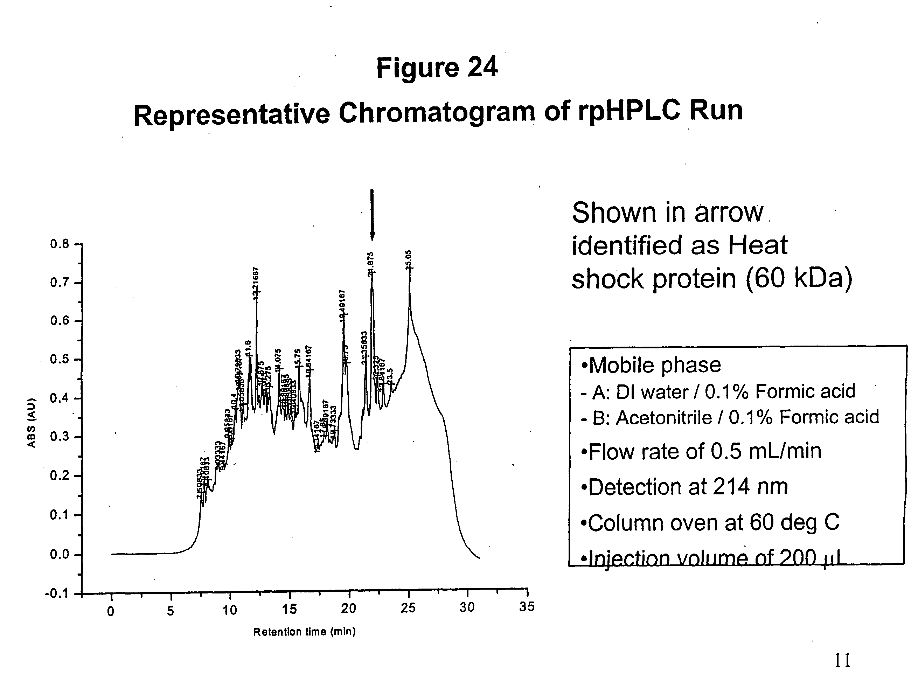

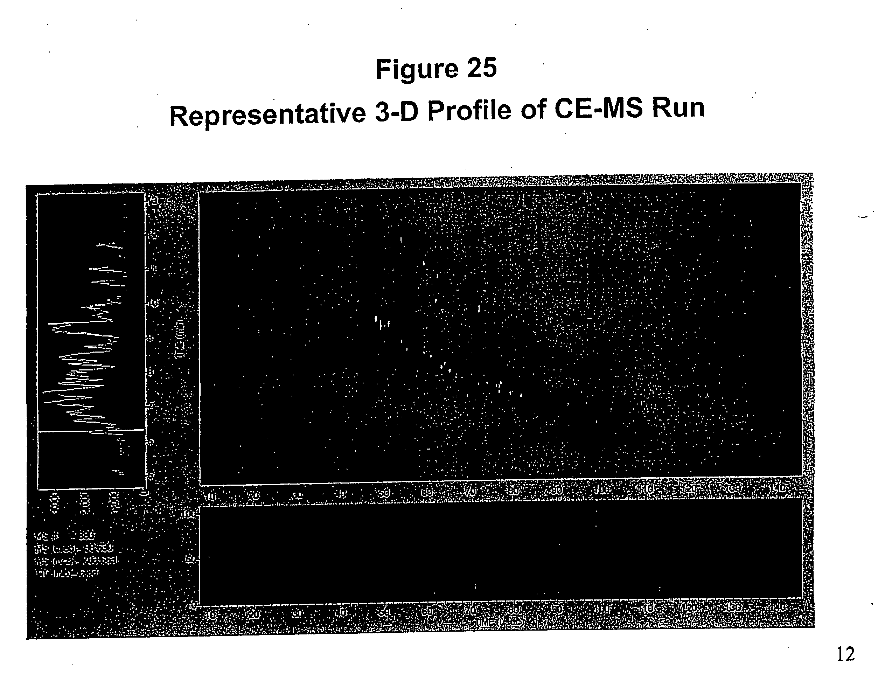

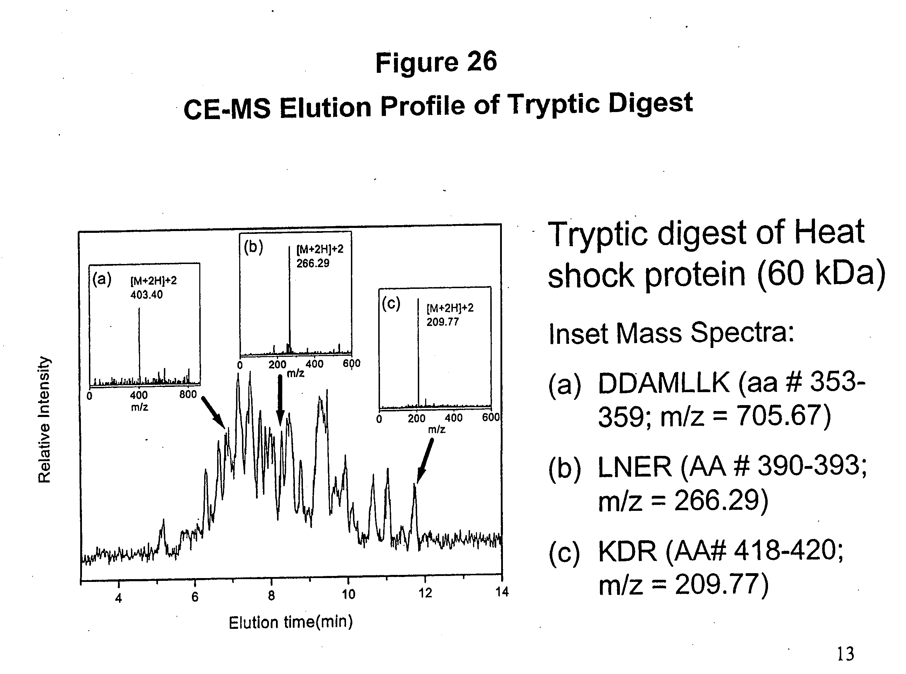

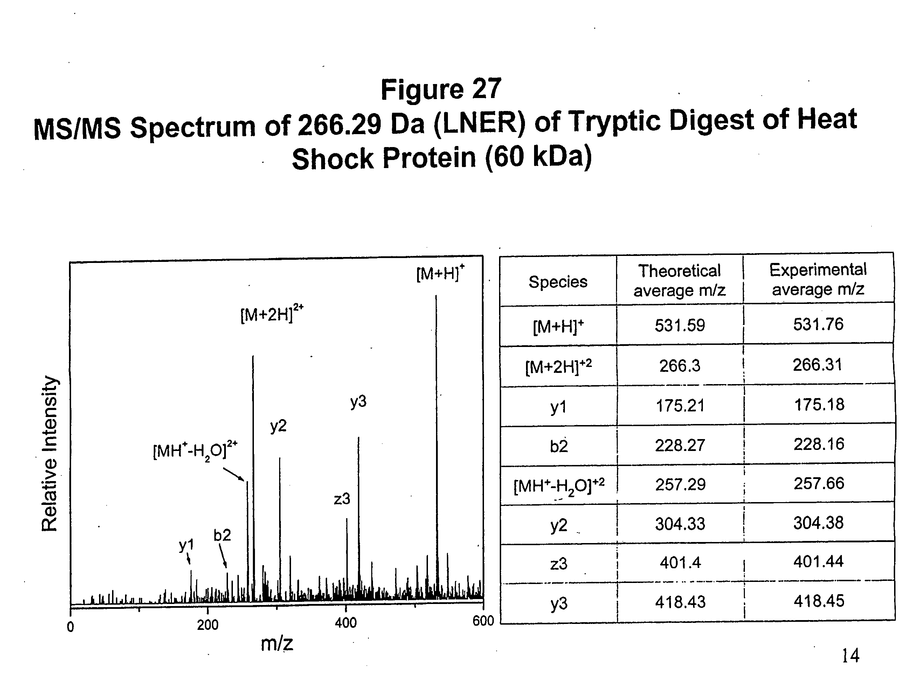

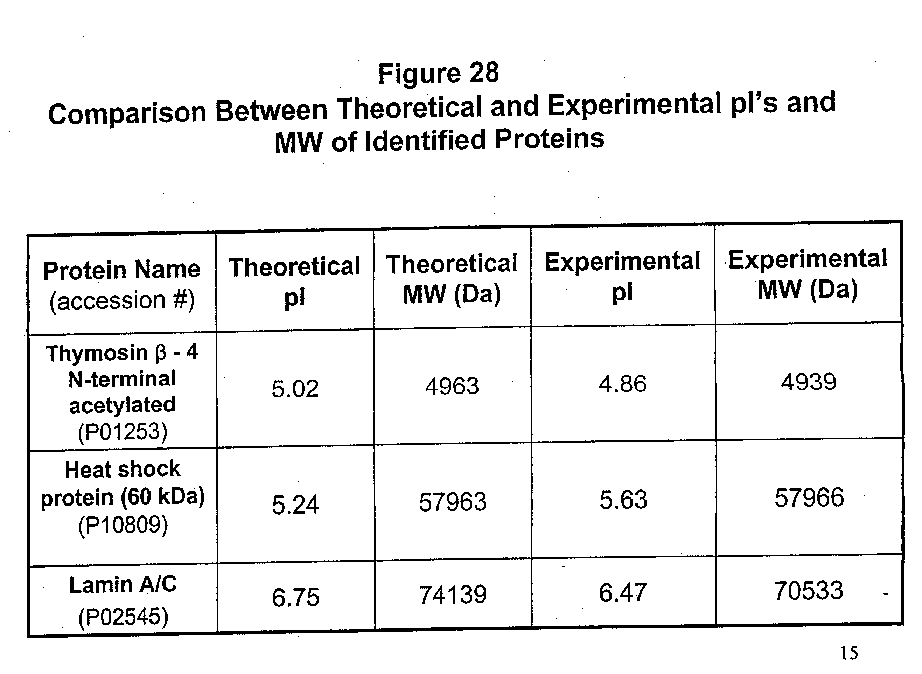

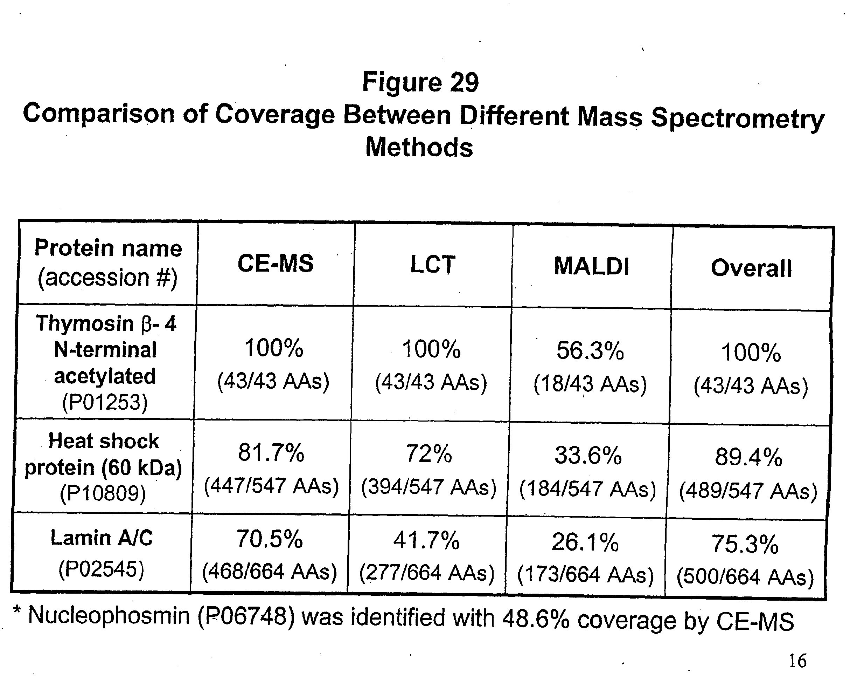

Figure 24 shows a representative chromatogram of an RP HPLC run. Figure 25 shows a representative 3D profile of a CE-MS run. Figure 26 shows a CE-MS elution profile of a tryptic digest. Figure 27 shows a MS/MS spectrum of a tryptic digest of heat shock protein. Figure 28 shows a table of theoretical and experimental pI's and MW of proteins identified. Figure 29 shows a comparison of coverage between different MS methods.

In conclusion, several proteins in malignant breast cancer cell line were successfully identified by an on-line capillary electrophoresis - tandem mass spectrometer method with higher peptide coverage than other mass spectrometry methods. The capillary column generated by multiple ionic coating procedure demonstrated increased stability. The on-line capillary electrophoresis-tandem mass spectrometry for analysis of proteome methods described resulted in the collection of 2-D Data of total ion count and MW during electrophoretic separation usingMS/MS with SWIFT technology. The tryptic digest of cancer cell line proteins for identification by database searching resulted in increased coverage.

### Example 11

### Microarray of Cancer Proteins Produced Utilizing Chromatofocusing and HPLC Techniques

In one exemplary embodiment of the present invention, a microarray of cancer proteins is produced utilizing chromatofocusing and HPLC techniques.

In order to develop a microarray of cancer proteins, prostate cancer cell line, LnCAP, was used. LnCAP cells were cultured in RPMI medium supplemented in 100 mm tissue culture dishes (Becton Dickinson, Franklin Lakes, NJ). Log phase cells were harvested, washed in PBS and pelleted. The LnCAP cell pellets were lysed and used for fractionation. Cell pellets were reconstituted in lysis buffer. The lysis buffer consisted of 7M urea, 2M thiourea, 100mM DTT, 0.5% biolyte 3-10,2% octyl glucoside and 1mM PMSF. The cell pellets were lysed at room temperature for half an hour, followed by centrifugation at 35,000 rpm at 4 degrees C for 1 hour. The supernatant was stored at 80 degrees C for future use. A PD 10 column, equilibrated with 25mM bis-tris in 6M urea and 0.2% octyl glucoside, was used to exchage the cell lysate from the lysis buffer to the above buffer.

LnCAP cell lysates were next fractionated using a pI gradient by chromatofocusing at 0.2 pI intervals. Two buffers, a start buffer and an elution buffer, were employed. The start buffer was 25mM bis-tris with pH 7.1, and the elution buffer was polybuffer74:water in a ratio of 1:10 with pH 4.0. Both buffers were prepared in 6M urea and 0.2% octyl glucoside. Iminodiacetic acid was used to adjust the pH of both buffers. The PS-HPCF 1D column was equilibrated with the start buffer until the pH of the effluent was 7.1. Sample was applied to the column with multiple injections. Once a stable baseline was achieved, the elution buffer was switched on to elute the proteins on the column in an isocratic mode. UV detection was performed at 280 nm and the pH of the effluent was monitored using a flow-through online pH probe. The pH fractions were collected in 0.2 pH intervals and 15 fractions in total were collected in the range of pH 7-4. The CF separation was stopped when the pH of the effluent reached 4.

Each of these pI fractions were collected and separated in the second dimension by NPS RP-HPLC. RP-HPLC was performed using PS-HPCF 2D (4.6 X 33mm) columns. Solvent A was 0.1% TFA in water and Solvent B 0.1% TFA in acetonitrile. The gradient was run from 5% to 15% in 1min, 15% B to 25% in 2 min, 25% to 31% in 2 min, 31% to 41% in 10 min, 41% to 47% in 6 min, 47% to 67% in 4 min, finally up to 100% B in 3 min and held for another 1 min, then back to 5% in 1 min at a flow rate of 1ml/min. The column temperature was 40° C higher than the ambient temperature. UV absorptions were monitored at 214 nm. RP fractions were collected using a fraction collector. The fractions were dried down completely by Speed-Vac at 75° C and stored at - 80° C until further use.

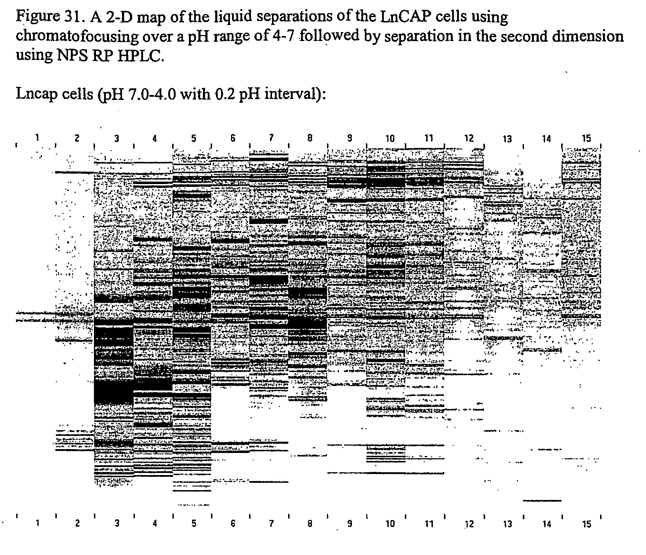

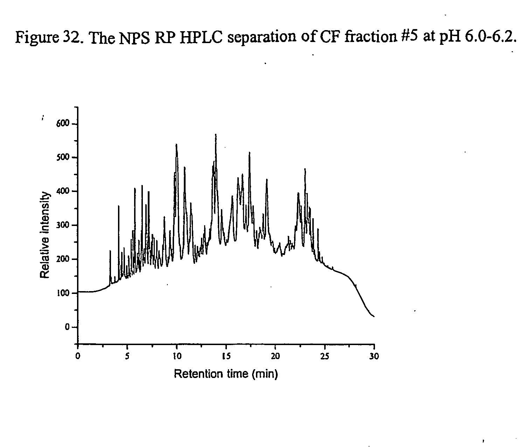

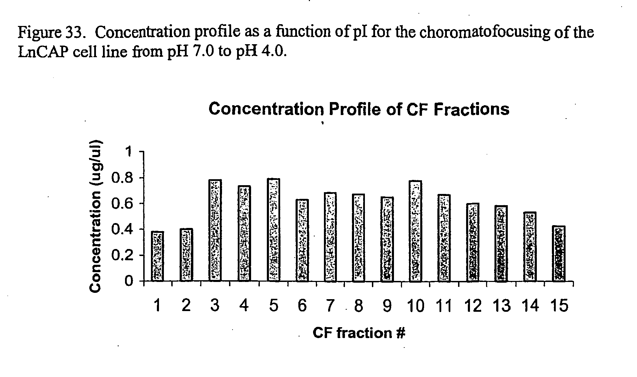

A profile of the total fractionation from pH 4-7 is shown in Figure 31. Some of the fractions obtained after chromatofocusing contained as many as 70-90 proteins. As a representative example the different proteins separated by NPS RP-HPLC from the pI fraction 5 are shown in Figure 32. It is estimated that the sample was fractionated into as many as 1400 protein bands. Many of these bands contain single proteins, but some contain more than one protein. The presence of one or more proteins could be confirmed by MW measurements using ESI-TOF MS. Nevertheless, this method provides cellular proteins with a relatively high degree of purity, which can be used for microarray experiments. The relative amount of protein collected in each fraction from the CF column and loaded onto the HPLC separation, as determined by the Bradford Protein assay, is shown in Figure 33.

More proteins are usually recovered from the mid-pH fractions than towards the end of the pH range as expected. In these experiments using analytical columns, generally up to 5 mg of protein could be loaded on the first dimension without overloading the column and losing significant resolution. The total recovery of protein from the first dimension has been found to be around 51 % over the range of pH 4-7. The latter can be increased by extending the pH range to 8.5. In addition, some acidic proteins (with pI below 4) and highly hydrophobic proteins might be lost during the fractionation. It is contemplated that an additional salt wash and isopropanol wash provides a means to recover these proteins. Nevertheless, on average it is expected that around 150µg total protein is recovered from each pI fraction. The proteins from each pI fraction are further separated by NPS RP-HPLC, into about 70-80 fractions with high degree of purity. The recovery of proteins in each fraction by NPS RP-HPLC depends on the proteins but on average is around 80 %. The result is that on average around 1.5 µg of protein is collected from each protein band. This corresponds to approximately 12 pg of protein or 0.05 femtomole for a 30 kDa protein. The sensitivity of the arrays can be increased ten fold by using a more concentrated protein solution as baits. Importantly, this method could be adapted to preparative scale such that large amounts of material would be available for constructing many protein arrays. In the present work approximately 100 microarrays were spotted from each collection.

The targeted UV peak in the 2^{nd} dimensional RP-HPLC chromatogram was collected and aliquoted into 3 fractions. One portion was dried down completely and used for microarray experiments. A second portion was dried down completely and reconstituted in 50% ACN with 2% formic acid. The resulting solution was directly infused into an ESI-TOFMS (LCT, Micromass, Inc.) at 10ul/min to determine intact molecular weight values of the proteins in the fraction. The other portion of the liquid band was dried down to eliminate ACN and TFA. 1M NH₄HCO₃ and 10 mM DTT were then added to a final concentration of 100mM and 1 mM, respectively. A sequence-specific endopeptidase such as trypsin was then mixed with the denatured proteins at the ratio of 1:50. The mixture was incubated at 37°C for 24 hours. MALDI-TOFMS (Micromass, Inc., TOFSpec2E) was subsequently employed to analyze peptide mass fingerprints (PMF), which were then used to search for the registered peptide masses of proteins in the database. The intact molecular weights from LCT and the PMF obtained using MALDI-TOFMS were together used to identify the protein in the database. Further confirmation of the protein identity was obtained by MS/MS sequence analysis using the MALDI QTOF MS (Micromass, Inc.).

The fractionated proteins were resuspended in 25 µl buffer containing 125 mM Tris-Cl (pH= 6.8), 4 % SDS and 2 % ß-mercaptoethanol (Sigma, St Louis, USA). This was the minimum volume required for spotting in the initial work. The samples were denatured by heating at 70 °C for 2h followed by a final denaturation at 100 °C for 10 min in a thermal cycler (Perkin Elmer Life Sciences, Boston, MA). The samples were transferred to a 96 well microtitre plate (MJ Research, Waltham, MA) and printed on nitrocellulose slides (Schleicher & Schuell, Keene, NH) using a GMS 417 microarray printer (Genetic MicroSystems, Woburn, MA). In later work the spotting was performed using Magna Spotter microarray printer (Bioautomation). Using this system the spotting volume of the protein was reduced to 2.5 uL, allowing the ability to use concentrated fractions as baits. Each spot measured approximately 300 µm in diameter and was placed 1200 µm apart. The plates used to print are 96 well V bottom plates from Major Scientific. The slides were either used immediately or stored at 4 °C. They were dried for 1 h at room temperature before further processing.

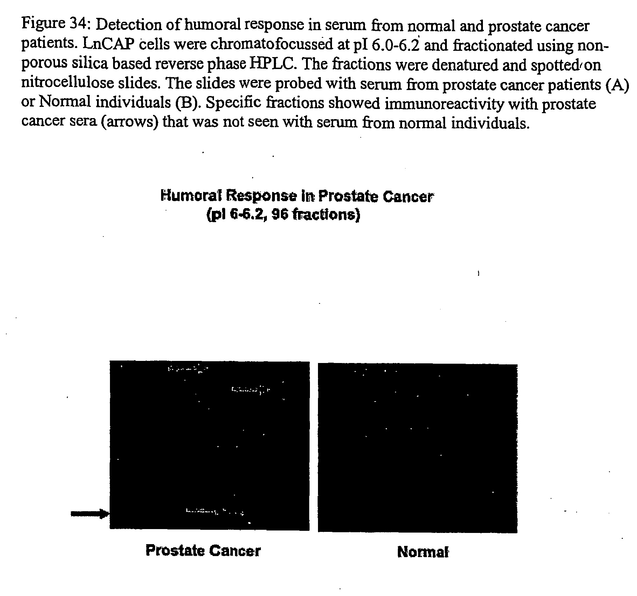

As a representative example, a slide-containing proteins fractionated in the pH range of 4-7 were probed with either serum from healthy individuals or from sera from prostate cancer patients. Figure 34 shows one such representative example of the array containing 96 fractions separated in the pH range of 6-6.2. Immunoreactivity was observed with specific fractions when probed with prostate cancer serum (Figure 34A, arrow) which was not seen in serum from healthy subjects (Figure 34B). This indicated the presence of auto-antibodies in serum from prostate cancer patients against specific cancer associated proteins.

The humoral response was next investigated. The humoral response generated against specific cancer proteins using a larger pool of serum samples. Nitrocelluose slides containing spotted proteins were blocked in TBS containing 1% non fat milk and 5 % normal donkey serum (Sigma, St Louis, USA) in 0.1% Tween-20 (Sigma, St Louis, USA), for 1 h at RT. The slides were rinsed with TBS and incubated with either serum from prostate cancer patients or normal individuals (1:50) in a microcaster chamber (Schleicher & Schuell, Keene, NH) for 2 h at RT. They were washed six times with TBS-T (TBS containing 0.1% Tween-20), each for 5 minutes. The slides were then incubated with biotin-conjugated anti-human IgG (1:200, Jackson ImmunoResearch Laboratories, West Grove, PA) for 30 min at RT. They were then washed with TBS-T and incubated with Cy5-conjugated Streptavidin (1:250, Jackson ImmunoResearch Laboratories, West Grove, PA) for 30 min. The slides were finally washed and dried as described above and analyzed using a microarray scanner (Axon Instruments Inc., Foster City, CA, USA).

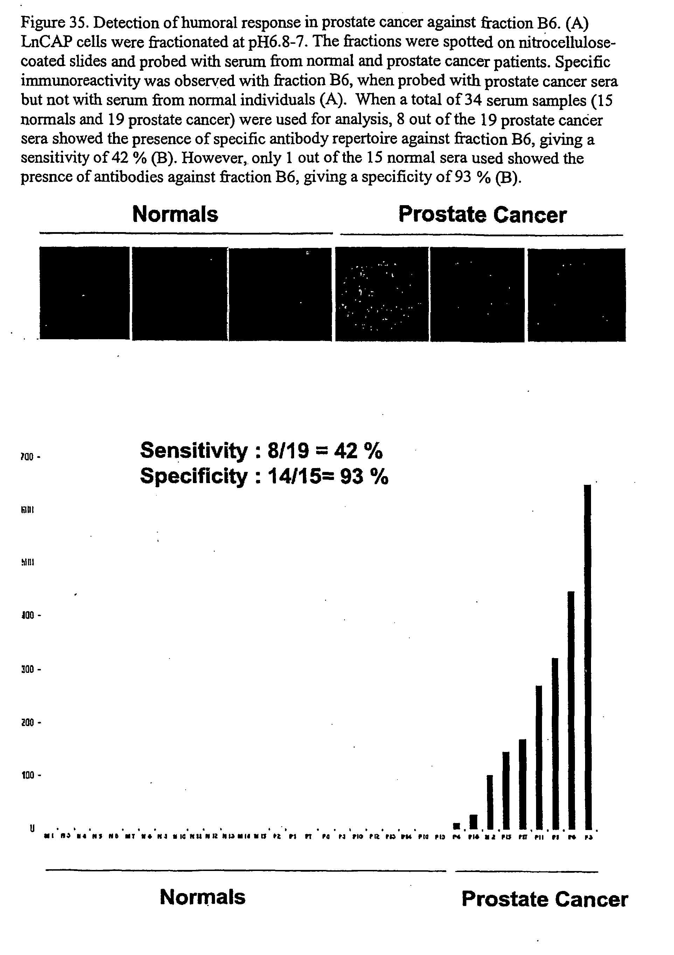

Figure 35 shows the profile of auto-antibodies against fraction B6, fractionated at pH 6.8-7. A specific repertoire of antibodies against fraction B6 was found in serum from prostate cancer patients while negligible immunoreactivity was observed using serum from normal individuals (Figure 35A). Quantitation of the data revealed 8 out of a total 19 PCA (prostate cancer) sera reacted with fraction B6 giving a sensitivity of 42% while only 1 out of the 15 normals showed reactivity, giving a specificity of 93 %. Such high degree of specificity makes fraction B6 a useful candidate to identify prostate cancer samples. This is especially so since prostate specific antigen (PSA), the routine marker used in the clinic shows a specificity of only 30-35 %. This implies that there is a high degree of false positivity (60-65 %) associated with PSA. As a result of such low specificity, PSA is used only as a primary screen to detect PSA. Thus in this context, fraction B6 provides a marker for monitoring the occurrence of PCA using serum samples to supplement PSA levels *(see, e.g.,* U.S. Patent App. Ser. No. 10/210,120 herein incorporated by reference in its entirety, for descriptions of cancer marker analysis). The low percentage of sensitivity observed with fraction B6 becomes less of an issue since PCA is wide spread in the general population.

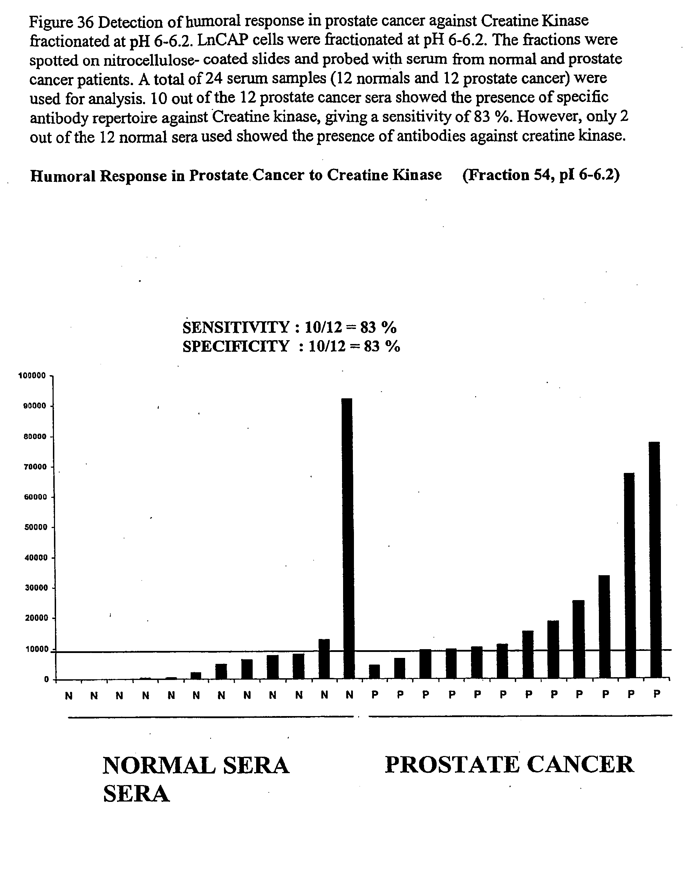

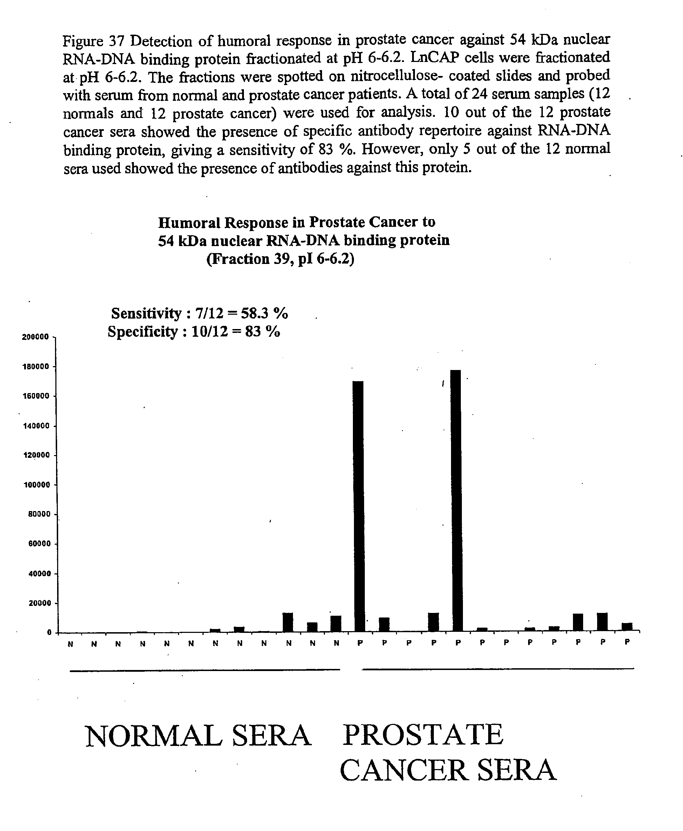

The sensitivity and specificity of monitoring PCA using serum markers could be increased by multiplexing data from multiple biomarkers. With this aim, additional spots were investigated for their immunoreactive profile using 24 serum samples, consisting of 12 normals and 12 cancers (Figs 36-37). Proteins in these spots were identified using MALDI-TOF and their molecular weights were determined using the LCT-MS. Thus Figure 36 shows the profile of auto-antibodies against mitochondrial creatine kinase which was fractionated from LnCAP cells at pH 6-6.2. Based on the profile of immunoreactivity shown by PCA and normal samples a threshold value of 900 normalized intensity units was used above which the serum was considered to show specific immune response. When such a criteria was used, 10 out the 12 PCA samples displayed a specific repertoire of auto-antibodies against mitochondrial creatine kinase. In contrast, only 2 out of 10 normals showed any reactivity. Thus the sensitivity and specificity for mitochondrial creatine kinase was 83 %. Interestingly, mitochondrial creatine kinase has been associated with adenocarcinoma and its detection in sera has been correlated to poor outcome of the cancer. In this regard the present results showing the presence of auto-antibody repertoire could be due to overexpression of this protein in PCA. Another interesting protein which showed presence of auto-antibody repertoire was the 54 kDa non-POU domain containing nuclear RNA-DNA binding protein. This was also fractionated from LnCAP cells at pH 6-6.2 (Figure 37). Among the 12 PCA sera tested 10 of the serum showed specific immune response. Thus, using a novel method of fractionating proteins from cancer cells and protein microarrays, this study identified 3 novel proteins that elicited a specific antibody response in PCA. Interestingly, the specificity of immune response to each of these three proteins showed high degree of specificity (58-93 %). This was much higher than the specificity exhibited by PSA (30-35 %).

An important issue in these experiments is the ability to identify the protein spots that show a humoral response. Identification is performed using a peptide mapping and MS/MS and MW measurements. ESI-TOF MS is used to measure the MW of the intact protein directly from the vial from which the protein was spotted. The sample can then be digested by trypsin and analyzed by MALDI-MS and MALDI-MS/MS for peptide mapping and database searching. The combination of intact MW and peptide mapping and MS/MS generally provided a reliable match against the database. The use of peptide mapping alone often provided an unreliable identification with a relatively low MOWSE score. The MS/MS of several peptides using the MALDI QTOF MS usually provided the additional information needed to unambiguously identify the protein.

The use of CF fractionation provides a number of distinct advantages in these protein array experiments. The method results in separation of large numbers of purified proteins in the liquid phase. In this initial work over a pH range of 4-7 nearly 1400 protein bands were observed by UV detection and collected. One could extend the range by changing the buffers so that additional proteins could be collected over a pH range up to 9.0. Further, the CF method has provided fractionation in intervals of 0.2 pI, where there is little overlap of proteins between adjacent pI fractions. This contrasts to previous work using the Rotofor as a means of liquid phase pI fractionation, where pI lanes are ~0.5 pI and there is often overlap of proteins between adjacent pI fractions. The CF method allows fractionation of up to 0.05 pI units, thus enabling additional separation of overlapping proteins. The use of ESI-TOF MS has shown that some of the protein bands may consist of more than one protein so that an additional stage of fractionation or a narrower pI range could be used to achieve further isolation of proteins if required.

An advantage of the CF method is that using analytical columns, it can provide a substantial amount of material for each protein band where between 3-5 mg of sample can be loaded on the column. This may result in as much as 1 µg of protein on average in each band so that significant amounts of protein may be spotted in each array spot. This will be important in the sensitivity for detection, especially for proteins expressed at lower levels where sensitivity will be at a premium. Also, the high reproducibility of the method allows for additional experiments to be run where the same protein can be recollected for further analysis of the spot by mass spectroscopy and a variety of traditional methods. Alternatively the CF experiment could be run using preparative columns, however, this is at the expense of resolution, where it was found that it is preferable to run the analytical experiment several times to collect additional material. In addition, the use of 2-D liquid phase separation of proteins has several other distinct advantages for these microarray experiments. The CF fractionation can be run in an hour while each of the 15 fractions can each be separated by NPS RP-HPLC in around 20 minutes. The entire fractionation can be performed in ~ 6 hours. In addition, the entire process can be automated so that large numbers of different cell lines can be fractionated for spotting on an array. In addition, the CF fractionation provides proteins as expressed by the cells. Analysis of these proteins by mass spectrometry, shows that many of the proteins do not have MW values that correspond to that of the database and are modified. These modifications as expressed by the cell are often important in the cell cycle and the onset of cancer. Modified isoforms of protein have been shown to have a very specific humoral response to patient plasma using 2-D gel-based studies.

## Claims

1. A method for generating protein microarrays, comprising:
a) providing:
i) at least one sample comprising a plurality of polypeptides;
ii) a first protein separation apparatus comprising a chromatofocusing apparatus;
iii) a second protein separation apparatus comprising an apparatus for performing non porous reverse phase HPLC;
iv) a solid surface; and
b) treating said at least one sample with said first protein separation apparatus to generate a first separated polypeptide preparation under conditions such that said first separated protein sample is separated into fractions of 0.5 pI units or less;
c) treating said first separated polypeptide preparation with said non porous reverse phase HPLC apparatus to generate a second separated polypeptide preparation; wherein said second separated polypeptide preparation comprises a plurality of polypeptide fractions, and wherein said polypeptides in said second separated protein fraction are separated under conditions such that said polypeptides maintain modifications as expressed by said cell so as to retain reactivity to an autoantibody from sera from a subject diagnosed with cancer; and
d) transferring at least a portion of said second separated polypeptide preparation onto said solid surface to generate a protein microarray.

2. The method of Claim 1, further comprising the steps of:
e) exposing said protein microarray to a biological fluid comprising sera from a subject diagnosed with cancer; and
f) detecting binding of autoantibodies in said serum to proteins on said protein microarray.

3. The method of Claim 1 or 2, wherein said solid surface comprises a nitrocellulose slide.

4. The method of claim 2, further comprising the step of identifying said proteins on said microarray that are bound by said antibodies.

5. The method of claim 4, wherein said identifying comprises mass spectrometry.

6. The method of claim 5, wherein said mass spectrometry comprises ESI-TOF mass spectrometry.

7. The method of claim 6, wherein said mass spectrometry further comprises trypsin digest followed by MALDI-MS.

8. The method of claim 2, wherein said proteins on said microarray that are bound by antibodies in said serum from a subject having cancer but are not bound by serum from a subject not having cancer are cancer markers.

9. The method of claim 1 or 2, wherein said cancer is prostate cancer.

10. The method of claim 8, wherein said cancer marker is selected from the group consisting of mitochondrial creatine kinase and the 54kDa non-POU domain.

11. The method of claim 1 or 2, wherein second separated polypeptide preparation comprises at least 1000 fractions.

12. The method of claim 11, wherein each of said fractions comprises 1 polypeptide.

13. The method of claim 1, wherein said treating steps are completed in 4 to 6 hours.

14. The method of claim 1 or 2, wherein each of said polypeptide fractions comprises as average of 1 µg of protein when said sample comprises 3-5 mg of protein.

## Patentansprüche

1. Verfahren zum Erzeugen von Protein-Microarrays, umfassend:
a) Bereitstellen von:
i) wenigstens einer Probe, umfassend mehrere Polypeptide;
ii) einer ersten Vorrichtung zur Proteintrennung, umfassend eine Chromatofokussierungs-Vorrichtung;
iii) einer zweiten Vorrichtung zur Proteintrennung, umfassend eine Vorrichtung zum Durchführen einer nichtporösen Umkehrphasen-HPLC;
iv) einer festen Oberfläche; und
b) Behandeln der wenigstens einen Probe mit der ersten Vorrichtung zur Proteintrennung, um ein erstes getrenntes Polypeptidpräparat unter solchen Bedingungen zu erzeugen, dass die erste getrennte Proteinprobe in Fraktionen von 0,5 pI-Einheiten oder weniger aufgetrennt ist;
c) Behandeln des ersten getrennten Polypeptidpräparats mit der nichtporösen Umkehrphasen-HPLC-Vorrichtung, um ein zweites getrenntes Polypeptidpräparat zu erzeugen; wobei das zweite getrennte Polypeptidpräparat mehrere Polypeptidfraktionen umfasst und wobei die Polypeptide in der zweiten getrennten Proteinfraktion unter Bedingungen getrennt sind, bei denen die Polypeptide Modifikationen, wie sie durch die Zelle exprimiert worden sind, beibehalten und so die Reaktivität gegen einen Autoantikörper aus Sera eines mit Krebs diagnostizierten Subjekts zu bewahren; und
d) Überführen von wenigstens einem Teil des zweiten getrennten Polypeptidpräparats auf die feste Oberfläche, um ein Protein-Microarray zu erzeugen.

2. Verfahren gemäß Anspruch 1, umfassend folgende weitere Schritte:
e) Exponieren des Protein-Microarrays an eine biologische Flüssigkeit, die Sera von einem mit Krebs diagnostizierten Subjekt umfasst; und
f) Nachweisen der Bindung von Autoantikörpern in dem Serum an Proteine auf dem Protein-Microarray.

3. Verfahren gemäß Anspruch 1 oder 2, wobei die feste Oberfläche einen Nitrocellulose-Objektträger umfasst.

4. Verfahren gemäß Anspruch 2, ferner umfassend den Schritt des Identifizierens der Proteine auf dem Microarray, die von den Antikörpern gebunden sind.

5. Verfahren gemäß Anspruch 4, wobei das Identifizieren Massenspektrometrie umfasst.

6. Verfahren gemäß Anspruch 5, wobei die Massenspektrometrie ESI-TOF-Massenspektrometrie umfasst.

7. Verfahren gemäß Anspruch 6, wobei die Massenspektrometrie ferner einen Trypsinaufschluss, gefolgt von MALDI-MS, umfasst.

8. Verfahren gemäß Anspruch 2, wobei die Proteine auf dem Microarray, die von Antikörpern in dem Serum eines Subjekts mit Krebs, jedoch nicht von Serum eines Subjekts ohne Krebs gebunden werden, Krebsmarker sind.

9. Verfahren gemäß Anspruch 1 oder 2, wobei der Krebs Prostatakrebs ist.

10. Verfahren gemäß Anspruch 8, wobei der Krebsmarker ausgewählt wird aus der Gruppe bestehend aus mitochondrialer Kreatinkinase und der 54-kDA nicht-POU-Domäne.

11. Verfahren gemäß Anspruch 1 oder 2, wobei das zweite getrennte Polypeptidpräparat wenigstens 1000 Fraktionen umfasst.

12. Verfahren gemäß Anspruch 11, wobei jede der Fraktionen 1 Polypeptid umfasst.

13. Verfahren gemäß Anspruch 1, wobei die Behandlungsschritte nach 4 bis 6 Stunden abgeschlossen sind.

14. Verfahren gemäß Anspruch 1 oder 2, wobei jede der Polypeptidfraktionen im Mittel 1 µg Protein umfasst, wenn die Probe 3-5 mg Protein umfasst.

## Revendications

1. Procédé d'élaboration de jeux ordonnés de microéchantillons protéiniques, comprenant les étapes consistant à :
a) fournir :
i) au moins un échantillon comprenant une pluralité de polypeptides ;
ii) un premier appareil de séparation de protéines comprenant un appareil de chromatofocalisation ;
iü) un deuxième appareil de séparation de protéines comprenant un appareil pour réaliser une HPLC en phase inverse non poreuse ;
iv) une surface solide ; et
b) traiter ledit au moins un échantillon avec ledit premier appareil de séparation de protéines pour élaborer une première préparation de polypeptides séparés dans des conditions telles que ledit premier échantillon de protéines séparées est séparé en fractions de 0,5 unités pI ou moins ;
c) traiter ladite première préparation de polypeptides séparés avec ledit appareil de HPLC en phase inverse non poreuse pour élaborer une deuxième préparation de polypeptides séparés ; dans lequel ladite deuxième préparation de polypeptides séparés comprend une pluralité de fractions polypeptidiques, et dans lequel lesdits polypeptides dans ladite deuxième fraction de protéines séparées sont séparés dans des conditions telles que lesdits polypeptides conservent les modifications exprimées par ladite cellule de façon à retenir une réactivité envers un auto-anticorps provenant de sérums issus d'un sujet diagnostiqué atteint d'un cancer ; et
d) transférer au moins une partie de ladite deuxième préparation de polypeptides séparés sur ladite surface solide pour élaborer un jeu ordonné de microéchantillons protéiniques.

2. Procédé selon la revendication 1, comprenant en outre les étapes consistant à :
e) exposer ledit jeu ordonné de microéchantillons protéiniques à un fluide biologique comprenant des sérums issus d'un sujet diagnostiqué atteint d'un cancer ; et
f) détecter la liaison des auto-anticorps dans ledit sérum avec les protéines sur ledit jeu ordonné de micro échantillons protéiniques.

3. Procédé selon la revendication 1 ou 2, dans lequel ladite surface solide comprend une lamelle de nitrocellulose.

4. Procédé selon la revendication 2, comprenant en outre l'étape consistant à identifier lesdites protéines sur ledit jeu ordonné de microéchantillons qui sont liées par lesdits anticorps.

5. Procédé selon la revendication 4, dans lequel ladite identification comprend une spectrométrie de masse.

6. Procédé selon la revendication 5, dans lequel ladite spectrométrie de masse comprend une spectrométrie de masse ESI-TOF.

7. Procédé selon la revendication 6, dans lequel ladite spectrométrie de masse comprend en outre un produit digéré de la trypsine puis une spectrométrie de masse MALDI.

8. Procédé selon la revendication 2, dans lequel lesdites protéines sur ledit jeu ordonné de micro échantillons qui sont liées par les anticorps dans ledit sérum issu d'un sujet atteint d'un cancer mais qui ne sont pas liées par le sérum issu d'un sujet n'étant pas atteint d'un cancer, sont des marqueurs du cancer.

9. Procédé selon la revendication 1 ou 2, dans lequel ledit cancer est le cancer de la prostate.

10. Procédé selon la revendication 8, dans lequel ledit marqueur du cancer est choisi dans le groupe constitué par la créatine-kinase mitochondriale et le domaine non POU de 54 kDa.

11. Procédé selon la revendication 1 ou 2, dans lequel la deuxième préparation de polypeptides séparés comprend au moins 1 000 fractions.

12. Procédé selon la revendication 11, dans lequel chacune desdites fractions comprend 1 polypeptide.

13. Procédé selon la revendication 1, dans lequel lesdites étapes de traitement sont effectuées en 4 à 6 heures.

14. Procédé selon la revendication 1 ou 2, dans lequel chacune desdites fractions polypeptidiques comprend une moyenne de 1 µg de protéine lorsque ledit échantillon comprend 3 à 5 mg de protéine.
